(19) Europäisches Patentamt
European Patent Office
Office européen des brevets

(11) Veröffentlichungsnummer: **0 303 925 B1**

(12) **EUROPÄISCHE PATENTSCHRIFT**

(45) Veröffentlichungstag der Patentschrift: **07.06.95**

(51) Int. Cl.$^{6}$: **C12N 15/70**

(21) Anmeldenummer: **88112864.9**

(22) Anmeldetag: **08.08.88**

# (54) Hochreprimierbare Expressionskontrollsequenzen.

(30) Priorität: **17.08.87 CH 3152/87**

(43) Veröffentlichungstag der Anmeldung:
**22.02.89 Patentblatt 89/08**

(45) Bekanntmachung des Hinweises auf die Patenterteilung:
**07.06.95 Patentblatt 95/23**

(84) Benannte Vertragsstaaten:
**AT BE CH DE FR GB IT LI NL**

(56) Entgegenhaltungen:
**EP-A- 0 067 540**
**EP-A- 0 130 074**
**EP-A- 0 138 437**
**EP-A- 0 186 069**
**EP-A- 0 207 459**
**DD-A- 237 675**

(73) Patentinhaber: **F. HOFFMANN-LA ROCHE AG**
**Postfach 3255**
**CH-4002 Basel (CH)**

(72) Erfinder: **Bujard, Hermann, Prof. Dr.**
**Remlerstrasse 9**
**D-6900 Heidelberg (DE)**
Erfinder: **Lanzer, Michael, Dr.**
**Uhlandstrasse 2**
**D-6947 Laudenbach (DE)**

(74) Vertreter: **Mezger, Wolfgang, Dr. et al**
**Grenzacherstrasse 124**
**Postfach 3255**
**CH-4002 Basel (CH)**

EP 0 303 925 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft hocheffiziente und hochreprimierbare Expressionskontrollsequenzen, Expressionsvektoren, die diese Expressionskontrollsequenzen enthalten, mit diesen Expressionsvektoren transformierte Mikroorganismen sowie Verfahren zu deren Herstellung mittels rekombinanter DNA Technologie. Die vorliegende Erfindung betrifft ferner Verfahren zur Herstellung von pro- und eukaryoten Proteinen mittels diesen hochreprimierbaren Expressionskontrollsequenzen, Expressionsvektoren und transformierten Mikroorganismen.

Die Produktionsmenge eines Proteins in einer Wirtszelle wird durch drei Hauptfaktoren bestimmt: die Kopienzahl seines Strukturgens innerhalb der Zelle, die Effizienz mit der die Strukturgenkopien transkribiert werden sowie die Effizienz mit der die resultierende Boten RNA ("mRNA") translatiert wird. Die Transkriptions- und Translationseffizienzen sind ihrerseits von Nukleotidsequenzen abhängig die normalerweise vor dem gewünschten Strukturgen bzw. der kodierenden Sequenz lokalisiert sind. Diese Nukleotidsequenzen (Expressionskontrollsequenzen) definieren, inter alia, diejenige Stelle an die die RNA Polymerase anheftet um die Transkription zu initiieren (die Promotersequenz) (siehe auch The EMBO Journal 5,2995-3000 [1986]) und an die die Ribosomen binden und mit der mRNA (dem Transkriptionsprodukt) in Wechselwirkung treten um die Translation zu initiieren.

Nicht alle Expressionskontrollsequenzen sind gleich effizient. Es ist deshalb oft von Vorteil die spezifische kodierende Sequenz für ein gewünschtes Protein von ihren benachbarten Nukeoltidsequenzen zu trennen und sie mit anderen Expressionskontrollsequenzen zu verknüpfen um eine höhere Expressionsrate zu erzielen. Nachdem dies bewerkstelligt ist, kann das neukombinierte DNA Fragment in ein Plasmid, mit hoher Kopienzahl, oder ein Derivat eines Bakteriophagen eingebaut werden, um die Strukturgenkopien innerhalb der Zelle zu erhöhen, wodurch gleichzeitig die Ausbeute an gewünschtem Protein verbessert werden kann.

Da die Ueberproduktion eines normalerweise nicht-toxischen Genprodukts für die Wirtszellen oft schädlich ist und die Stabilität eines speziellen Wirtszell-Vektorsystems erniedrigt, sollte eine Expressionskontrollsequenz, zusätzlich zur Verbesserung der Transkriptions- und Translationseffizienz eines klonierten Gens, kontrollierbar sein um die Regulierung der Expression während des Wachstums der Mikroorganismen zu ermöglichen. Als regulierbare Expressionskontrollsequenzen kommen beispielsweise solche in Frage, die während des Wachstums der Wirtszellen abgedreht werden können, und dann zu einem gewünschten Zeitpunkt wieder angedreht werden können um die Expression von grossen Mengen des gewünschten Proteins mittels dieser Expressionskontrollsequenz zu begünstigen.

Verschiedene Expressionskontrollsequenzen, die die vorstehend genannten Bedingungen erfüllen, wurden zur Expression von DNA-Sequenzen und Genen, welche für gewünschte Proteine kodieren, verwendet. Derartige Expressionskontrollsequenzen sind beispielsweise bekannt aus Science 198, 1056-1063 (1977) (Itakura et al.), Proc. Natl. Acad. Sci. U.S.A. 76, 106-110 (1979) (Goeddel et al.), Nature 283, 171-174 (1980) (Emtage et al), Science 205, 602-607 (1979) (Martial et al.), Gene 5, 59-76 (1979) (Bernard et al.), Gene 25, 167-178 (1983) (Ammann et al.), Proc. Natl. Acad. Sci. U.S.A. 80, 21-25 (1983) (de Boer et al.), sowie aus den europäischen Patentanmeldungen, Publikations-Nrn. 41767 und 186069.

In der europäischen Patentanmeldung, Publikations-Nr. 67540 werden ebenfalls Expressionskontrollsequenzen, die die vorstehend genannten Bedingungen erfüllen, offenbart. Diese sind durch Kombination des trp-Promoters mit dem lac-System entstanden. Diese Expressionskontrollsequenzen bewirken einen Repressionsfaktor von etwa 50.

In der DD-237675 werden Expresionskontrollsequenzen offenbart, die durch Kombination von T7-Promotoren mit dem TEM 1-$\beta$-lactamase-Gen(bla-Gen) erhalten wurden. Diese sind jedoch im Unterschied zu den vorstehend erwähnten Expressionssequenzen nicht regulierbar.

Erfindungsgemäss wurde nun gefunden, dass durch die Kombination von Promotersequenzen mit niedriger Signalstärke und hoher in vivo Promoterstärke mit Operator/Repressorsystemen mit hoher Komplexbildungsrate ($K_{ass}$), hocheffiziente und hochreprimierbare Expressionskontrollsequenzen erhalten werden. Diese Expressionskontrollsequenzen zeichnen sich gegenüber den bekannten vor allem dadurch aus, dass sie mehr als 1000-fach reprimierbar sind und nach Induktion bei idealen Wachstumstemperaturen eine hohe RNA-Syntheserate vermitteln (>10 $P_{bla}$-Einheiten).

Die vorligende Erfindung betrifft daher Expressionskontrollsequenzen, die durch die Kombination von Promotersequenzen mit niedriger Signalstärke von etwa $1 \cdot 10^6$ - $1.5 \cdot 10^8$ $M^{-1} \cdot sec^{-1}$, vorzugsweise von etwa $6 \cdot 10^7$ $M^{-1} \cdot sec^{-1}$, und hoher in vivo Promoterstärke von 10-100 $P_{bla}$ - Einheiten, vor- zugsweise von mehr als 20 $P_{bla}$-Einheiten mit Operator/Repressorsystemen mit hoher Komplexbildungsrate von etwa $1 \cdot 10^8$ - $1 \cdot 10^{11}$ $M^{-1} \cdot sec^{-1}$, vorzugsweise von $2 \cdot 10^9$ $M^{-1} \cdot sec^{-1}$ gekennzeichnet sind und die einen Repressionsfaktor von >1000 bewirken.

Die Signalstärken von Promotoren werden durch die Komplexbildungsrate ($K_{ass}$) zwischen Promoter und RNA-Polymerase beschrieben. Die $K_{ass}$-Werte werden wie in Beispiel 3 beschrieben bestimmt.

Die in vivo Promoterstärke ist durch die RNA-Syntheserate, die durch eine einzelne Promotersequenz ausgelöst wird, definiert und wird in $P_{bla}$-Einheiten gemessen. Hierzu kann auf die Publikation von Deuschle et al. (The EMBO Journal 5, 2987-2994 [1986]) verwiesen werden.

Die Komplexbildungsrate ($K_{ass}$) von Operator/Repressorsystemen beschreibt die Geschwindigkeit, mit der ein Repressor an den Operator bindet. Der für das lac-Operator/Repressorsystem beschriebene $K_{ass}$-Wert von $2 \cdot 10^9 M^{-1} \cdot sec^{-1}$ ist in Biochemistry 25, 3845-3852 [1986] beschrieben.

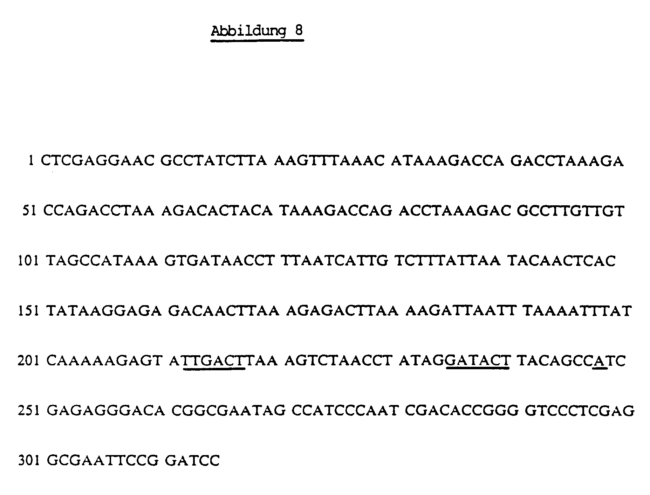

Als Promotersequenzen der erfindungsgemässen Expressionskontrollsequenzen kommen natürliche Promotersequenzen, sowie durch Mutation, bzw. Synthese gezielt veränderte funktionelle Varianten und Kombinationen dieser Promotersequenzen aus gram-negativen Organismen wie z.B. E.coli, aus gram-positiven Organismen, wie z.B. B.subtilis und B.stearothermophilis, sowie aus den entsprechenden Phagen in Frage. Bevorzugte Promotersequenzen der erfindungsgemässen Expressionskontrollsequenzen sind solche aus T-Coliphagen, wobei der T7AI Promoter (dessen Nukleotidsequenz in Abb. 8 gezeigt ist und der nachstehend auch als AI Promoter ($P_{AI}$) bezeichnet wird) besonders bevorzugt ist.

Als Operator/Repressorsysteme kommen alle durch chemische Induktoren direkt induzierbaren Systeme in Frage, die natürlicherweise oder nach entsprechenden Veränderungen (z.B. durch Mutation) einen Repressionsfaktor von >1000 ermöglichen, wobei unter diesen direkt induzierbaren Systemen nicht über SOS-Funktion (lexA/recA-System) oder durch Temperatur induzierbare System, wie z.B. das $P_L$-Operator/Repressorsystem, zu verstehen sind. Zu den durch chemische Induktion direkt regulierbaren Systemen gehören z.B. die Regulationseinheiten des Lactose-, Galaktose-, Tryptophan- und Tetrazyklin-Operons sowie andere negativ kontrollierbare Operons, d.h. über Operator/Repressor-Wirkung regulierte Operons. Hierzu kann auf das Lehrbuch von Miller und Reznikoff ("The operon", Cold Spring Harbor Laboratory, 1980), sowie auf die Publikation von Hillen et al. (J. Mol. Biol. 172, 185-201 [1984]) verwiesen werden. Besonders bevorzugte Operator/Repressorsysteme sind das natürliche lac-Operator/Repressorsystem (Miller und Reznikoff, supra) sowie durch Mutation bzw. Synthese gezielt veränderte Varianten dieser vorstehend genannten Operator/Repressorsysteme, welche einen Repressionsfaktor von >1000 ermöglichen. Der Ausdruck "Repressionsfaktor" beschreibt den Quotienten der in vivo Promotorstärken in An- und Abwesenheit eines Induktors.

Die Herstellung der erfindungsgemässen Expressionskontrollsequenzen kann nach bekannten, in der Literatur beschriebenen Methoden der rekombinanten DNA Technolgie erfolgen. Hierzu kann auf das Lehrbuch von Maniatis et al. ("Molecular Cloning" Cold Spring Harbor Laboratory, 1982) verwiesen werden.

Die Promotersequenzen mit niedriger Signalstärke und hoher in vivo Promoterstärke können mit einem oder mehreren Operator/Repressorsystem(en) zu einer erfindungsgemässen Expressionskontrollsequenz verbunden werden. Bei der Verwendung eines einzelnen Operator/Repressorsystems kann dieses innerhalb oder ausserhalb der Promotersequenz mit niedriger Signalstärke und hoher in vivo Promoterstärke lokalisiert sein. Folglich kann das Operator/Repressorsystem in die Promotersequenz integriert werden, diese teilweise ersetzen oder dieser in verschiedenen Abständen voran- oder nachgestellt werden. Vorzugsweise wird ein Operator/Repressorsystem in die Promotersequenz integriert, wobei eine besonders bevorzugte Integrationsstelle die Spacer-Region zwischen Position -12 und -29 ist (Nomenklatur wie in Abb. 8). Bei der Verwendung von zwei Operator/Repressorsystemen können beide innerhalb oder ausserhalb oder das eine innerhalb und das andere ausserhalb der Promotersequenz mit niedriger Signalstärke und hoher in vivo Promoterstärke lokalisiert sein. Vorzugsweise wird das eine in der Spacer-Region und das andere stromaufwärts in 5′-Richtung ("upstream") integriert, so dass über eine Repressorbindung eine maximale Kooperativität zwischen den beiden Operatorsequenzen des Operator/Repressorsystems erzielt wird, wodurch ein Repressionsfaktor bis ungefähr 30.000 erhalten werden kann.

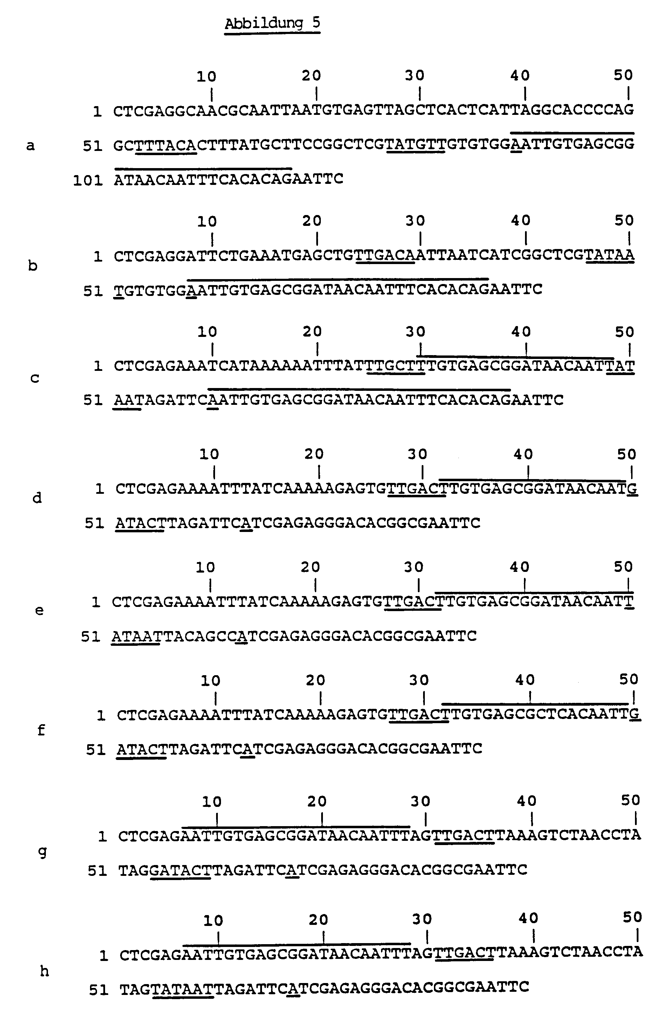

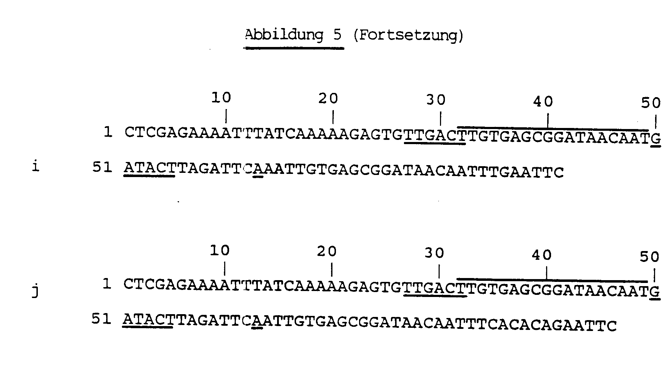

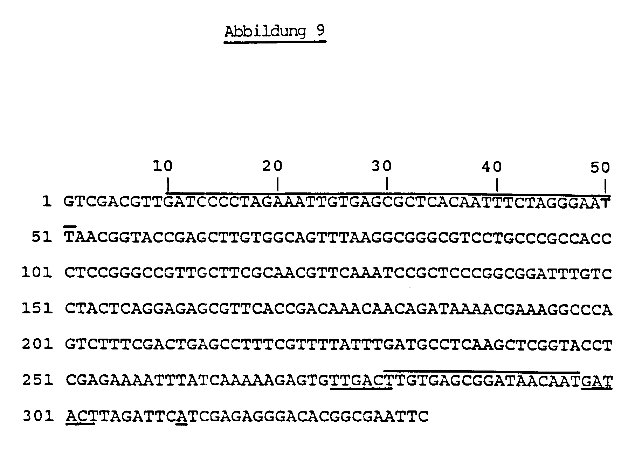

Die bevorzugten Expressionskontrollsequenzen der vorliegenden Erfindung wurden über chemische DNA-Synthese erhalten, wobei funktionelle Teile der lac-Operatorsequenz mit funktionellen Teilen der T7AI Promotersequenz kombiniert wurden. Die Konstruktion der so erhaltenen bevorzugten Expressionskontrollsequenzen A1OPSA1, A10PSA10P21, A10PSA10P29, A1pOPSA1 und OPA1OPSA1 wird im Detail im nachstehenden Beispiel 2 beschrieben. Die Nukleotidsequenzen dieser Expressionskontrollsequenz sind in Abb. 5 bzw. Abb. 9 und die sie charakterisierenden Eigenschaften zusammengefasst in Tabelle 8 gezeigt.

Die vorstehend genannten lac-Operatorsequenzen werden durch den lac-Repressor negativ reguliert. Um genügende Mengen an Repressormolekülen innerhalb der Zelle zu erhalten, kann das entsprechende Gen durch bekannte Methoden, wie beispielsweise durch Integration des $lacI^q$-Gens (Miller und Reznikoff, supra; Calos, Nature 274, 762-765 [1978]) in einen Vektor oder das Chromosom eines Bakteriums in Ueberschuss exprimiert werden.

Die erfindungsgemässen Expressionskontrollsequenzen können in an sich bekannter Weise in jeden geeigneten Expressionsvektor, der in gram-negativen und/oder gram-positiven Bakterien replizieren kann, eingebaut werden. Geeignete Vektoren können aus Segmenten von chromosomalen, nichtchromosomalen und synthetischen DNA-Sequenzen zusammengesetzt sein, wie z.B. verschiedene bekannte Plasmide und Phagen DNA's. Hierzu kann auf das vorstehend genannte Lehrbuch von Maniatis et al. verwiesen werden. Besonders geeignete Vektoren sind Plasmide der pDS-Familie (Bujard et al., Methods in Enzymology, Hrsg. Wu und Grossmann, Academic Press, Inc., Vol. 155, 416-433 [1987]).

Der Einbau der erfindungsgemässen Expressionskontrollsequenzen kann aber auch in das Chromosom von gram-negativen und gram-positiven Bakterienzellen erfolgen, wodurch auf die beim Arbeiten mit Vektoren notwendigen Selektionsmittel, wie z.B. Antibiotika, verzichtet werden kann.

Als geeignete DNA-Sequenzen, die mittels der erfindungsgemässen Expressionskontrollsequenzen exprimiert werden können, kommen solche in Frage, die pro- oder eukaryote Proteine in vivo oder in vitro kodieren. Beispielsweise können solche DNA-Sequenzen Enzyme, Hormone, Proteine mit immunregulatorischer, antiviraler oder antitumor Aktivität, Antikörper, Antigene und andere nützliche pro- oder eukaryote Proteine kodieren.

Als Proteine, die mittels der erfindungsgemässen Expressionskontrollsequenzen exprimiert werden können, kommen beispielsweise Malaria-Oberflächenantigene, insbesondere das 5.1 Oberflächenantigen, das CS-Protein und das p190-Protein von Plasmodium falciparum, Lymphokine, Interferone, Insulin und Insulin Vorläufer, HIV 1 und 2 Hüll- und Strukturproteine, Wachstumshormone und Wachstumshormon-Releasing Faktoren in Frage.

Die Methoden zur Expression von für pro- oder eukaryote Proteine kodierenden DNA-Sequenzen mittels in Expressionsvektoren eingebauten erfindungsgemässen Expressionskontrollsequenzen sind an sich bekannt und in dem vorstehend genannten Lehrbuch von Maniatis et al. beschrieben. Sie umfassen die folgenden Massnahmen:

(a) Transformation eines geeigneten Bakteriums, vorteilhaft E.coli, Salmonella typhimurium oder B.subtilis mit einem Expressionsvektor in welchem die kodierende DNA eines gewünschten pro- oder eukaryoten Proteins mit einer vorstehend genannten Expressionskontrollsequenz operabel verknüpft ist;
(b) Kultivierung des so erhaltenen Bakteriums unter geeigneten Wachstumsbedingungen; und
(c) Isolierung und, falls gewünscht, Reinigung des gewünschten Proteins.

Die Expression von für pro- oder eukaryote Proteine kodierenden DNA-Sequenzen mittels in das Chromosom eines Bakteriums, vorteilhaft E.coli, Salmonella typhimurium oder B.subtilis, eingebauten erfindungsgemässen Expressionskontrollsequenzen kann durch die folgenden Verfahrensschritte erfolgen:

(a) Einbau einer vorstehend genannten Expressionskontrollsequenz, die mit der kodierenden Sequenz eines gewünschten pro- oder eukaryoten Protein operabel verknüpft ist, in das Chromosom eines geeigneten Bakteriums;
(b) Kultivierung des so erhaltenen Bakteriums unter geeigneten Wachstumsbedingungen; und
(c) Isolierung und, falls gewünscht, Reinigung des gewünschten Proteins.

Die Auswahl eines geeigneten Wirtsorganismus wird von verschiedenen dem Fachmann bekannten Faktoren bestimmt. So spielen beispielsweise Kompatibilität mit dem ausgewählten Vektor, Toxizität des Expressionsproduktes, Expressionscharakteristika, notwendige biologische Sicherheitsvorkehrungen und Kosten eine Rolle und es muss ein Mittelweg zwischen allen diesen Faktoren gefunden werden.

Als geeignete Wirtsorganismen kommen gram-negative und gram-positive in Frage, beispielsweise E.coli, Salmonella typhimurium oder B.subtilis Stämme. Ein besonders bevorzugter Wirtsorganismus der vorliegenden Erfindung ist der E.coli Stamm M15. Ausser dem oben genannten E.coli Stamm können aber auch andere allgemein Zugängliche E.coli Stämme, beispielsweise E.coli 294 (ATCC Nr. 31446), E.coli RR1 (ATCC Nr. 31343) und E.coli W3110 (ATCC Nr. 27325) verwendet werden.

Die folgenden Beispiele tragen zum besseren Verständnis der vorliegenden Erfindung bei. Diese Beispiele können besser verstanden werden wenn sie im Zusammenhang mit den begleitenden Abbildungen und Tabellen gelesen werden. In diesen Abbildungen und Tabellen treten die folgenden Abkürzungen und Symbole auf:

B, C, E, H, Ha, K, P, Sa, X und Xb bezeichnen Schnittstellen für die Restriktionsendonukleasen BamHI, ClaI, EcoRI, HindIII, HpaI, KpnI, PstI, SalI, XhoI bzw. XbaI.

repräsentiert die Promotoren der Gene bla, lacI und neo; repräsentiert die ribosomalen Bindungsstellen der Gene bla, cat, neo, lacI und lacZ, repräsentiert die Terminatoren $t_o$, T1 und TE, wobei ein Pfeil die funktionelle Orientierung der Terminatoren angibt; repräsentiert die Expressionskontrollsequenzen A1OPSA1, lacOP29 und $P_{N25}$; repräsentiert den Operator OP in der Expressionskontrollsequenz OPA1OPSA1; repräsentiert die für die Replikation benötigte Region (repl.); repräsentiert kodierende Regionen für Dihydrofolatreduktase (dhfr), Chloramphenicolacetyltransferase (cat), lac Repressor (lacI), β-Lactamase (bla), β-Galaktosidase (lacZ) und Neomycinphosphotransferase (neo).

Abbildung 1

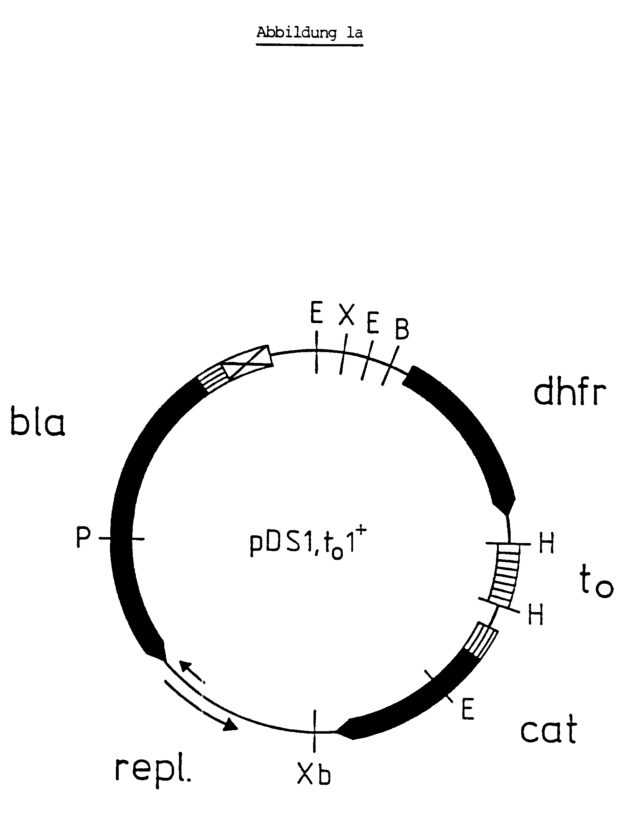

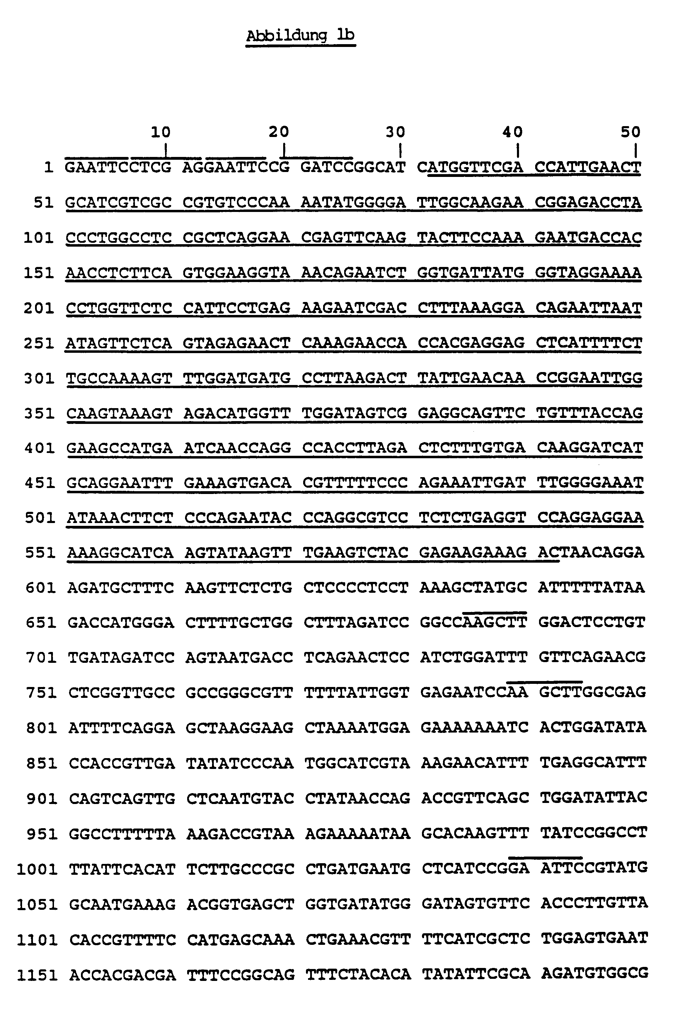

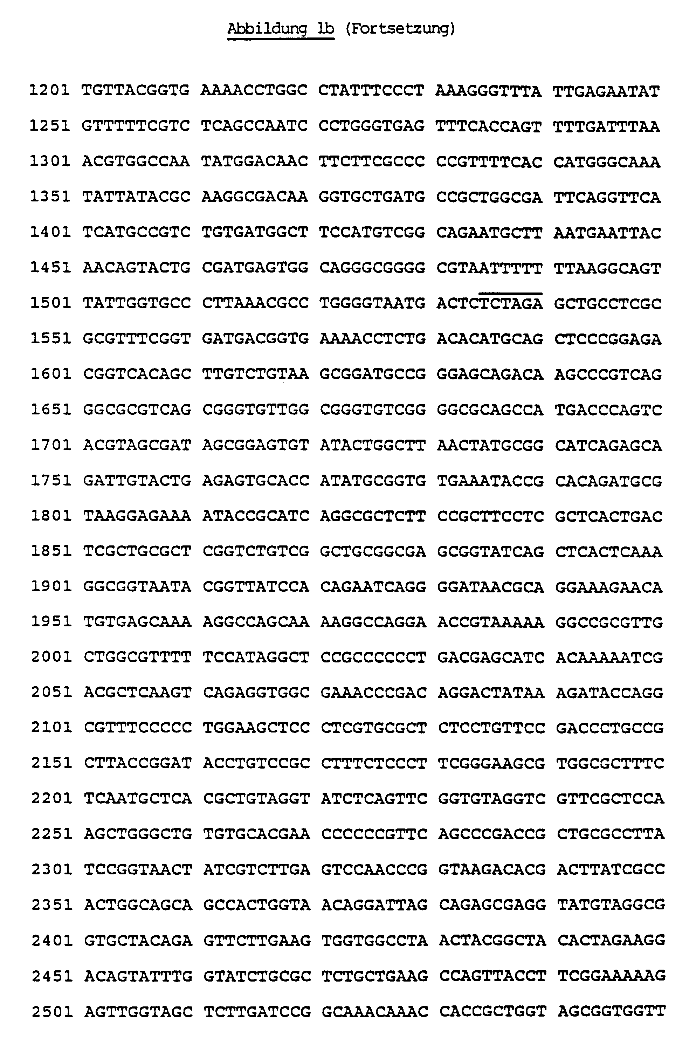

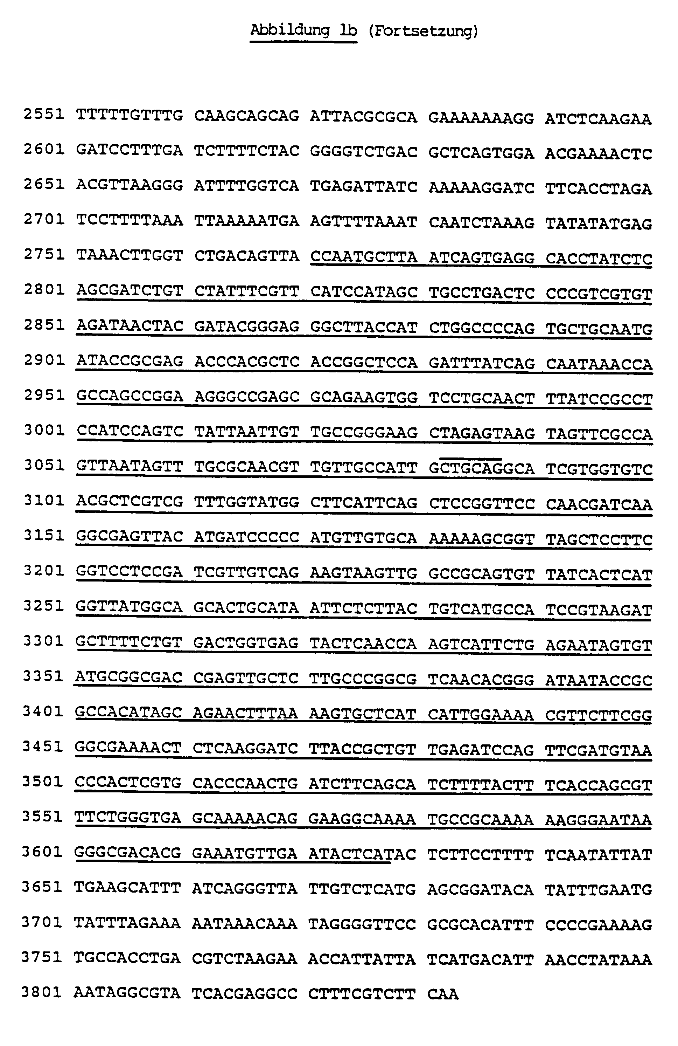

Schematische Darstellung sowie Nukleotidsequenz des Plasmids pDS1,to1⁺. In Abb.1a ist das Plasmid schematisch dargestellt. In der Nukleotidsequenz (Abb.1b) sind die Erkennungsstellen für die in der schematischen Darstellung aufgeführten Restriktionsendonukleasen überstrichen, während die für β-Lactamase (bla) bzw. Dihydrofolatreduktase (dhfr) kodierenden Regionen unterstrichen sind.

Abbildung 2

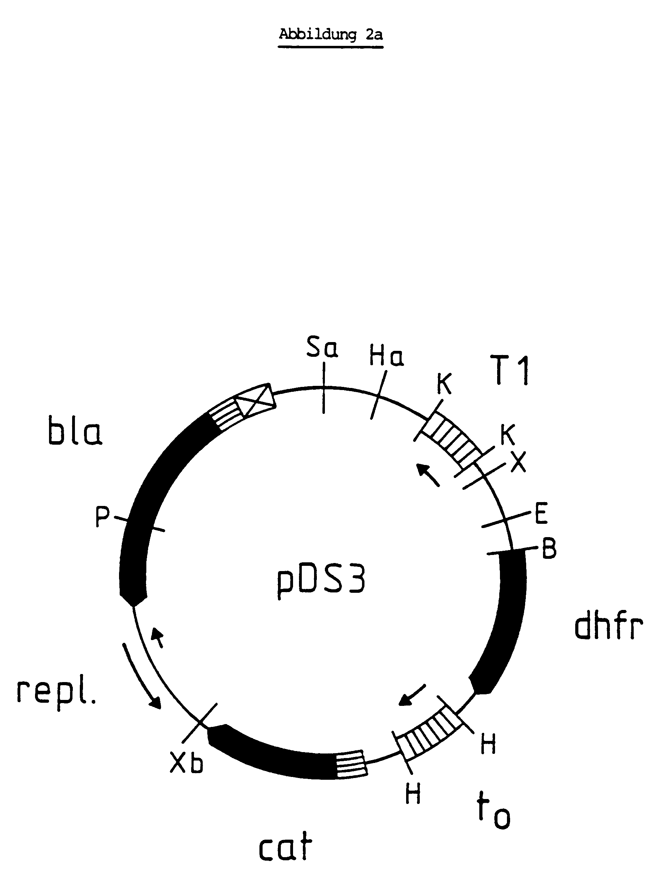

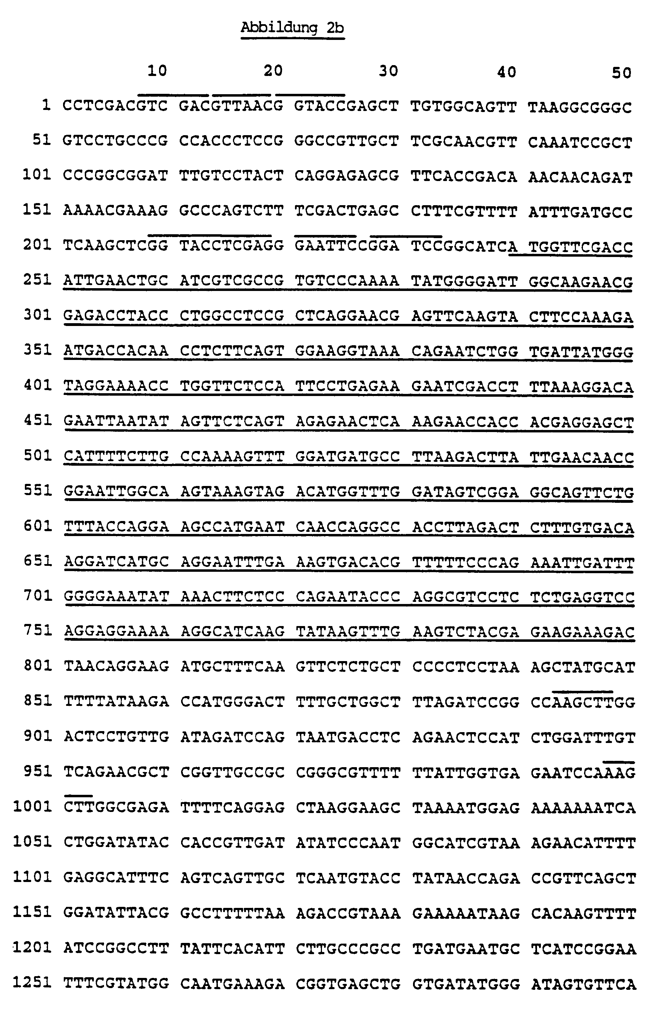

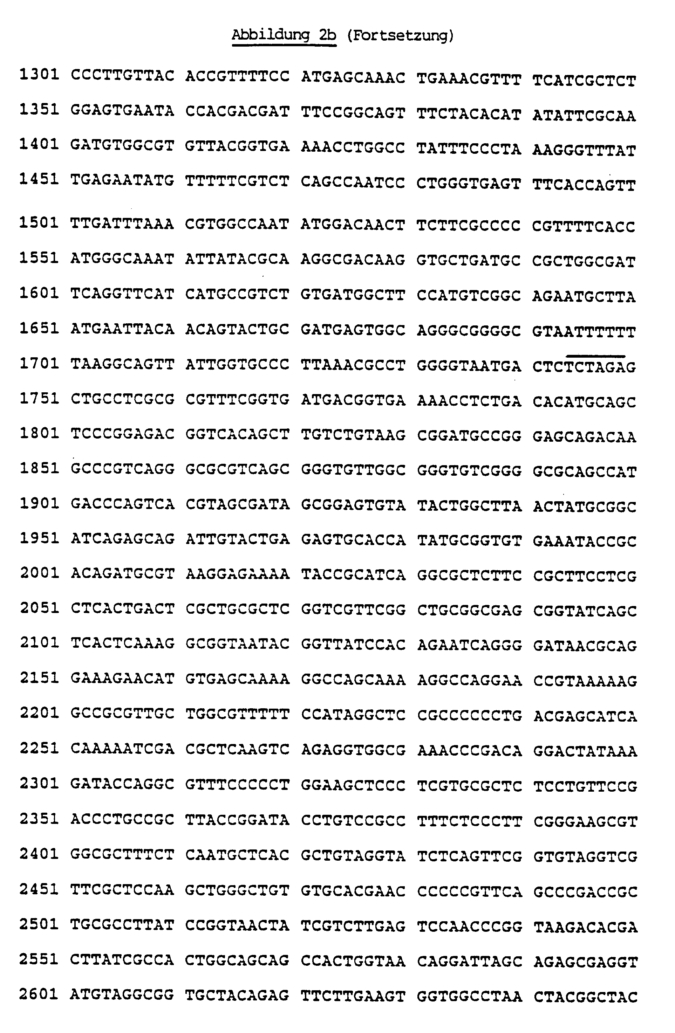

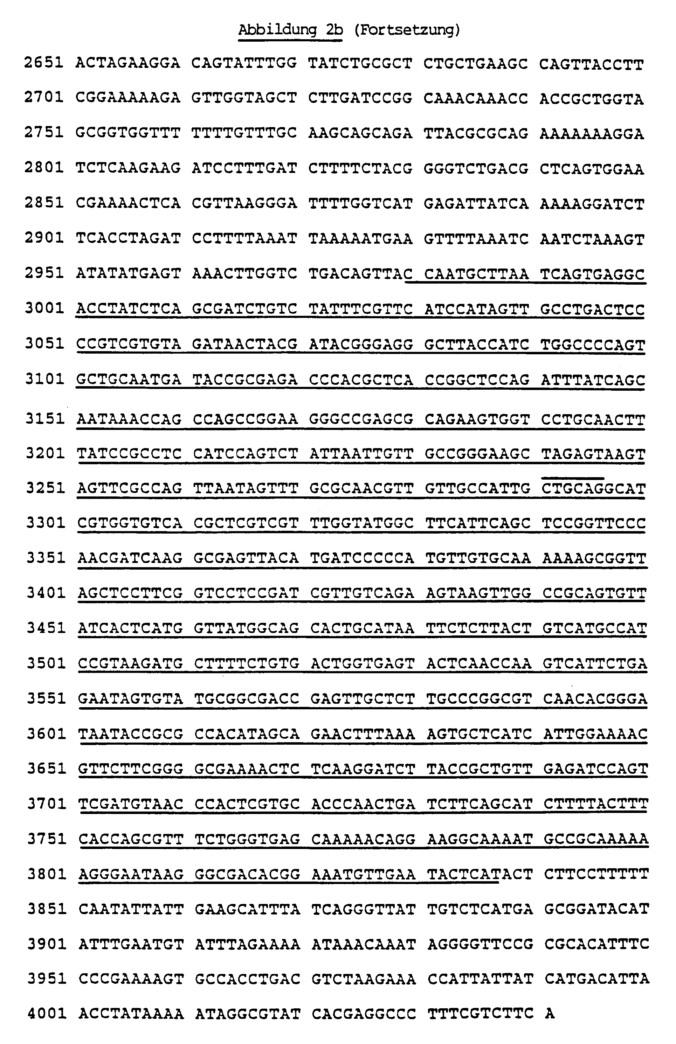

Schematische Darstellung sowie Nukleotidsequenz des Plasmids pDS3. In Abb.2a ist das Plasmid schematisch dargestellt. In der Nukleotidsequenz (Abb.2b) sind die Erkennungsstellen für die in der schematischen Darstellung aufgeführten Restriktionsendonukleasen überstrichen, während die für β-Lactamase (bla) bzw. Dihydrofolatreduktase (dhfr) kodierenden Regionen unterstrichen sind.

Abbildung 3

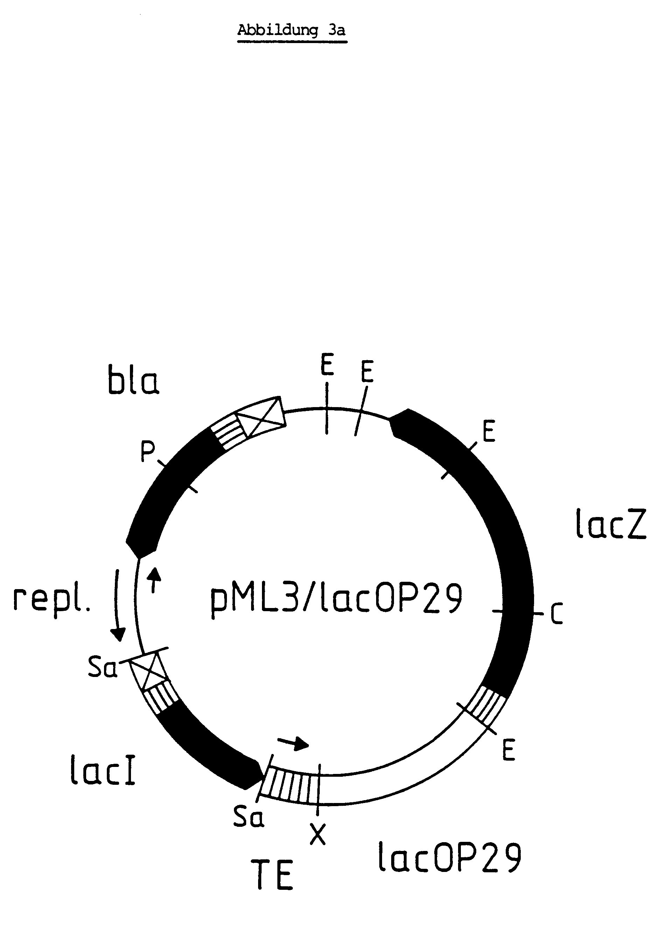

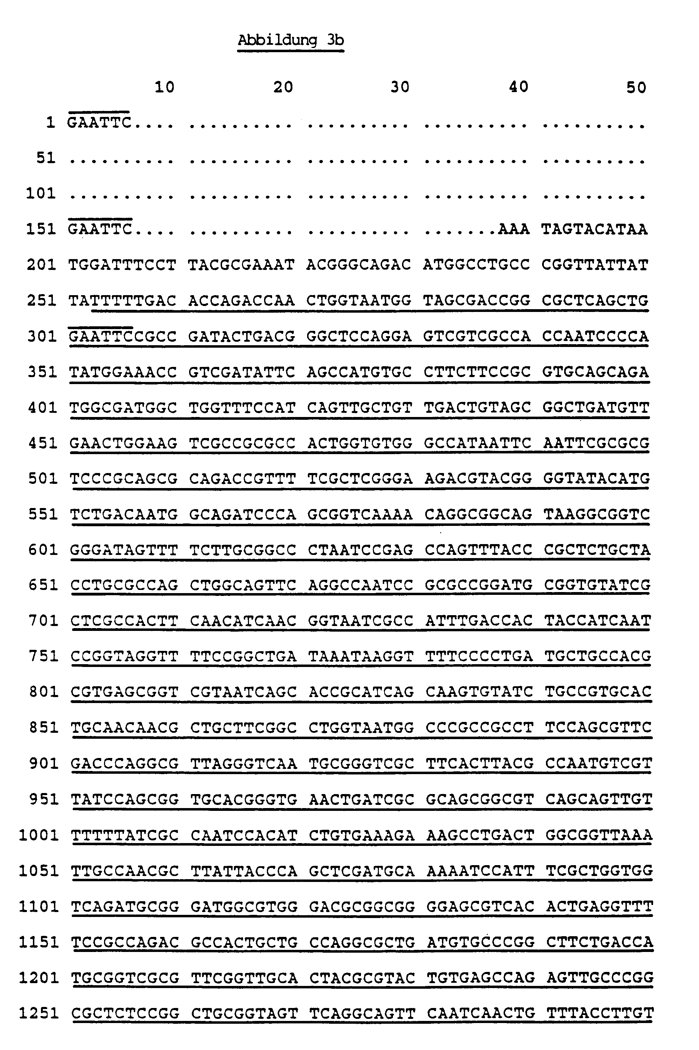

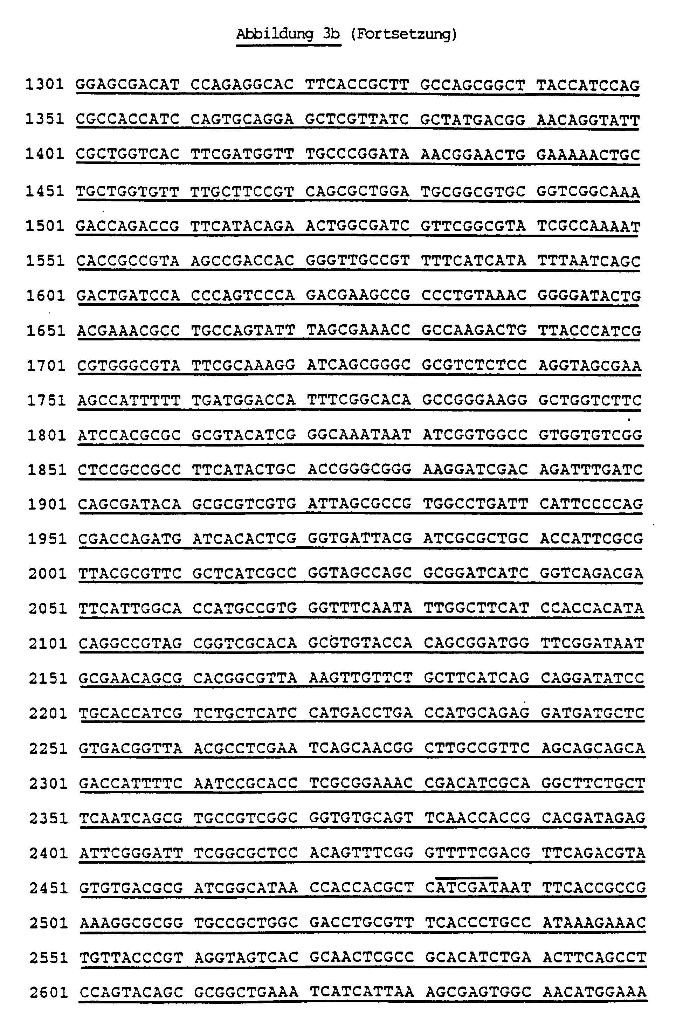

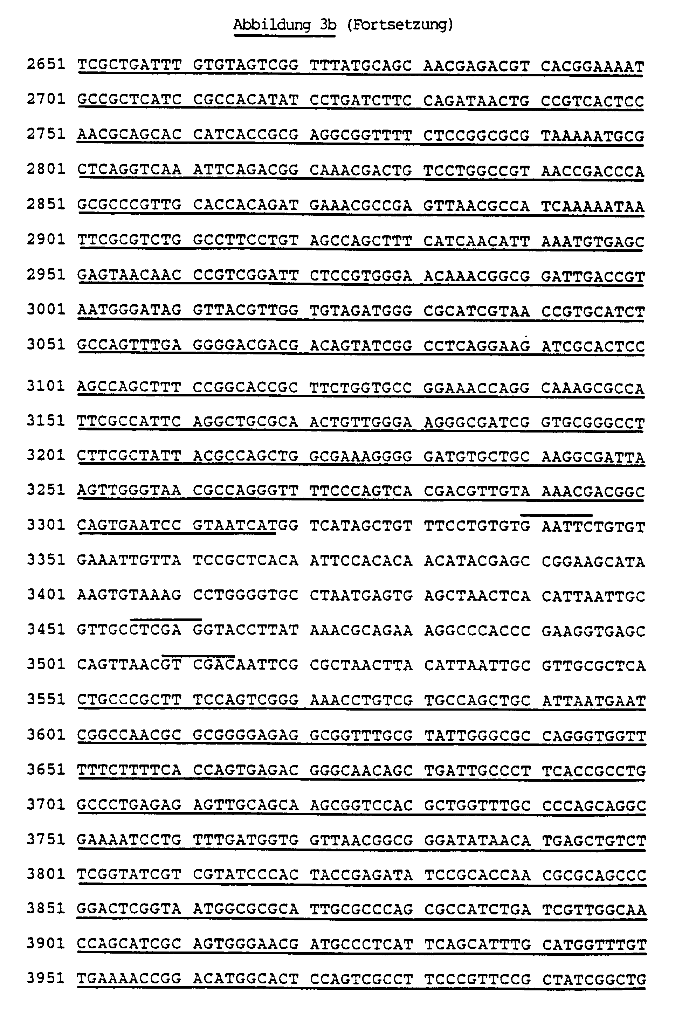

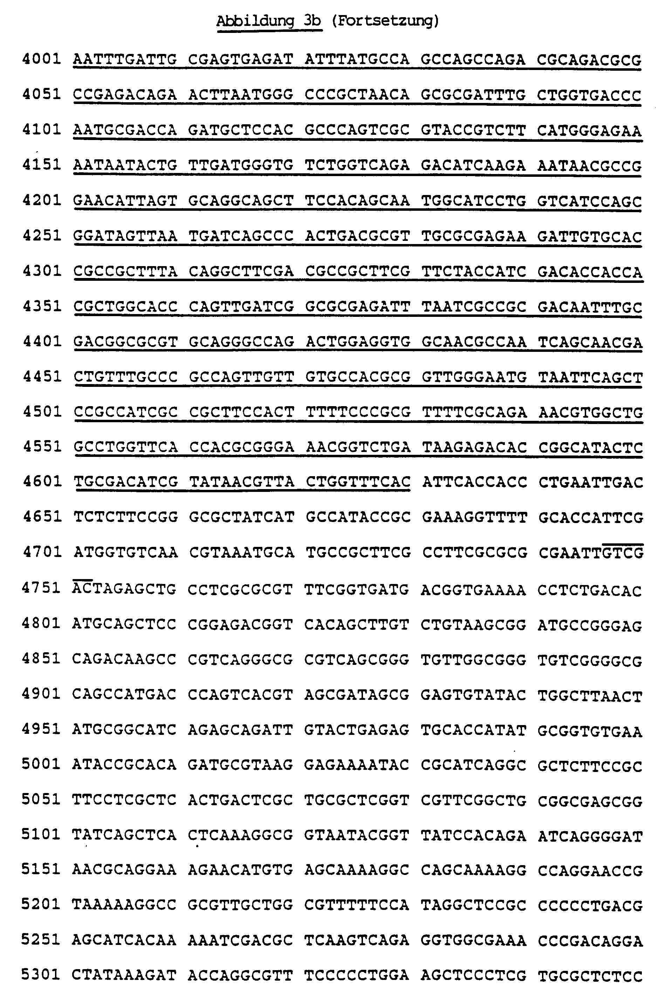

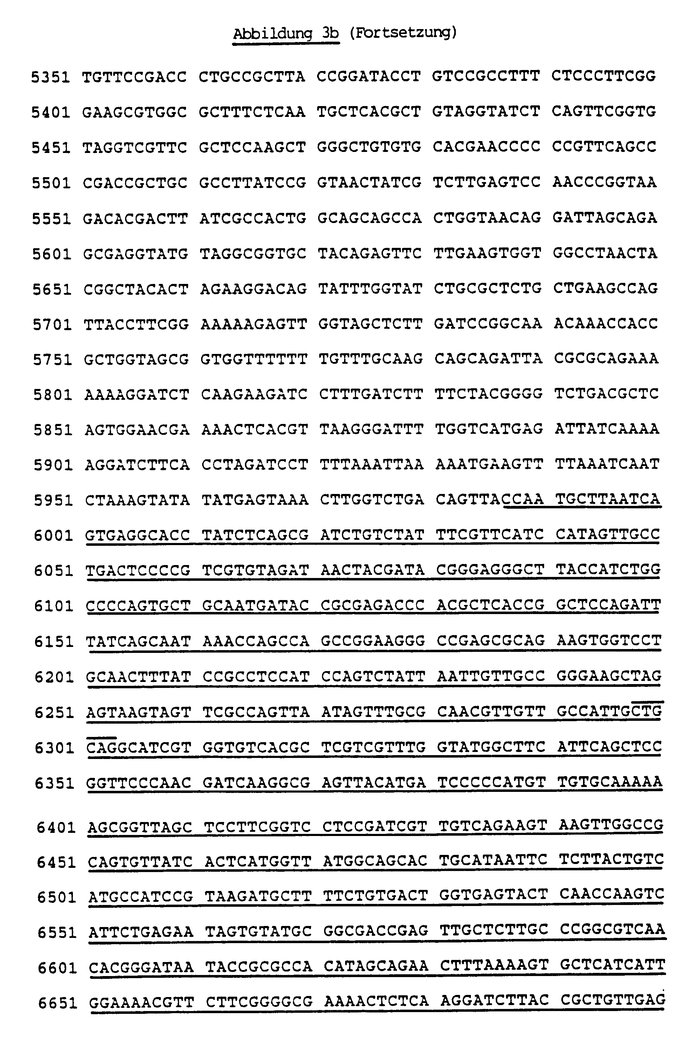

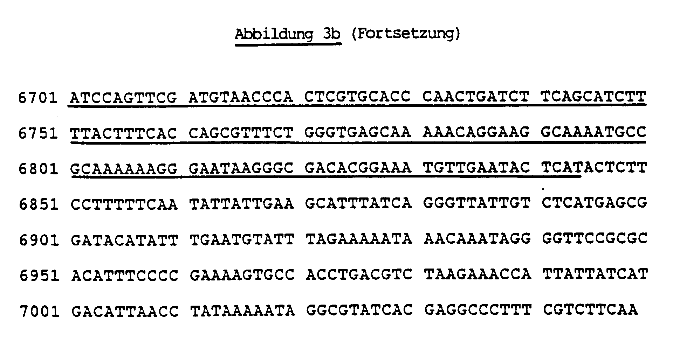

Schematische Darstellung sowie Nukleotidsequenz des Plasmids pML3/lacOP29. In Abb.3a ist das Plasmid schematisch dargestellt. In der Nukleotidsequenz (Abb.3b), die soweit bekannt angegeben ist, sind die Erkennungsstellen für die in der schematischen Darstellung aufgeführten Restriktionsendonukleasen überstrichen, während die für β-Lactamase (bla), lac-Repressor (lacI) bzw. β-Galaktosidase (lacZ) kodierenden Regionen unterstrichen sind.

Abbildung 4

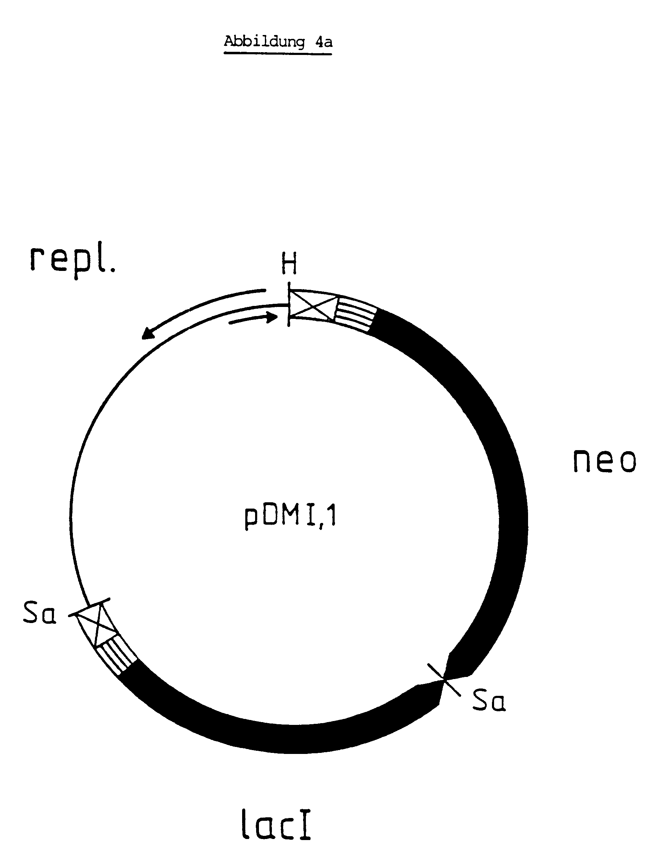

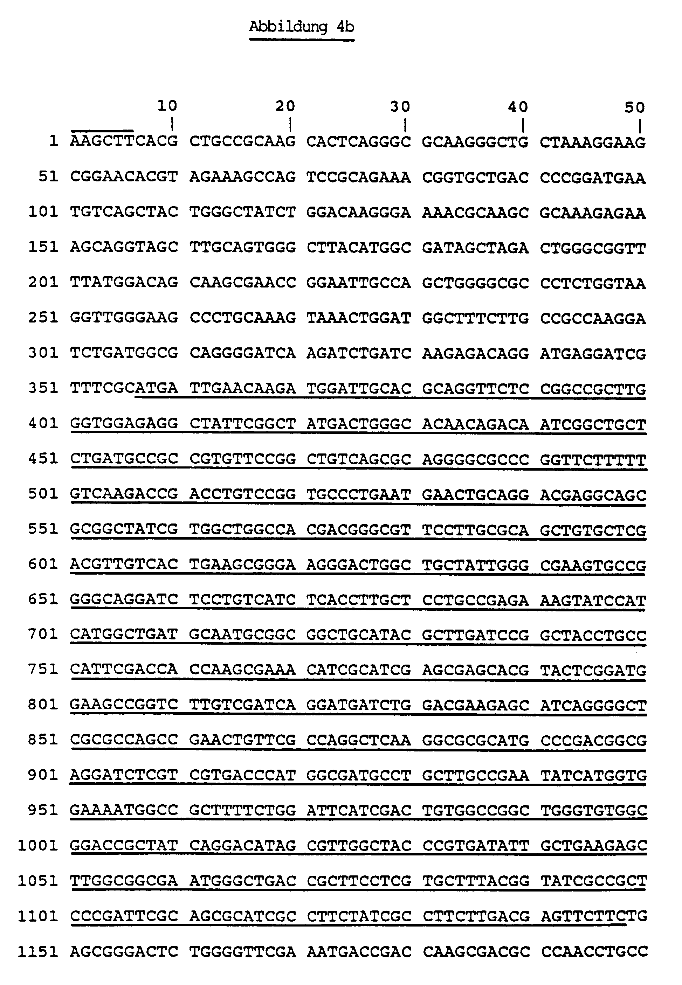

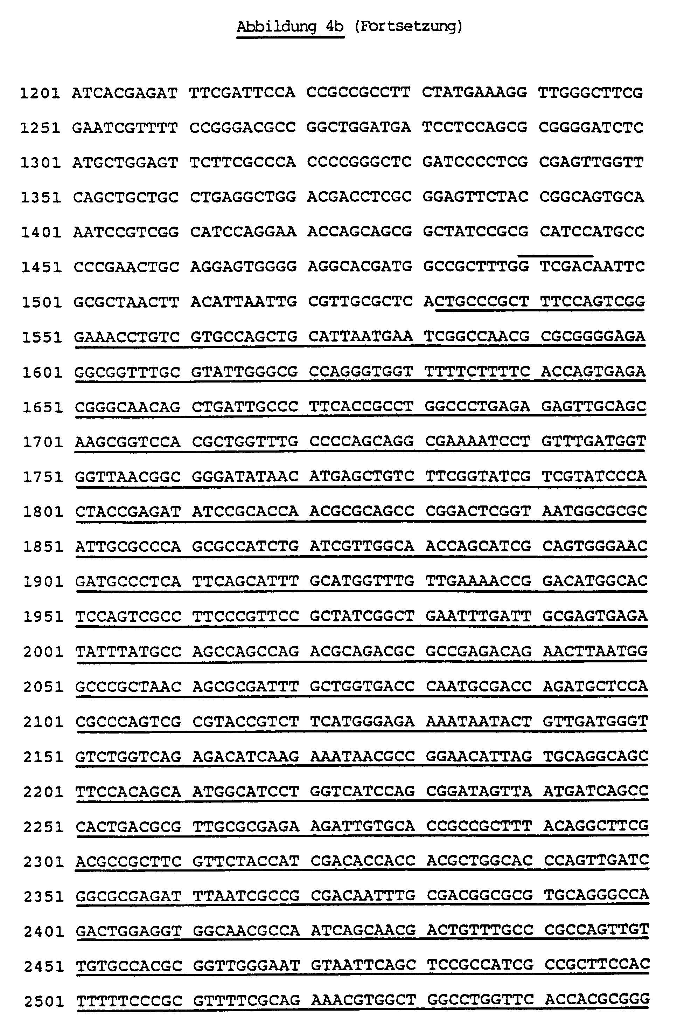

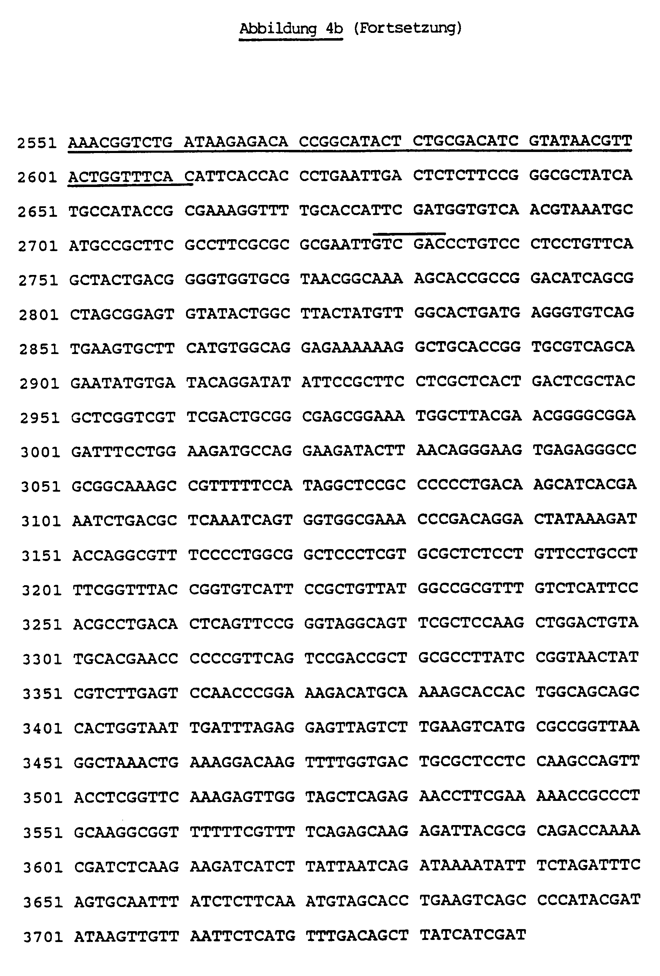

Schematische Darstellung sowie Nukleotidsequenz des Plasmids pDMI,1. In Abb.4a ist das Plasmid schematisch dargestellt. In der Nukleotidsequenz (Abb.4b) sind die Erkennungsstellen für die in der schematischen Darstellung aufgeführten Restriktionsendonukleasen überstrichen, während die für Neomycinphosphotransferase (neo) bzw. lac-Repressor (lacI) kodierenden Regionen unterstrichen sind.

Abbildung 5

Nukleotidsequenzen der XhoI-EcoRI Fragmente mit den Expressionskontrollsequenzen lacOP29(a), tacOP29(b), N25OPSN25OP29(c), A1OPSA1(d), A1OPSCONA1(e), A1pOPSA1(f), OPUA1(g), OPUA1CON-(h), A10PSA10P21(i) bzw. A10PSA10P29(j). In diesen Sequenzen sind das Nukleotid, an dem die RNA-Synthese startet (+1), sowie die Regionen bei den Positionen -10 und -35 unterstrichen, während lac-Operator Sequenzen überstrichen sind.

Abbildung 6

Nukleotidsequenz des EcoRI-Fragments mit dem Promotor $P_{N25}$. In der Sequenz sind das Nukleotid, an dem die RNA-Synthese startet, sowie die Regionen bei den Positionen -10 und -35 unterstrichen.

Abbildung 7

Nukleotidsequenzen der XhoI-EcoRI Fragmente mit den Expressionskontrollsequenzen N25*/0 bzw. N25OPSN25. In den Sequenzen sind jeweils das Nukleotid, an dem die RNA-Synthese startet, sowie die Regionen bei den Positionen -10 und -35 unterstrichen, während lac-Operator Sequenzen überstrichen sind.

Abbildung 8

Nukleotidsequenz des XhoI-EcoRI Fragments mit dem Promoter $P_{AI}$. In der Sequenz sind das Nukleotid, an dem die RNA-Synthese startert (Position +1), sowie die Regionen um die Positionen -10 und -35 unterstrichen.

Abbildung 9

Nukleotidsequenz des SalI-EcoRI Fragments mit der Expressionskontrollsequenz OPA1OPSA1. In der Sequenz sind das Nukleotid, an dem die RNA-Synthese startet, sowie die Regionen um die Positionen -10 und -35 unterstrichen, während lac-Operator Sequenzen überstrichen sind.

Abbildung 10

Schematische Darstellung des Plasmids pDS1/$P_{N25}$,to1$^+$. Vom Promotor $P_{N25}$ aus wird im Uhrzeigersinn transkribiert.

Abbildung 11

Schematische Darstellung der Konstruktion des Plasmids pDS3/A1OPSA1. Von der Expressionskontrollsequenz A1OPSA1 aus wird im Uhrzeigersinn transkribiert.

Abbildung 12

Schematische Darstellung der Konstruktion des Plasmids pDS3/OPA1OPSA1. Die Nukleotidsequenz des SalI-EcoRI Fragments mit der Expressionskontrollsequenz OPA1OPSA1, von der aus im Uhrzeigersinn transkribiert wird, ist in Abb.9 aufgeführt.

Abbildung 13

Schematische Darstellung der Konstruktion des Plasmids pML3/A1OPSA1. Bei dieser Konstruktion wurde das XhoI-EcoRI Fragment des Plasmids pML3/lacOP29 mit der Expressionskontrollsequenz lacOP29 durch das entsprechende XhoI-EcoRI Fragment mit der Expressionskontrollsequenz A1OPSA1 ersetzt.

Abbildung 14

Schematische Darstellung der Konstruktion des Plasmids pML3/OPA1OPSA1. Zur Konstruktion dieses Plasmids wurden drei DNA-Fragmente eingesetzt: das SalI-EcoRI Fragment aus pDS3/OPA1OPSA1 mit der Expressionskontrollsequenz OPA1OPSA1, das EcoRI-ClaI Fragment aus pML3/lacOP29 mit Teilen des lacZ-Gens und das grössere ClaI-XhoI Fragment aus pML3/lacOP29. Bei dieser Konstruktion wurden Schnittstellen für SalI und XhoI miteinander verknüpft, wobei beide Schnittstellen zerstört wurden.

Abbildung 15

Zeitabhängiger Verlauf der Komplexbildung von RNAP und Promoter $P_{N25}$ bei einer Konzentration an aktiver RNAP von 0.42nM und einer Promotorkonzentration von 0.02nM.

Abbildung 16

Graphische Darstellung des Ausdruckes $-\ln[(A_o-X)/A_o]$ in Abhängigkeit von der Reaktionszeit bei einer Konzentration an aktiver RNAP von 0.42nM und einer Konzentration an Promotor $P_{N25}$ von 0.02nM.

Abbildung 17

Graphische Darstellung des zeitabhängigen Verlaufs der Komplexbildung von RNAP und Promotor $P_{N25}$ bei einer Promotorkonzentration von 0.02nM und einer Konzentration an aktiver RNAP von 0.32nM (Versuch 2), bzw. 0.15nM (Versuch 3).

Abbildung 18

Graphische Darstellung des Ausdrucks $-\ln[(A_o-X)/A_o]$ in Abhängigkeit von der Reaktionszeit bei einer Promotorkonzentration von 0.02nM und einer Konzentration an aktiver RNAP von 0.32nM (Versuch 2), bzw. 0.15nM (Versuch 3).

Abbildung 19

Bestimmung der Komplexbildungsrate für den Promotor $P_{AI}$ mit dem Promotor $P_{N25}$ als internem Standard: -ln[($B_o$-Y)/$B_o$] aufgetragen gegen -ln[($A_o$-X)/$A_o$].

Abbildung 20

Bestimmung der Komplexbildungsrate für die Expressionskontrolsequenz A1OPSA1 mit dem Promotor $P_{AI}$ als internem Standard: -ln[($B_o$-Y)/$B_o$] aufgetragen gegen -ln[($A_o$-X)/$A_o$].

Abbildung 21

Bestimmung des Faktors zur Umrechnung von β-Galaktosidase-Einheiten in $P_{bla}$-Einheiten: β-Galaktosidase-Einheiten unter Kontrolle er Expressionskontrollsequenzen tacOP29, N23OP29, OPUA1 und OPUA1CON aufgetragen gegen die entsprechenden $P_{bla}$-Einheiten.

Beispiel 1

Beschreibung der verwendeten Plasmide

A. Prinzipien

Die Plasmide pDS1,$t_o1^+$ (Abb. 1), pDS3 (Abb. 2), pML3/lacOP29 (Abb. 3) und pDMI,1 (Abb. 4) wurden zur Herstellung und Charakterisierung der Eigenschaften der Expressionskontrollsequenzen eingesetzt. Mit diesen Plasmiden transformierte E. coli Zellen wurden bei der Deutschen Sammlung von Mikroorganismen (DSM) in Göttingen am 11.12.1984 [E. coli M15 (pDS1,$t_o1^+$), DSM-Nr. 3135], bzw. am 3.10.1985 [E. coli M15 (pDS5 / RBSII, 3A + 5A; pDMI,1), DSM-Nr. 3517], bzw. am 5.8.1987 [E. coli M15 (pDS3), DSM-Nr. 4198; E. coli M15 (pML3/lacOP29), DSM-Nr. 4199], nach dem Budapester Vertrag hinterlegt.

B. Das Plasmid pDS1,$t_o1^+$

Der Anteil von pDS1,$t_o1^+$ (Abb. 1), der zwischen den Schnittstellen für die Restriktionsendonukleasen XbaI und EcoRI liegt und die Replikationsregion sowie das Gen für β-Lactamase, das den Zellen Ampicillinresistenz verleiht, enthält, stammt aus dem Plasmid pBR322 (Bolivar et al., Gene 2, 95-113 [1977]; Sutcliffe, Cold Spring Harlor Symp. Quant. Biol. 43, 77-90 [1979]). Der verbleibende Teil des Plasmids trägt Schnittstellen für die Restriktionsendonukleasen XhoI, EcoRI und BamHI gefolgt vom Gen der Dihydrofolatreduktase der Maus-Zellinie AT-3000 (Chang et al., Nature 275, 617-624 [1978]; Masters et al., Gene 21, 59-63 [1983]), dem Terminator $t_o$ des E. coli-Phagen Lambda (Schwarz et al., Nature 272, 410-414 [1978]) und dem Promotor-freien Gen der Chloramphenicolacetyltransferase (Marcoli et al., FEBS Letters, 110, 11-14 [1980]).

C. Das Plasmid pDS3

Das Plasmid pDS3 (Abb. 2) unterscheidet sich vom Plasmid pDS1,$t_o1^+$ einerseits in einer Region, die neben Schnittstellen für verschiedene Restriktionsendonukleasen den Terminator T1 des E. coli rrnB Operons (Brosius et al., J. Mol. Biol. 148, 107-127 [1981]) trägt, und andererseits durch das Fehlen der Schnittstelle für die Restriktionsendonuklease EcoRI im Gen für die Chloramphenicolacetyltransferase.

D. Das Plasmid pML3/lacOP29

Der Anteil von pML3/lacOP29 (Abb. 3), der zwischen den Schnittstellen für die Restriktionsendonukleasen SalI und EcoRI liegt und die Replikationsregion sowie das Gen für β-Lactamase enthält, stammt aus dem Plasmid pBR322 (Bolivar et al., supra; Sutcliffe, supra). Der verbleibende Teil des Plasmids trägt neben dem vollständigen lac I-Gen (Farabough, Nature 274,765-769 [1978]), das den lac-Repressor kodiert, den Terminator $T_E$ des E. coli Phagen T7 (Dunn und Studier, J. Mol. Biol., 166, 477-535 [1983]), die Expressionskontrollsequenz lacOP29 (siehe Beispiel 2) und das Promotor-freie Gen für die β-Galaktosidase (Kalnins et al., EMBO J. 2, 593-597 [1983]).

E. Das Plasmid pDMI,1

Das Plasmid pDMI,1 (Abb. 4) trägt das Gen der Neomycinphosphotransferase aus dem Transposon Tn5 (Beck et al., Gene 19, 327-336 [1982]), das E. coli-Zellen Kanamycinrestistenz verleiht und das lac I-Gen (Farabough, supra) mit der Promotormutation $I^q$ (Calos, Nature 274, 762-765 [1978]), das den lac-Repressor kodiert. Ausserdem enthält das Plasmid pDMI,1 eine Region des Plasmids pACYC184 (Chang und Cohen, J. Bacteriol. 134, 1141-1156 [1978]), die alle Informationen enthält, die für die Replikation und stabile Weitergabe an die Tochterzellen notwendig sind. Das plasmid pDMI,1 ist kompatibel mit den oben beschriebenen Plasmiden un ihren Derivaten.

Beispiel 2

Herstellung und Klonierung der Expressionskontrollsequenzen

A. Prinzipien

Nach Herstellung der Expressionskontrollsequenzen wurden sie zur Charakterisierung ihrer Eigenschaften in geeignete Plasmide integriert.

B. Herstellung der Expressionskontrollsequenzen

1. Expressionskontrollsequenzen lacOP29, tacOP29, A1OPSCONA1, OPUA1, OPUA1CON, N25OPSN25OP29, $P_{AI}$, A1OPSA1, A1pOPSA1, A10PSA10P21 und A10PSA10P29.

Zur Herstellung dieser Expressionskontrollsequenzen wurden zunächst einzelsträngige DNA-Fragmente chemisch synthetisiert (Bannwarth und Iaiza, DNA 5, 413-419 [1986]). Diese Fragmente wurden dann nach in der Literatur beschriebenen Methoden (Maniatis et al., supra) miteinander hybridisiert und ligiert. Die Sequenzen der so erhaltenen doppelsträngigen XhoI-EcoRI Fragmente mit den entsprechenden Expressionskontrollsequenzen sind in Abb. 5, bzw. Abb. 8 aufgeführt.

2. Expressionskontrollsequenzen $P_{N25}$, N25*/0 und N25OP29

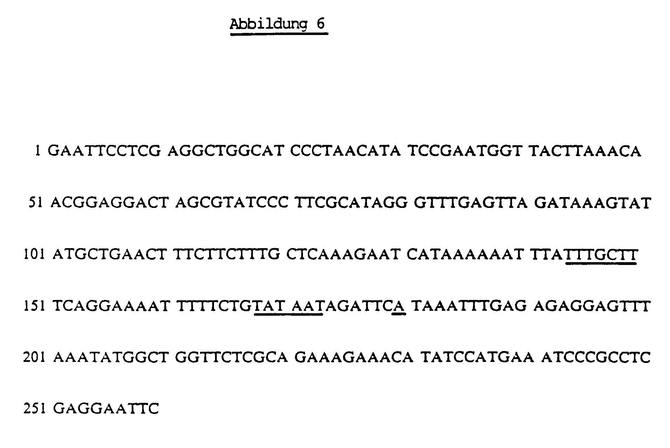

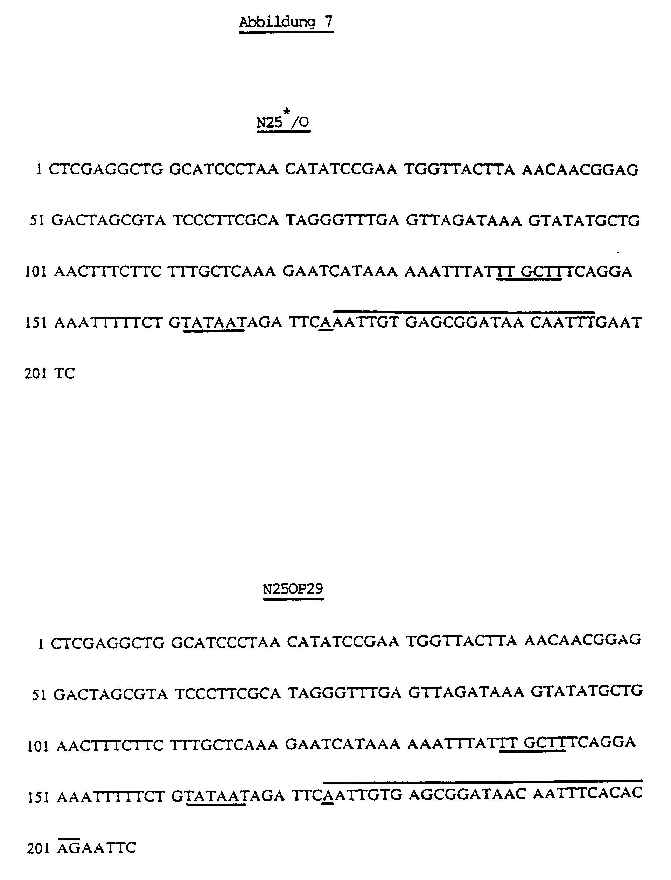

Die Expressionskontrollsequenzen $P_{N25}$, N25*/0 und N25OP29 (Abb. 6 und Abb. 7) können wie im obigen Abschnitt beschrieben, hergestellt werden.

3. Die Expressionskontrollsequenz OPA1OPSA1

Die Sequenz der Expressionskontrollsequenz OPA1OPSA1 ist in Abb. 9 dargestellt. Ihre Herstellung ist in Abschnitt D beschrieben.

C. Integration des Promotors $P_{N25}$ in das Plasmid pDS1, $t_o1^+$

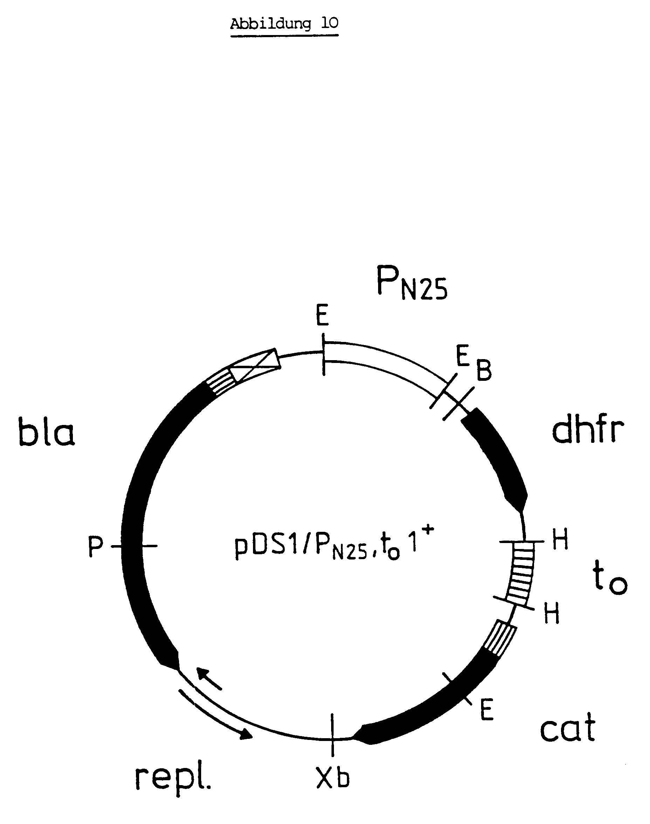

Der Promotor $P_{N25}$ wurde als Teil eines EcoRI-Fragments (siehe Abb. 6) nach in der Literatur beschriebenen Methoden (Maniatis et al., supra) in das Plasmid pDS1, $t_o1^+$ (Abb. 1) integriert, wodurch das Plasmid pDS1/$P_{N25}$, $t_o1^+$ erhalten wurde (Abb. 10). Dieses Plasmid wurde als Quelle zur Gewinnung des Promotors $P_{N25}$ in grösserer Menge verwendet.

D. Integration der Expressionskontrollsequenzen in das Plasmid pDS3

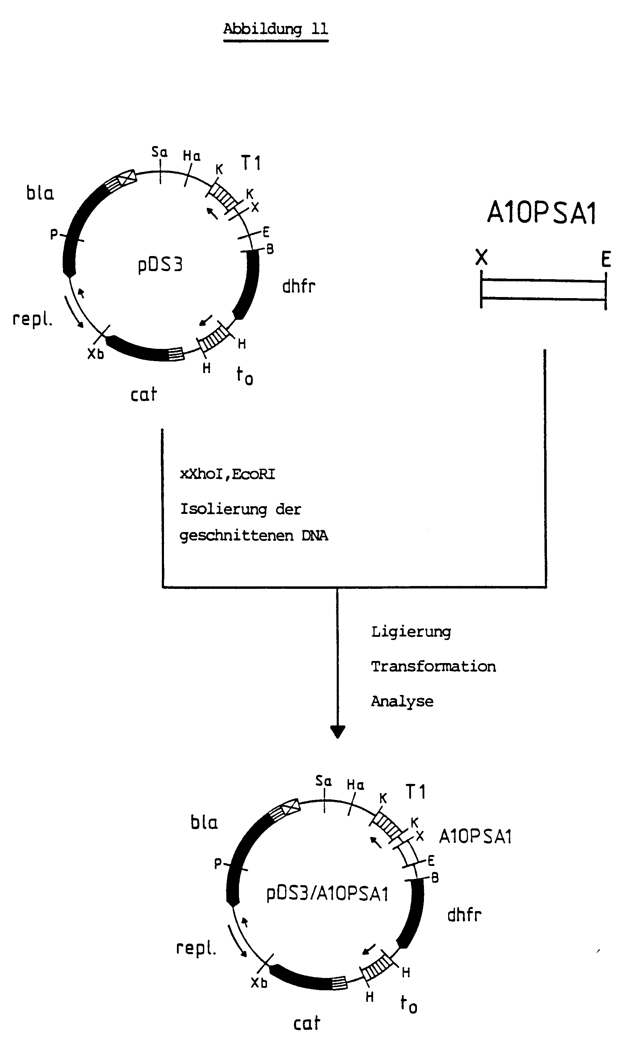

In aus der Literatur bekannter Weise (Maniatis et al., supra) wurden die Expressionskontrollsequenzen lacOP29, tacOP29, OPUA1, OPUA1CON, $P_{N25}$, N25*/0, N25OP29, N25OPSN25OP29, $P_{AI}$, A1OPSA1, A10PSA10P21, A10PSA10P29, A1OPSCONA1 und A1pOPSA1 in das Plasmid pDS3 (Abb. 2) integriert. Eine solche Klonierung ist exemplarisch am Beispiel der Expressionskontrollsequenz A1OPSA1 in Abbildung 11 schematisch dargestellt.

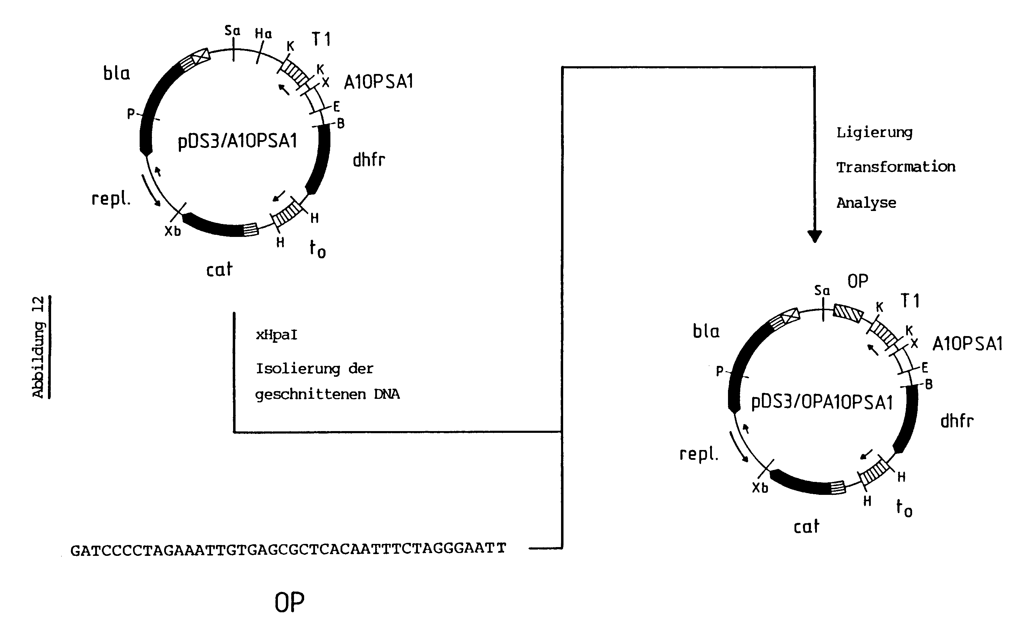

Die Expressionskontrollsequenz OPA1OPSA1 wurde durch Integration eines chemisch synthetisierten DNA-Fragments, das eine palindromische Sequenz des lac-Operators enthält, in die HpaI-Schnittstelle des Plasmids pDS3/A1OPSA1 (Abb. 11) hergestellt (Abb. 12).

Die pDS3-Derivate mit den entsprechenden Expressionskontrollsequenzen wurden sowohl zur Bestimmung der Komplexbildungsraten zwischen Promotor und RNAP als auch zur Bestimmung der Promotorstärken der einzelnen Expressionskontrollsequenzen eingesetzt.

E. Integration der Expressionskontrollsequenzen in das Plasmid pML3/lacOP29

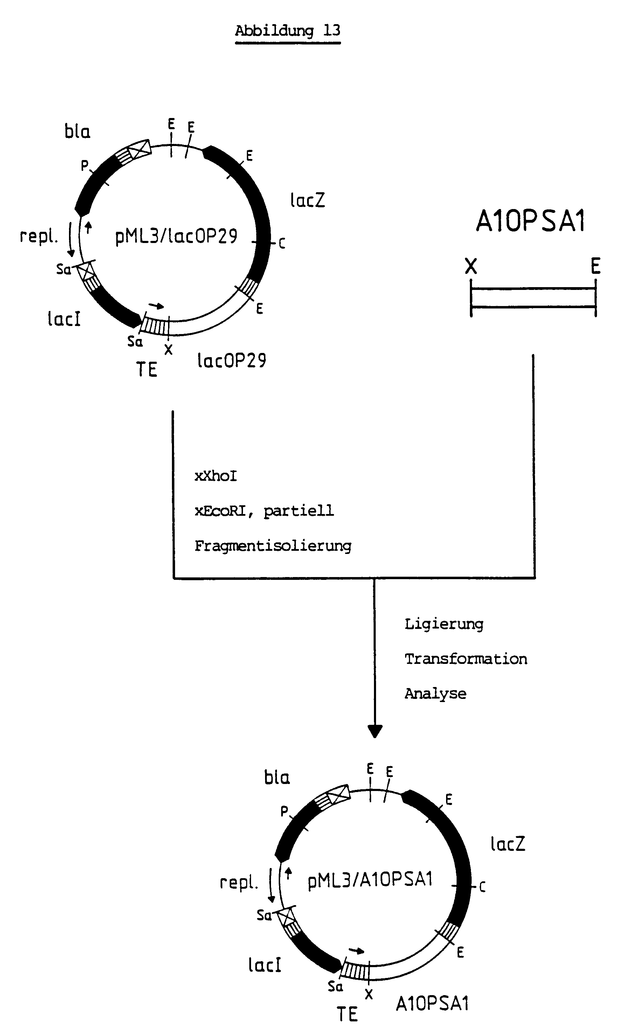

Das Plasmid pML3/lacOP29 (Abb. 3) trägt zwischen den Schnittstellen für die Restriktionsendonukleasen XhoI und EcoRI die Expressionskontrollsequenz lacOP29. Nach in der Literatur beschriebenen Methoden (Maniatis et al., supra) wurden Derivate dieses Plasmids hergestellt (Abb. 13), in denen lacOP29 durch jeweils eine der Expressionskontrollsequenzen tacOP29, N25OP29, N25OPSN25OP29, A1OPSA1, A10PSA10P21, A10PSA10P29, A1OPSCONA1, A1pOPSA1, OPUA1, bzw. OPUA1CON ersetzt wurde.

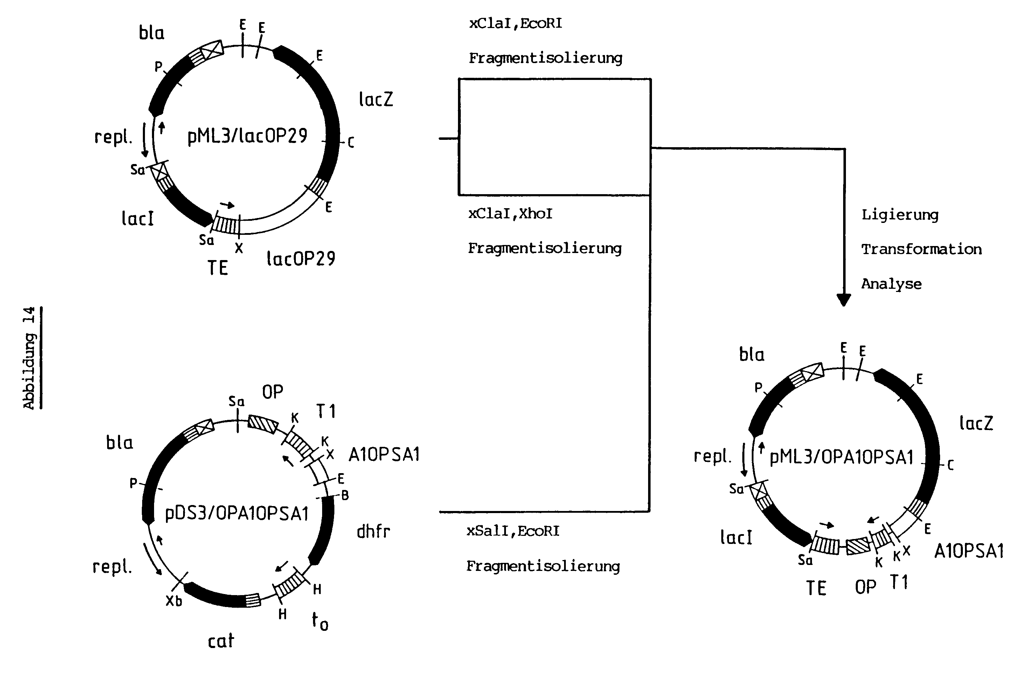

Das Plasmid pML3/OPA1OPSA1 mit der Expressionskontrollsequenz OPA1OPSA1 wurde, wie in Abbildung 14 schematisch beschrieben, hergestellt.

Die erhaltenen pML3-Derivate mit den entsprechenden Expressionskontrollsequenzen wurden zur Bestimmung der in vivo Promotorstärken unter reprimierten Bedingungen eingesetzt.

Beispiel 3

Bestimmung der Signalstärke

A. Prinzipien

Zur Bestimmung der Signalstärke der Promotoren bzw. Promotor/Operator-Elementen wurde zunächst die Komplexbildungsrate ($K_{ass}$) zwischen der E.coli RNA-Polymerase (RNAP) und dem Promotor $P_{N25}$ absolut bestimmt. Anschliessend wurden die $K_{ass}$-Werte für die übrigen Signale über Relativmessungen mit dem Promotor $P_{N25}$ als internem Standard ermittelt.

B. Bestimmung von $K_{ass}$ für RNAP und Promotor $P_{N25}$

Die $K_{ass}$ für RNAP und Promotor $P_{N25}$ wurde mittels Filterbindungsversuchen bestimmt, mit deren Hilfe die Ausbildung von RNAP/Promotor-Komplexen in Abhängigkeit von der RNAP-Konzentration und der Reaktionszeit quantitativ bestimmt werden kann. Da sowohl RNAP als auch Promotoren biologisches Material darstellen, setzt die Auswertung solcher Versuche voraus, dass für beide Reaktanten die Konzentration der aktiven Moleküle bekannt ist. So muss z.B. für die RNAP nicht die Proteinkonzentration sondern die Konzentration der aktiven Polymerasemoleküle berücksichtigt werden. Aehnliches gilt für den Promotor $P_{N25}$ sowie die einzelsträngige fd-DNA, die als Kompetitor für unspezifische Bindung sowie zur Termination der Assoziationsreaktionen verwendet wurde. Demzufolge wird nachfolgend im Anschluss an das Kapitel über die theoretische Herleitung der Komplexbildungsraten ausführlich auf die Herstellung und Analyse aller Reaktanten eingegangen, bevor die eigentlichen Versuche zur Bestimmung von $K_{ass}$ beschrieben werden.

1. Theoretische Herleitung der $K_{ass}$

Für eine bimolekulare Reaktion zweiter Ordnung zwischen RNAP und Promotoren gilt das allgemeine Reaktionsschema

$$R + P \underset{K_{diss}}{\overset{K_{ass}}{\rightleftharpoons}} RP$$

worin

R die freie Konzentration der RNAP,
P die freie Konzentration der Promotoren,
RP die Konzentration der RNAP/Promotor-Komplexe,
$K_{ass}$ die Komplexbildungsrate, und
$K_{diss}$die Dissoziationskonstante darstellt.

Für den zeitlichen Verlauf der Reaktion gilt:

$dP/dt = -K_{ass} \times R \times P + K_{diss} \times RP$

Ist die Halbwertszeit der RNAP/Promotor-Komplexe gross -also die Dissoziationskonstante $K_{diss}$ klein - kann der Term $K_{diss}$ x RP vernachlässigt werden und es gilt für die zeitliche Aenderung der Promotorkonzentration:

$dP/dt = -K_{ass} \times R \times P$ (1)

R und P ändern sich mit der Zeit. Liegt aber die RNAP in hohem Ueberschuss vor, kann ihre Konzentration während des Ablaufs der Reaktion als konstant betrachtet werden (d.h. wenn R = konst. dann ist dR/dt = O) und es gilt:

$K_{ass} \times R = m$ (2)

Demnach gilt nun für die zeitliche Aenderung der Promotorkonzentration (siehe Gleichungen (1) und (2)):

$dP/dt = - m \times P$

bzw. ungeformt:

$-dP/P = m \times dt$

Nach Integration dieser Gleichung erhält man:

$- \ln P$ I $m \times t$ (3).

Gleichung (3) entspricht einer Reaktionsgleichung erster Ordnung, also formal dem "Zerfall von freiem Promotorfragment in RNAP/Promotor-Komplexe", mit der Geschwindigkeitskonstanten m [1/sec]. P ist hierbei der Anteil an freiem Promotor zum Zeitpunkt t (für t = O sec ist P = 1). Dieser Anteil kann experimentell bestimmt werden (siehe Abschnitt 6). Aus der Kenntnis von $A_0$ (Gesamtmenge der Promotoren) und von X (Menge von RNAP/Promotor-Komplexen zum Reaktionszeitpunkt t) kann die sogenannte "pseudo first order"-Konstante m mit Hilfe der Gleichung (3) berechnet werden:

$P = (A_0 - X)/A_0$

und man erhält

$m = - \ln[(A_0 - X)/A_0]/t$ (4)

Da die Ausbildung der Promotor/Operator-Komplexe eine bimolekulare Reaktion zweiter Ordnung darstellt, ist die Konstante m abhängig von der Konzentration der RNAP. Ist diese bekannt, so kann die Komplexbildungsrate $K_{ass}$ unter Berücksichtigung der Gleichungen

$m = K_{ass} \times R$ (2)

und

$m = - \ln[(A_0 - X)/A_0]/t$ (4)

wie folgt berechnet werden:

$K_{ass} \times R = - \ln[(A_0 - X)/A_0]/t$

bzw. umgeformt:

$K_{ass} = - \ln[(A_0 - X)/A_0]/(t \times R)$ (5)

Bei der Herleitung von $K_{ass}$ wurde angenommen, dass die Rückreaktion, d.h. der Zerfall von RNAP/Promotor-Komplexen, vernachlässigbar ist ($K_{diss}$ x RP = O). Ein solcher Zerfall ist eine Reaktion erster Ordnung und somit unabhängig von der Konzentration der Reaktanten. Die Assoziation ist hingegen konzentrationsabhängig. Deshalb muss zur Bestimmung von $K_{ass}$ die Konzentration der Reaktanten so hoch gewählt worden, dass sich der Assoziationsvorgang in einer Zeit abspielt, in welcher der Zerfall von Komplexen vernachlässigt werden kann. Dies setzt die Kenntnis über die Stabilität der RNAP/Promotor-Komplexe voraus.

Weiterhin wurde angenommen, dass während des Reaktionsverlaufs die Konzentration der freien RNAP als konstant betrachtet werden kann. Dazu muss die RNAP in hohem Ueberschuss relativ zu den Promotoren vorliegen, wobei zu berücksichtigen ist, dass die RNAP auch an unspezifisch DNA-Sequenzen binden kann. Die Anzahl solcher unspezifischen Bindungsstellen kann gering gehalten werden, in dem Promotoren auf kleinen, isolierten DNA-Fragmenten untersucht werden. Bei einem Ueberschuss von ungefähr 10 RNAP Molekülen pro Promotor kann die Konzentration der freien RNAP während des Reaktionsverlaufs als konstant betrachtet werden.

2. Herstellung der Probe mit dem Promotor $P_{N25}$

Das Plasmid pDS1/$P_{N25}$,$t_o1^+$ (Abb. 10) enthält den Promotor $P_{N25}$ auf einem 254 bp EcoRI-Fragment (Abb. 6). Dieses Plasmid wurde durch Integration des genannten EcoRI-Fragments in die EcoRI-Schnittstelle des Plasmids pDS1, $t_o1^+$ (Abb. 1), die der XhoI-Schnittstelle benachbart ist, konstruiert. Zur Reinigung des Promotorfragments wurde zunächst das Plasmid pDS1/$P_{N25}$,$t_o1^+$ gereinigt (Maniatis et al., supra) und 0.5 mg dieses Plasmids wurden dann mit Hilfe der Restriktionsendonuklease EcoRI nach Angaben des Herstellers geschnitten. Nach Phenolextraktion und Aethanolpräzipitation (Maniatis et al., supra) wurde die geschnittene DNA in TE-Puffer (10 mM Tris-HCl, 1 mM EDTA, pH 7.6) gelöst, und nach Zugabe von Probenpuffer in 6%-igen Polyacrylamidgelen elektrophoretisiert (Maniatis et al., supra). Anschliessend wurden die den Promotor $P_{N25}$ tragenden Fragmente mit einem Skalpell aus dem Gel ausgeschnitten, und auf DEAE-Papier (DE 81, Whatman, England) elektrophoretisiert. Nach dreimaligem Waschen mit Aethanol und TE-Puffer wurde das Papier an der Luft getrocknet, danach wurde die DNA mit einem Puffer enthaltend 10 mM Tris-HCl, 1 mM EDTA und 1.5 M NaCl, pH 7.6, eluiert. Nach einer 1:3 Verdünnung der resultierenden DNA-Lösung mit TE-Puffer wurde die DNA mit Aethanol präzipitiert, das Sediment mit 80%-igem Aethanol gewaschen und anschliessend die DNA in TE-Puffer gelöst.

Die Konzentration dieser DNA-Lösung wurde zunächst wie von Mahler et al. beschrieben [J. Mol. Biol. 9, 801-811 (1964)], spektrophotometrisch bestimmt. Ein Teil der DNA-Stammlösung wurde dann 1:30 in TE-Puffer verdünnt und anschliessend wurde die Extinktion dieser Verdünnung gegen TE-Puffer als Blindwert gemessen. Folgende Werte wurden erhalten:

$\Delta E_{260}$ = 0.108 $\Delta E_{260}/\Delta E_{280}$ = 1.86
$\Delta E_{280}$ = 0.058

Unter Berücksichtigung des Umrechnungsfaktors von 1 $\Delta E_{260}$ = 50 $\mu$g DNA/ml und des Verdünnungsfaktors ergab sich für die Stammlösung eine DNA-Konzentration von 162 $\mu$g/ml. Dieser Wert entspricht unter Berücksichtigung der Länge des Promotorfragments (254 bp) einer Konzentration von 0.96 pmol DNA-Fragment/$\mu$l. Das Verhältnis der Extinktionen bei 260 und 280 nm lässt Aussagen über die Reinheit der DNA-Lösung zu. Der erhaltene Wert für $\Delta E_{260}/\Delta E_{280}$ von 1.86 lässt auf eine hohe Reinheit der DNA-Lösung schliessen.

Die Konzentration der DNA-Lösung wurde zusätzlich durch den Vergleich dieser Lösung mit einer RNA-freien Lösung des Plasmids pDS1, $t_o1^+$ bekannter Konzentration ($\Delta$E-Messung) bestimmt. Dazu wurden je 0.09 pmol der das Promotorfragment enthaltenden DNA-Lösung mit 0.2, 0.1, 0.5 bzw. 0.025 pmol der geschnittenen pDS1, $t_o1^+$ Plasmid-DNA gemischt und in einem 6%-igen PAA-Gel charakterisiert (Maniatis et al., supra). Das erhaltene Pherogramm wurde densitometrisch ausgewertet, wobei für das Promotorfragment im Vergleich zur geschnittenen pDS1, $t_o1^+$ Plasmid-DNA eine Konzentration von 0.8 pmol/$\mu$l erhalten wurde.

Zur Bestimmung der Reinheit des Promotorfragments wurden zunächst 0.36 pmol dieses Fragments in einem PAA-Gel charakterisiert. Dabei konnte nach Anfärben mit Ethidiumbromid keine Verunreinigung nachgewiesen werden. Weiterhin wurde das Fragment mit $^{32}P$ radioaktiv markiert (siehe unten) und ebenfalls elektrophoretisch charakterisiert. Dabei konnte nach Autoradiographie nur das Promotorfragment nachgewiesen werden konnte.

Zusammenfassend kann festgestellt werden, dass das isolierte EcoRI-Fragment mit dem Promotor $P_{N25}$ nicht mit wesentlichen Mengen an RNA oder DNA verunreinigt war und in einer Konzentration von 0.88±0.08 pmol/µl vorlag. In den Versuchen zur Bestimmung der Konzentration der aktiven Polymerase (siehe unten) wurde als Promotorprobe eine Mischung aus dem isolierten EcoRI-Fragment mit dem Promotor $P_{N25}$ und aus dem entsprechenden, mit $^{32}P$ radioaktiv markierten Fragment eingesetzt. Diese radioaktiv markierte DNA wurde folgendermassen hergestellt: Zunächst wurde die DNA mit Hilfe von CIP (calf intestinal alkaline phosphatase) dephosphoryliert (Maniatis et al., supra) und dann mit Hilfe von T4-Polynukleotidkinase durch den Einbau von $^{32}P$ ($\gamma$-$^{32}P$ ATP, Amersham, 3000 Ci/mMol) markiert (Maniatis et al., supra). Anschliessend wurde das radioaktiv markierte EcoRI-Fragment mit dem Promotor $P_{N25}$ durch Chromatographie an Sephadex G75 (Pharmacia, Schweden) gereinigt. Zur Bestimmung der spezifischen Aktivität dieser Probe wurde einerseits die Radioaktivität vermessen (Cerenkow) und andererseits die DNA-Konzentration elektrophoretisch gegen das vorbehandelte, isolierte EcoRI-Fragment bestimmt (siehe oben). Durch Mischen des unbehandelten und des radioaktiv markierten EcoRI-Fragments wurden abschliessend Proben mit dem Promotor $P_{N25}$ von definierter Konzentration und spezifischer Aktivität erhalten.

3. Herstellung und Charakterisierung von einzelsträngiger M13mp8-DNA

Einzelsträngige M13mp8-DNA wurde sowohl als Kompetitor für die unspezifische Bindung von RNAP an DNA als auch zur Termination von Assoziationsreaktionen eingesetzt. Die Herstellung und Charakterisierung dieser DNA ist nachfolgend beschrieben. E.coli JM 101 Zellen (Maniatis et al., supra; GIBCO-BRL, Basel) wurden mit 0.01 pmol M13mp8 RFI-DNA (Pharmacia, Schweden) nach der von Morrison beschriebenen Methode [Methods of Enzymology 68, 326-331 (1979)] transformiert. Die Zellen wurden dann auf Indikatorplatten enthaltend Isopropylthiogalactosid (IPTG) und X-gal (5-bromo-4-chloro-3-indolyl-$\beta$-D-Galactosid) ausplattiert. Hierzu kann auf das Lehrbuch von J.H. Miller "Experiments in Molecular Genetics", Cold Spring Harbor Laboratory (1972)] und den Artikel von Messung u. Vieira in Gene 19, 269-276 (1982) verwiesen werden. Trübe Plaques mit transformierten E.coli JM 101 Zellen wurden aus der Top-Agar Schicht dieser Indikatorplatten mit einer Pasteurpipette ausgestochen, in 10 ml LB-Medium überführt und für 8 Stunden im Schüttelinkubator inkubiert (37°C, 220 Upm). Anschliessend wurden die Zellen abzentrifugiert und der Ueberstand enthaltend M13 mp8-Phagen zur Neuinfektion von E.coli JM 101 Zellen verwendet. Dazu wurden zunächst E.coli JM 101 Zellen in 500 ml M9-Minimalmedium (J.H. Miller, supra) bis zu einer $OD_{600} = 2$ aufgewachsen (37°C; 220 Upm) und danach wurde 1 ml M13mp8 Phagen enthaltender Ueberstand (siehe oben) zu diesen Zellen zugegeben. Nach weiteren 12 Stunden Inkubation bei 37°C wurden die Zellen abzentrifugiert. Die Phagen wurden aus dem Ueberstand mit 3% Polyäthylenglykol (PEG-6000) und 0.5 M NaCl ausgefällt und anschliessend abzentrifugiert. Das Sediment wurd in 20 ml TE-Puffer aufgenommen und bei 65°C jeweils zweimal mit Phenol (aequilibriert in 200 mM Tris HCl, pH 8) und Phenol/Chloroform (1:1) extrahiert.

Anschliessend wurde die so freigesetzte einzelsträngige M13 mp8-DNA mit Aethanol gefällt und in 1 ml TE-Puffer gelöst. Die Konzentration dieser DNA-Lösung wurde spektrophotometrisch bestimmt (Mahler et al., supra), wobei der Umrechnungsfaktor von 1 $OD_{260}$ = 36 µg einzelsträngige DNA/ml zugrunde gelegt wurde.

4. Bestimmung der Konzentration an aktiver Polymerase in RNAP-Lösungen

Zur Bestimmung der Konzentration von aktiver E.coli RNA-Polymerase in RNAP-Lösungen wurde nach Inkubation der RNAP in Gegenwart eines Ueberschusses von Promotorfragmenten der Anteil der gebildeten RNAP/Promotor-Komplexe mit Hilfe von Filterbindungsversuchen ermittelt. Aus diesem Anteil wurde dann unter Berücksichtigung der eingesetzten Menge an promotorfragmenten die Konzentration der gebundenen E.coli RNA-Polymerase berechnet. Die so ermittelte Konzentration an bindungsfähigen RNAP Molekülen wurde gleichgesetzt mit der Konzentration der freien, aktiven RNAP.

Im folgenden ist eine solche Konzentrationsbestimmung beschrieben. 0.12 pmol Promotorfragment (siehe Abschnitt B, 2.; spezifische Aktivität: 4 x $10^4$ cpm/pmol) wurden für 2 Minuten bei 37°C in 50 µl Bindungspuffer (BP) (20 mM Tris-HCl, pH 8,0 10 mM $MgCl_2$, 0.1 mM EDTA, 1 mM DTT, 5% Glyzerin, 120 mM KCl) inkubiert. E.coli RNAP (Pharmacia, Schweden) wurde bei 0°C in BP enthaltend 120 mM KCl verdünnt. Je 100 µl einer solchen Verdünnung wurden für 2 Minuten bei 37°C inkubiert und dann zur Lösung mit dem Promotorfragment pipettiert. Die Ansätze wurden für 5 Minuten bei 37°C gehalten, danach wurden 300 µl BP enthaltend 0.8 µg einzelsträngiger M13mp8-DNA (siehe Abschnitt B, 3.), welche bei 37°C, für 2 Minuten vorinkubiert worden war, zugegeben. Nach weiteren 5 Minuten Inkubation bei 37°C wurden die Ansätze bei 37°C über Nitrozellulose filtriert. Ein solcher Filtrationsvorgang ist nachfolgend beschrieben. Zunächst wurde ein Nitrozellulosefilter (Nitrozellulosefilter BA, 0.45 µm; Sartorius, Göttingen) in kleine quadratische Stücke (4 x 4 mm) zerschnitten und in BP enthaltend 40 mM KCl eingeweicht. Ein solches Stück wurde anschliessend auf einen ebenfalls eingeweichten Glasfaserfilter (GF/A, Whatman) transferiert, welcher sich auf einer Glasfritte befand. Diese Glasfritte stand wiederum in einem 37°C Wasserbad und war an eine Wasserstrahlpumpe angeschlossen. Die Reaktionsansätze (siehe oben) wurden mit einer Filtrationsgeschwindigkeit von 1 ml/Minute filtriert. Danach wurde der Filter mit 1 ml BP enthaltend 40 mM KCl (vorgewärmt auf 37°C) gespült. Zur Eluation der filtergebundenen DNA wurde der Nitrozellulosefilter mit einer Pipettenspitze (Eppendorf) in einem Eppendorfröhrchen zerdrückt und nach Zugabe von 20 µl Elutionspuffer (EP) (10 mM Tris/HCl, 1 mM EDTA, 0.1% SDS, pH 8) mit einer Pipettenspitze ausgequetscht. Der Elutionsansatz wurde für 30 Minuten auf Eis gestellt und anschliessend für 5 Minuten zentrifugiert (Eppendorf-Tischzentrifuge, 12000 Upm). Danach wurde die Elutionslösung abgehoben und in ein weiteres Eppendorfröhrchen überführt. Zum Filter wurden 50 µl TE-Puffer gegeben und der Ansatz für 3 Minuten geschüttelt (Eppendorf-Schüttler). Anschliessend wurde die Spüllösung abgehoben und mit der Elutionslösung vereinigt. Nach insgesamt drei Elutions- und Spülschritten waren etwa 95% der gebundenen DNA eluiert (Kontrolle durch Zählen der an den Filter gebundenen Restaktivität; Cerenkow). Nach dem Entfernen eventuell vorhandener Filterbruchstücke durch Zentrifugation (siehe oben) wurde die Radioaktivität des Eluats (210 µl) gemessen.

Die Berechnung der Konzentration an aktiver Polymerase in einer RNAP-Lösung ist nachfolgend beschrieben. Bei Ueberschuss von aktiver Polymerase gegenüber dem Promotorfragment bilden die Zählwerte ein Plateau, wobei der Plateauwert einer Menge von 0.12 pMolen Promotorfragment (gleiche Fragmentkonzentration wie in den Versuchen eingesetzt) entspricht. Aus den im Unterschuss von Polymerase gegenüber dem Promotorfragment erhaltenen Zählwerten kann nun für jede RNAP-Verdünnung die Konzentration an aktiver Polymerase unter Berücksichtigung des Verdünnungsfaktors über das Verhältnis Zählwert Unterschuss RNAP/Zählwert Ueberschuss RNAP ermittelt werden.

5. Bestimmung der Halbwertszeit von RNAP/Promotor-Komplexen

Bei der Herleitung der $K_{ass}$ wurde angenommen, dass der Zerfall von RNAP/Promotor-Komplexen während der Versuchszeit vernachlässigbar ist (siehe Abschnitt B, 1.). Diese Annahme wurde in Experimenten überprüft, mit deren Hilfe die Halbwertszeit von RNAP/Promotor-Komplexen bestimmt werden konnte. Eine solche Bestimmung ist nachfolgend beschrieben. Zunächst wurden RNAP/$P_{N25}$-Komplexe durch Inkubation von 0.06 pMolen Promotorfragment (siehe Abschnitt B, 2.; spezifische Aktivität: 2.4 x $10^6$ cpm/pMol) mit 1.2 pMolen aktiver Polymerase in BP enthaltend 120 mM KCl für 5 Minuten bei 37°C ausgebildet. Nach Zugabe von 5 µg einzelsträngiger M13mp8-DNA (siehe Abschnitt B, 3.) wurden aus dem Ansatz, der weiter bei 37°C gehalten wurde, zu verschiedenen Zeiten (0-180 Minuten) Proben entnommen und bei 37°C über Nitrozellulosefilter filtriert (siehe Abschnitt B, 4.). Die filtergebundene Radioaktivität wurde, wie in Abschnitt B, 4. gemessen, und die erhaltenen Werte gegen die Reaktionszeit aufgetragen. Die Auswertung dieses Diagramms ergab, dass unter diesen Versuchsbedingungen Komplexe aus RNAP und Promotor $P_{N25}$ eine Halbwertszeit von ungefähr 3 Stunden haben.

6. Bestimmung der $K_{ass}$ für RNAP und den Promotor $P_{N25}$

Die kinetischen Messungen zur Bestimmung von $K_{ass}$ für RNAP und dem Promotor $P_{N25}$ wurden unter "pseudo first order" Bedingungen, d.h. bei hohem RNAP-Ueberschuss, durchgeführt. Dazu werden die Versuchsbedingungen und Reaktionszeiten so gewählt, dass die Rückreaktion, d.h. der Zufall von gebildeten RNAP/Promotor-Komplexen, vernachlässigt werden konnte (Versuchsdauer maximal 7 Minuten bei einer Halbwertszeit der Komplexe von ungefähr 180 Minuten; siehe Abschnitt B, 5.). Die Bindungsreaktionen wurden alle nach einem einheitlichen Schema durchgeführt, wobei in drei unabhängigen Versuchsreihen sowohl die Reaktionsvolumina als auch die Konzentration der Reaktanten variiert wurden. Dabei wurden die $P_{N25}$ Promotorfragmente in Puffer vorinkubiert, bevor die ebenfalls in Puffer vorinkubierte RNAP-Lösung, deren Konzentration an aktiver Polymerase in parallel durchgeführten Versuchen wie in Abschnitt B, 4. beschrieben, bestimmt wurde, zugegeben und die Reaktion durch Mischen des Ansatzes gestartet wurde. Nach den gewählten Reaktionszeiten (1-120 Sekunden) wurde die Reaktion durch Zugabe von einzelsträngiger M13mp8-DNA (siehe Abschnitt B, 3.) gestoppt. Danach wurden die gebildeten RNAP/Promotor-Komplexe über Filterbindungsversuche (siehe Abschnitt B, 4.) quantitativ erfasst. Nachfolgend sind drei Versuche zur Bestimmung der Komplexbildungsraten $K_{ass}$ beschrieben.

Versuch 1

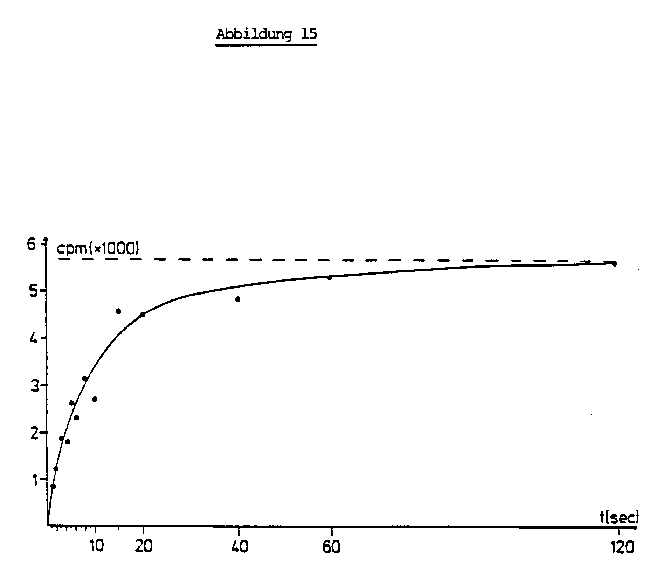

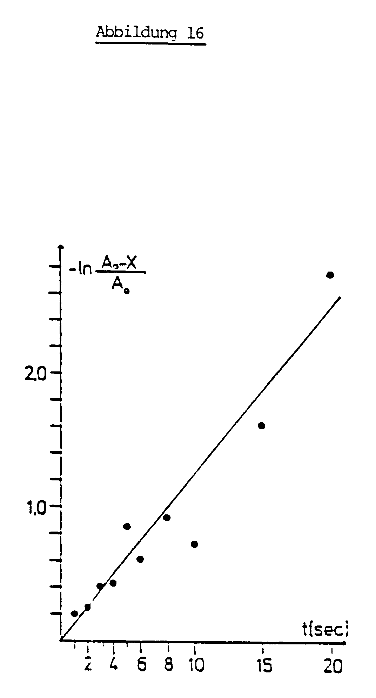

50 $\mu$l $P_{N25}$ Promotorfragment (0.045 nM, spezifische Aktivität 3 x $10^6$ cpm/pMol) in BP enthaltend 120 mM KCl wurden für 2 Minuten bei 37 °C inkubiert. RNAP wurde bei 0 °C schrittweise (jeweils 1:10 bis 1:20) in BP enthaltend 120 mM KCl verdünnt. Anschliessend wurden 100 $\mu$l einer 1:5000 Verdünnung für 2 Minuten bei 37 °C vorinkubiert (die Konzentration von aktiver Polymerase betrug 0.42 nM; siehe Abschnitt B, 4.). Die Lösungen mit dem $P_{N25}$ Promotorfragment und der RNAP wurden vereinigt und die Assoziationsreaktion durch Mischen des Ansatzes gestartet. Nach 10 Sekunden Inkubation bei 37 °C wurden 300 $\mu$l für 2 Minuten bei 37 °C Inkubierte BP-Lösung enthaltend 0.8 $\mu$g einzelsträngige M13mp8-DNA, zugegeben. Nach Inkubation für 5 Minuten bei 37 °C wurde der Ansatz, wie vorstehend beschrieben, bei 37 °C über Nitrozellulose filtriert, das Filter mit 200 $\mu$l BP enthaltend 40 mM KCl gespült und anschliessend die filtergebundene Radioaktivität bestimmt (Cerenkow). Weitere elf Versuche wurden unter gleichen Bedingungen durchgeführt, wobei die Assoziationsreaktionen nach 1, 2, 3, 4, 5, 6, 7, 8, 15, 20, 60 bzw. 120 Sekunden durch Zugabe der einzelsträngigen M13mp8-DNA gestoppt wurden. Die erhaltenen Zählwerte aller zwölf Teilversuche wurden in Abbildung 15 gegen die Reaktionszeit aufgetragen, wobei die gestrichelte Linie die maximal filterbindbare Radioaktivität darstellt. Zur Bestimmung dieses Wertes wurden 50 $\mu$l $P_{N25}$ Promotorfragment mit 100 $\mu$l BP enthaltend 120 mM KCl und 1 pMol aktiver Polymerase für 3 Minuten bei 37 °C inkubiert. Danach wurde die Reaktion gestoppt und der Ansatz wie beschrieben aufgearbeitet. Die maximal binddbare Radioaktivität ($A_0$) entspricht der Menge an eingesetzten Promotorfragmenten (bzw. 100% RNAP/$P_{N25}$-Komplexen). Die Differenz aus $A_o$ und der zum Zeitpunkt t gebundenen Radioaktivität (X) ist ein relatives Mass für das freie Promotorfragment. Der Ausdruck $(A_0\text{-}X)/A_0$ gibt dann den Anteil von freiem Promotorfragment zum Zeitpunkt t an. Für alle Teilversuche wurde der Ausdruck $-\ln[(A_0\text{-}X)/A_0]$ berechnet und gegen die Reaktionszeit t aufgetragen (siehe Abb. 16). Die Steigung m der Regressionsgeraden entspricht der Geschwindigkeitskonstanten m der Gleichungen (3) und (4) aus Abschnitt 1 und wurde als m = 0.12/Sekunde berechnet. Unter Berücksichtigung der Konzentration an aktiver Polymerase von 0.42 nM (wie in Abschnitt B, 4. bestimmt) ergab sich über die Beziehung $K_{ass}$ = m/R (Gleichung (2) in Abschnitt 1) eine $K_{ass}$ von 2.8 x $10^8$ $M^{-1}$ • $sek^{-1}$.

Versuche 2 und 3

Die Versuche 2 und 3 wurden in zum Versuch 1 analoger Weise durchgeführt, wobei die Reaktanten bezogen auf die Reaktionslösungen in folgenden Konzentrationen vorlagen:

| | | |
|---|---|---|
| Versuch 2 | Promotorfragment $P_{N25}$:<br>aktive RNAP:<br>Reaktionszeiten: | 0.02 nM<br>0.32 nM<br>2, 4, 8, 10, 15, 20 und 60 Sekunden |
| Versuch 3 | Promotorfragment $P_{N25}$:<br>aktive RNAP:<br>Reaktionszeiten: | 0.02 nM<br>0.15 nM<br>2, 4, 8, 10, 15, 20 und 60 Sekunden |

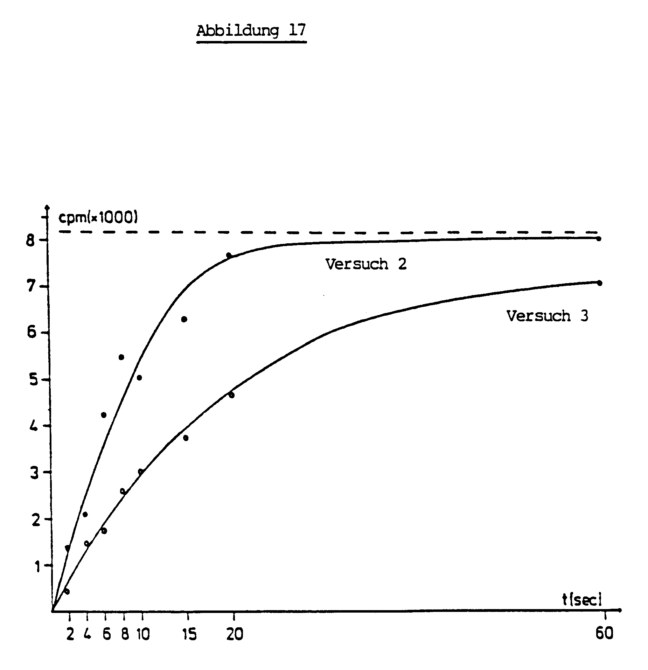

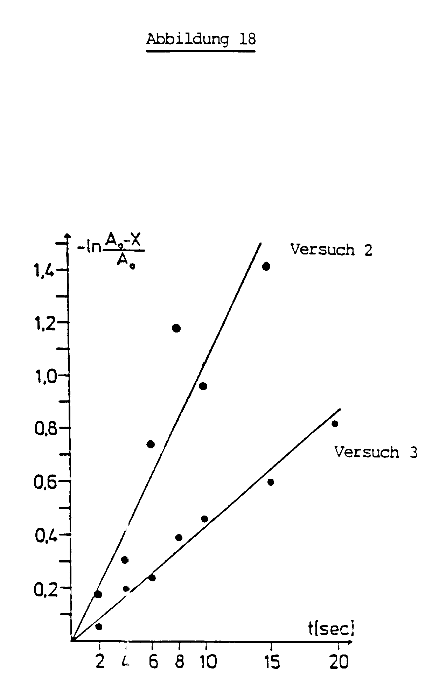

Die erhaltenen Zählwerte wurden, wie für Versuch 1 beschrieben, ausgewertet (siehe Abb. 17 und 18), wobei folgende Werte ermittelt wurden:

| | | |
|---|---|---|
| Versuch 2 | m = 0.104/Sek.; | $K_{ass}$ = 3.2 x $10^8$ $M^{-1}$ • $sek^{-1}$ |
| Versuch 3 | m = 0.04/Sek.; | $K_{ass}$ = 2.7 x $10^8$ $M^{-1}$ • $sek^{-1}$ |

Gemittelt über alle drei Versuche wurde als Komplexbildungsrate $K_{ass}$ für RNAP und den Promotor $P_{N25}$ folgender Wert erhalten:

$K_{ass}$ = 2.9 x $10^8$ $M^{-1}$ • $sek^{-1}$.

Dieser Wert kann mit einem Fehler von annähernd 15% behaftet sein, da die Messfehler sowohl bei der Konzentrationsbestimmung von Promotorfragmenten und selektiver RNAP als auch bei den Filterbindungsversuchen in der Grössenordnung von 10% leigen.

C. Bestimmung der Komplexbildungsraten für RNAP und verschiedene Expressionskontrollsequenzen über Relativmessungen mit den Promotoren $P_{N25}$ bzw. $P_{AI}$ als internem Standard

1. Theorie

Die Komplexbildungsraten für RNAP und die Expressionskontrollsequenzen tacOP29, N25*/0, N25OP29, N25OpSN25Op29, AI, A1OPSA1, A1OPSCONA1, A1pOPSA1 und OPA1OPSA1 wurden über Relativmessungen mit den Promotoren $P_{N25}$ bzw. $P_{AI}$ als internen Standard ermittelt. Dabei wurden folgende Ueberlegungen zugrunde gelegt:

In einer Mischung verschiedener Promotoren kompetieren diese entsprechend ihrer Komplexbildungsraten von RNAP, falls diese im Unterschuss vorliegt.

Für die zeitliche Aenderung der Promotorkonzentration einer bimolekularen Reaktion zweiter Ordnung zwischen RNAP und Promotor gilt bei hoher Halbwertzeit der gebildeten Komplexe Gleichung (1) (siehe Abschnitt B) $dP/dt = -K_{ass} \times R \times P$.

In einer Reaktionsmischung mit verschiedenen Promotoren ist die Konzentration an freier RNAP zu jeder Zeit für alle Promotoren gleich. Deshalb gelten für zwei Promotoren a und b mit den Komplexbildungsraten $K_{ass,a}$ und $K_{ass,b}$ folgende Beziehungen:

$$- dP_a/P_a \times 1/K_{ass,a} = R \times dt$$
$$- dP_b/P_b \times 1/K_{ass,b} = R \times dt$$

Da R x dt für beide Promotoren gleich ist, gilt:

$$- dP_a/P_a \times 1/K_{ass,a} = - dP_b/P_b \times 1/K_{ass,b}$$

oder umgeformt:

$$- dP_a/P_a = - K_{ass,a}/K_{ass,b} \times dP_b/P_b$$

Nach Integration erhält man:

$$- \ln[(A_0-x)/A_0] = - K_{ass,a}/K_{ass,b} \times \ln[(B_0-y)/B_0]$$

bzw.

$$K_{ass,b} = K_{ass,a} \times -\ln[(B_0-y)/B_0]/-\ln[(A_0-x)/A_0] \qquad (6)$$

mit $A_0$ und $B_0$ als Gesamtmenge der im RNAP Ueberschuss gebundenen Promotoren a bzw. b, sowie mit x und y als der Menge an im RNAP Unterschuss gebildeten Komplexen aus RNAP und Promotor a, bzw. aus RNAP und Promotor b.

2. Bestimmung von Komplexbildungsraten mit dem Promotor $P_{N25}$ als internem Standard

Radioaktiv-markierte DNA Fragmente (siehe Abschnitt B.2) mit den entsprechenden Expressionskontrollsequenzen (siehe Beispiel 2) wurden in parallelen Ansätzen in einem Volumen von 30µl mit unterschiedlichen Mengen an RNAP für 2 Min. bei 37°C in Bindungspuffer (siehe Abschnitt B.6) inkubiert.

Dann wurden 20µl Bindungspuffer (37°C) mit 1µg einzelsträngiger M13mp8-DNA (siehe Abschnitt B.3) zur Beendigung der Assoziationsreaktion zugegeben, bevor nach weiteren 2 Min. bei 37°C die Ansätze über Nitrozellulosefilter filtriert wurden (siehe Abschnitt B.4).

Die auf dem Filter zurückgehaltenen Komplexe wurden eluiert (siehe Abschnitt B.4) und nach einer Extraktion mit Phenol mit Aethanol präzipitiert, bevor die DNA in 30µl Probenpuffer aufgenommen wurde. Anschliessend wurde ein Drittel der vorhandenen DNA nach der Vorschrift von Maniatis (supra) in 6%-igen Polyacrylamidgelen mit 8.3 M Harnstoff elektrophoretisiert und über Autoradiographie sichtbar dargestellt.

Für jedes Verhältnis von RNAP zu Promotor wurde zunächst für jeden Promotor die Menge an gebundenem Promotor (X für $P_{N25}$, Y für den zu vermessenden Promotor) bestimmt. Dazu wurden die einzelnen Spuren in den Autoradiogrammen, die jeweils einem Versuch mit einer gewissen Menge an RNAP entsprachen, densitometrisch vermessen (Densitometer Elscript 400, Firma Hirschmann, Unterhachingen, BRD). Aus Spuren, deren Verhältnis von RNAP zu Promotor grösser als/gleich 1 war, ergeben sich die Mittelwerte für die Gesamtmengen an bindungsfähigen Promotoren ($A_0$ für $P_{N25}$, $B_0$ für den zu vermessenden Promotor).

Zur Ermittlung der Komplexbildungsraten wurden nun für jedes RNAP/Promotor-Verhältnis die Werte für $-\ln((A_0-x)/A_0)$ und $-\ln((B_0-y)/B_0)$ berechnet und graphisch gegeneinander aufgetragen. Die Steigung der erhaltenen Geraden m entspricht dem Ausdruck $-\ln[(B_0-y)/B_0]/-\ln[(A_0-x)/A_0]$ aus Gleichung (6). Mit Hilfe dieses Wertes und der Komplexbildungsrate des Promotor $P_{N25}$ ergibt sich nun mit Hilfe der Gleichung (6) die Komplexbildungsrate für den vermessenen Promotor.

Exemplarisch ist nachfolgend die Ermittlung der Komplexbildungsrate für den Promotor AI beschrieben. In sieben parallelen Ansätzen wurden jeweils ungefähr 0.02 pMol Fragment mit dem Promotor $P_{N25}$ - (~10.000cpm) und ungefähr 0.02 pMol Fragment mit dem Promotor $P_{AI}$ (~10.000 cpm) mit unterschiedlichen Mengen an RNAP (0.004-0.12 pMol) inkubiert. In Tabelle 1 sind für die einzelnen Versuche die RNAP/Promotor-Verhältnisse, die erhaltenen densitometrischen Werte sowie die daraus berechneten Werte aufgeführt.

Tabelle 1

| RNAP/Promotor | densitrometrischer Wert | | $-\ln[(A_0-x)/A_0]$ | $-\ln[(B_0-y)/B_0]$ |
|---|---|---|---|---|
| | $P_{N25}$ | $P_{AI}$ | | |
| 3 | 25338[a] | 26844[a] | | |
| 1,5 | 30331[a] | 30510[a] | | |
| 1,0 | 25361[a] | 22977[a] | | |
| 0,5 | 23085($x_1$) | 17586($y_1$) | 1.97 | 1.05 |
| 0,3 | 15729($x_2$) | 10843($y_2$) | 0.89 | 0.51 |
| 0,2 | 13226($x_3$) | 9296($y_3$) | 0.67 | 0.41 |
| 0,1 | 7203($x_4$) | 3535($y_4$) | 0.31 | 0.14 |

[a] Werte zur Bestimmung des Mittelwertes $A_0$ bzw. $B_0$ für den Promotor $P_{N25}$ bzw. $P_{AI}$.

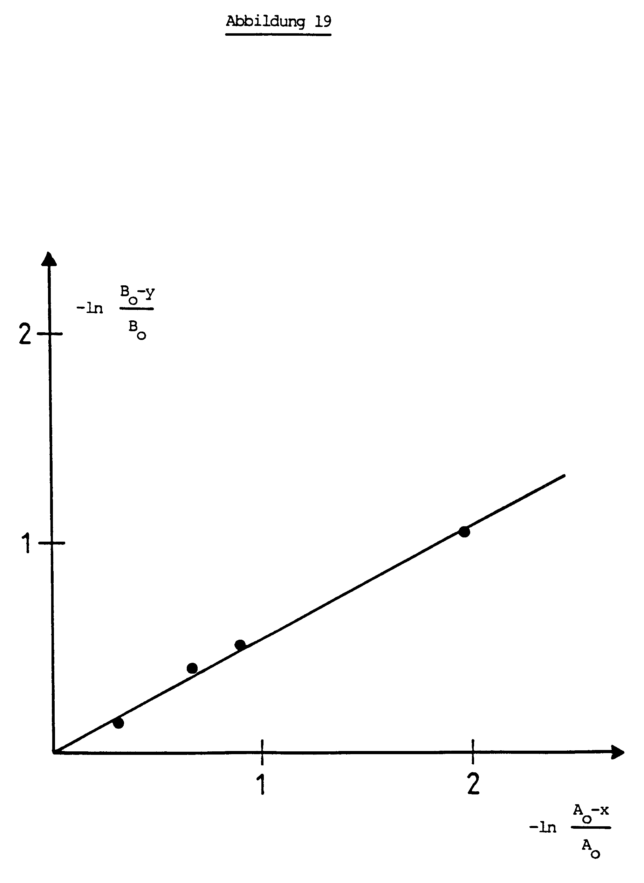

In Abbildung 19 sind die in Tabelle 1 aufgeführten Werte graphisch dargestellt. Für die Steigung der Geraden wurde der Wert m = 0.5 erhalten. Damit ergibt sich mit Hilfe von Gleichung (6) (siehe oben):

$K_{ass,\ AI} = K_{ass,\ N25} x m$ ($K_{ass,\ N25} = 2.9x10^8 M^{-1} x sec^{-1}$)
$K_{ass,\ AI} = 2.9x0.5 \ x \ 10^8 M^{-1} x sec^{-1}$
$K_{ass,\ AI} = 1.5x10^8 M^{-1} x sec^{-1}$

Für die übrigen Expressionskontrollsequenzen wurden folgende Komplexbildungsraten erhalten.

tacOP29 : $K_{ass} = 0,85x10^8 M^{-1} x sec^{-1}$
N25*/0 : $K_{ass} = 2.9x10^8 M^{-1} x sec^{-1}$
B25OP29 $K_{ass} = 2.9x10^8 M^{-1} x sec^{-1}$
N25OPSN25OP29: $K_{ass} = 2.9x10^8 M^{-1} x sec^{-1}$

Diese Werte können mit einem Fehler von annähernd 15% behaftet sein.

3. Bestimmung der Komplexbildungsraten mit dem Promotor $P_{AI}$ als internen Standard

Die Komplexbildungsraten der Expressionskontrollsequenzen A1OPSA1, A1OPSCONA1, A1pOPSA1 und OPA1OPSA1 wurden mit dem Promotor $P_{AI}$ als internem Standard wie in Abschnitt C.2 beschrieben, bestimmt. Exemplarisch ist nachfolgend die Ermittlung der Komplexbildungsrate für das Element A1OPSA1 beschrieben. In Tabelle 2 sind für die vier durchgeführten Parallelversuche mit unterschiedlicher Menge an RNAP die RNAP/Promotor-Verhältnisse, die erhaltenen densitometrischen Werte, sowie die daraus berechneten Werte aufgeführt:

Tabelle 2

| RNAP/Promotor | densitometrischer Wert | | $-\ln[(A_0-x)/A_0]$ | $-\ln[(B_0-y)/B_0]$ |
|---|---|---|---|---|
| | $P_{AI}$ | A1OPSA1 | | |
| 3 | 61856($A_0$) | 69018($B_0$) | 0.00 | 0.00 |
| 0,5 | 41477($x_1$) | 24983($y_1$) | 1.11 | 0.45 |
| 0,2 | 22484($x_2$) | 9054($y_2$) | 0.45 | 0.14 |
| 0,1 | 11303($x_3$) | 5517($y_3$) | 0.20 | 0.08 |

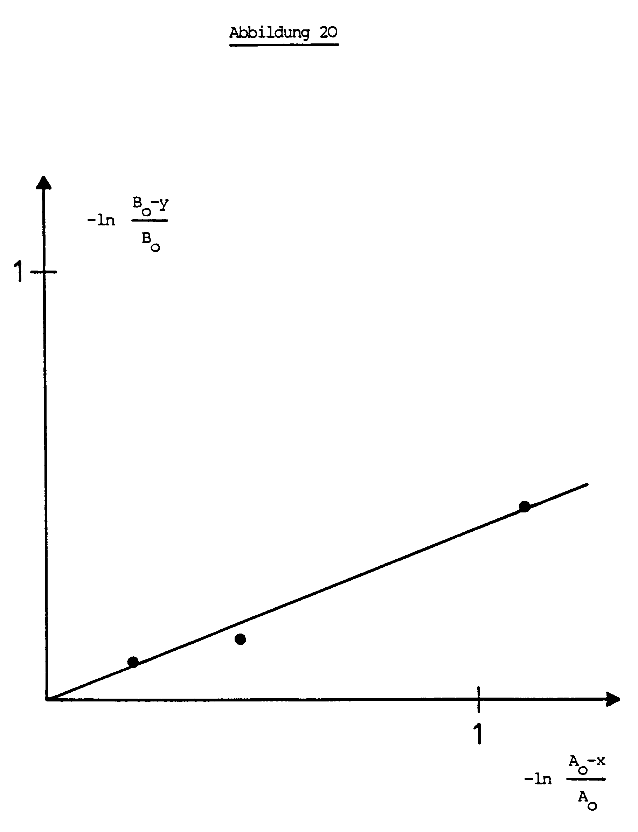

In Abbildung 20 sind die in Tabelle 2 aufgeführten Werte graphisch dargestellt. Für die Steigung der Geraden wurde der Wert m = 0,4 erhalten. Damit ergibt sich mit Hilfe von Gleichung (6):

$K_{ass,\ A1OPSA1} = K_{ass,A1} x m (K_{ass,\ A1} = 1.5x10^8\ M^{-1} xsec^{-1}$
$K_{ass,\ A1OPSA1} = 1.5x0.4x10^8\ M^{-1} xsec^{-1}$
$K_{ass,\ A1OPSA1} = 0.6x10^8\ M^{-1} xsec^{-1}$

Für die übrigen Expressionskontrollsequenzen wurden folgende Komplexbildungsraten erhalten:
A1OPSCONA1 : $K_{ass} = 1.7x10^8\ M^{-1} xsec^{-1}$
A1pOPSA1 : $K_{ass} = 0.6x10^8\ M^{-1} xsec^{-1}$
OPA1OPSA1 : $K_{ass} = 0.6x10^8\ M^{-1} xsec^{-1}$
Diese Werte können mit einem Fehler von annähernd 15% behaftet sein.

Beispiel 4

Bestimmung der in vivo Promotorstärken (induziert) der Expressionskontrollsequenzen

A. Prinzip

Die in vivo Promotorstärken der Expressionskontrollsequenzen wurden relativ zum Promotor für das β-Lactamase Gen ($P_{bla}$) als internem Standard nach dem von Deuschle et al. (supra) beschriebenen Verfahren bestimmt. Dazu wurden pDS3-Derivate mit den entsprechenden Expressionskontrollsequenzen in E. coli M15 Zellen, die das Plasmid pDMI,1 enthielten, transformiert. Aus Kulturen dieser Transformanten wurde dann die in Anwesenheit von IPTG (Induktor) während einer bestimmten Zeit synthetisierte, radioaktiv markierte RNA isoliert und anschliessend in getrennten Ansätzen gegen folgende, im Ueberschuss vorliegenden einzelsträngigen DNA-Proben hybridisiert: 1) M13mp9dhfr-DNA, 2) M13mp9bla-DNA und 3) M13mp9-DNA als Kontrolle. Nach RNase-Behandlung zum Abbau nicht hybridisierter RNA wurden die Ansätze über Nitrozellulosefilter filtriert. Da RNA/DNA-Hybride auf Nitrozellulose zurückgehalten werden, ist die filtergebundene Radioaktivität ein Mass für die in den einzelnen Ansätzen vorhandene Menge an RNA, die Komplementär zu den eingesetzten DNA-Proben ist. Nach Abzug der in der Kontrolle (einzelsträngige M13mp9-DNA) gebundenen Radioaktivität wurde das Verhältnis von durch einzelsträngige M13mp9dhfr-DNA gebundener Radioaktivität (die entsprechende RNA wurde unter Kontrolle der zu vermessenden Expressionskontrollsequenz synthetisiert) zu durch einzelsträngige M13mp9bla-DNA gebundener Radioaktivität (diese RNA wurde unter Kontrolle von $P_{bla}$ synthetisiert) bestimmt. Nach einer erforderlichen Korrektur gibt dieses Verhältnis die Promotorstärke der vermessenen Expressionskontrollsequenz in $P_{bla}$-Einheiten an.

B. Herstellung der einzelsträngigen M13mp9, M13mp9dhfr und M13mp9bla Phagen-DNAs

Zur Herstellung des Phagen M13mp9dhfr wurde das BamHI-HindIII Fragment des Plasmids pDS1,$t_o1^+$, enthaltend das dhfr-Gen in DNA des Phagen M13mp9 (Pharmacia Schweden; geschnitten mit BamHI und HindIII) nach bekannten Methoden (Maniatis et al., supra) integriert. Der Phage M13mp9bla wurde in analoger Weise hergestellt, wobei das EcoRI-PstI Fragment aus dem Plasmid pDS1,$t_o1^+$ mit Teilen des bla-Gens in DNA des Phagen M13mp9 (geschnitten mit EcoRI und PstI) integriert wurde. Danach wurde einzelsträngige M13mp9-DNA, M13mp9dhfr-DNA und M13mp9bla-DNA in analoger Weise, wie in Beispiel 3 B.3 für die Herstellung von einzelsträngiger M13mp8-DNA beschrieben, hergestellt.

C. Bestimmung der in vivo Promotorstärken (induziert)

Die Bestimmung der Promotorstärken nach dem von Deuschle et al. (supra) beschriebenen Verfahren wurde wie folgt durchgeführt:

1) Herstellung von in vivo markierter $^3$H-RNA

E. coli M15-Zellen, enthaltend das Plasmid pDMI,1, sowie ein pDS3-Derivat mit einer der verschiedenen Expressionskontrollsequenzen (siehe Beispiel 2), wurden in 20%-igem Glyzerin bei -20 °C gelagert. 10ml LB-Medium, enthaltend 100 µg/ml Ampicillin und 25 µg/ml Kanamycin wurden mit einer vorstehend genannten Stammkultur angeimpft und über Nacht bei 37 °C im Schüttelinkubator (180 Upm) wachsen gelassen. 0.1ml dieser Uebernachtkultur wurden in 25ml auf 37 °C vorgewärmtes M9-Minimalmedium (J.H. Miller, supra), enthaltend 5% Caseinhydrolysat, 0.1% Bactotrypton, 0.05% Hefeextrakt, 0.05% NaCl, 0.5% Glyzerin, 1mM IPTG, 100µg/ml Ampicillin und 25 µg/ml Kanamycin, verdünnt. Die Zellen wurden bei 37 °C im Schüttelinkubator (250 Upm) bis zu einer optischen Dichte von $OD_{600} = 0.6$ wachsen gelassen. Zu 10ml dieser Kultur wurden 0.5mCi 5,6-$^3$H-Uridin (40-60mCi/mMol, 1mCi/ml wässrige Lösung; Amersham, Braunschweig, FRG) zugegeben. Nach 45 Sekunden wurde die Kultur mit Hilfe von flüssigem Stickstoff schnell auf 0 °C abgekühlt, bevor die Zellen abzentrifugiert und anschliessend in TES-Puffer (20mM Tris-HCL, pH 8.0, 10 mM EDTA, 100 mM NaCl, 1% SDS) resuspendiert wurden. Nach Inkubation für 3 Minuten bei 95 °C wurde die resultierende Mischung lysierter Zellen nach dem von Glisin et al. (Biochemistry 13, 2633-2637 [1974]) beschriebenen Verfahren zentrifugiert (CsCl-Gradientenzentrifugation, 150.000xg, 16 Stunden, 20 °C). Nach Entfernung des Ueberstandes wurden die Zentrifugenröhrchen 0.8 cm über dem Boden mit Hilfe eines erhitzten Skalpells abgeschnitten. Die RNA im unteren Teil des Röhrchens wurde mit 2 x 80 µl TE-Puffer, enthaltend 0.2% SDS, gelöst und anschliessend mit Aethanol in Gegenwart von 3M Natriumacetat präzipitiert. Der Niederschlag wurde mit 80%igem Aethanol gewaschen, im Vakuum getrocknet und anschliessend in Hybridisierungspuffer (siehe unten) gelöst. In der Regel wurden 200-300 µg RNA mit einer spezifischen Aktivität von 1-3 x $10^5$ cpm (Cerenkow) / µg RNA aus 10ml Kultur erhalten.

2) Hybridisierung der RNA an einen Ueberschuss von einzelsträngiger DNA

Alle Hybridisierungen wurden bei 42 °C in 20µl Hybridisierungspuffer (50% Formamid, 300mM NaCl, 20mM Tris-HCL, pH 8.0, 0.5mM EDTA) während 2 Stunden durchgeführt. In einem typischen Experiment wurden 10µl der in vivo $^3$H-RNA (~5 x $10^5$ cpm) mit 10 µl einzelsträngiger M13mp9-DNA (0.2 pMole, Kontrolle), M13mp9dhfr-DNA (0.2 pMole), bzw. M13mp9bla-DNA (0.2 pMole) gemischt, für 3 Minuten bei 65 °C inkubiert und dann für 2 Stunden bei 42 °C gehalten.

3) Quantifizierung der hybridisierten RNA

Die Hybridisierungsansätze wurden mit 2 x SSC-Puffer zehnfach verdünnt und durch Nitrozellulosefilter filtriert (0.45 µm BA85 Filter, Minifold-System, Schleicher und Schüll, FRG). Die Kapazität der verwendeten Filter für einzelsträngige M13mp9-DNA betrug ungefähr 6pMole/$cm^2$. Die Filter wurden mit 2ml 2 x SSC-Puffer gewaschen, für 30 Minuten im Vakuum bei 80 °C gebacken und dann für 1 Stunde bei 42 °C in 100 ml 2 x SSC-Puffer, enthaltend 50 µg/ml RNase A, inkubiert. Anschliessend wurden die Filter dreimal mit je 100ml 2 x SSC-Puffer während 10 Minuten bei 42 °C gewaschen. Nach dem Trocknen der Filter wurde die auf ihnen zurückgehaltene Radioaktivität in Szinbillationsflüssigkeit ("universal liquid scintillator", NEN) gezählt. Das Verhältnis von dhfr- zu bla-spezifischer RNA wurde unter Berücksichtigung der Anzahl der Uridine innerhalb der verschiedenen RNAs berechnet. Da die für dhfr und bla spezifischen einzelsträngigen DNA-Inserts für 169, bzw. 148 Uridine kodieren (148/169 = 0.87), ergibt sich die in vivo Promotorstärke S einer gewünschten Expressionskontrollsequenz in $P_{bla}$-Einheiten nach der Formel:

$$S = 0.87x(cpm_{dhfr}-cpm_{Kontrolle})/(cpm_{bla}-cpm_{Kontrolle}).$$

D. In vivo Promotorstärken der Expressionskontrollsequenzen

Für jede der verschiedenen Expressionskontrollsequenzen wurde die Bestimmung der in vivo Promotorstärke, wie in Abschnitt C beschrieben, mindestens dreimal durchgeführt, wobei die synthetisierte, radioaktiv markierte RNA jeweils zweimal bestimmt wurde. Für die Expressionskontrollsequenz A1OPSA1 wurden beispielsweise folgende Werte bei der Bestimmung der synthetisierten RNA erhalten:

| Messung-Nr. | M13mp9-DNA | Einzelsträngige M13mp9dhrf-DNA | M13mp9bla-DNA |
|---|---|---|---|
| 1 | 38 | 33 581 | 839 |
| 2 | 22 | 36 099 | 831 |

Damit ergaben sich für die in vivo Promotorstärken die folgenden Werte:

$S_1$ - 0.87x(33581-38)/(839-38) = 36.4 $P_{bla}$-Einheiten
$S_2$ - 0.87x(36099-22)/(831-22) = 38.8 $P_{bla}$-Einheiten.

Als Mittelwert wurde eine in vivo Promotorstärke von 37,6 $P_{bla}$-Einheiten berechnet.

Gemittelt über insgesamt drei Bestimmungen wurde für die Expressionskontrollsequenz A1OPSA1 eine Promotorstärke von 38.1 ± 3.4 $P_{bla}$-Einheiten berechnet. In Tabelle 3 sind für alle getesteten Expressionskontrollsequenzen die, wie vorstehend beschrieben, ermittelten Promotorstärken aufgeführt.

Tabelle 3

| Expressionskontrollsequenz | in vivo Promotorstärke, induziert [$P_{bla}$-Einheiten] |
|---|---|
| lacOP29 | 5.5 ± 1.0 |
| tacOP29 | 17.6 ± 1.8 |
| N25 | 26.2 ± 2.0 |
| N25*/0 | 8.0 ± 1.5 |
| N25OP29 | 7.7 ± 1.3 |
| N25OPSN25OP29 | 9.3 ± 1.2 |
| Al | 66.1 ± 2.5 |
| A1OPSA1 | 38.1 ± 3.4 |
| A1OPSCONA1 | 16.8 ± 2.0 |
| A1pOPSA1 | 25.1 ± 1.3 |
| OPA1OPSA1 | 38.1 ± 3.4 |
| A10PSA10P29 | 31.5 ± 3.0 |
| A10PSA10P21 | 32.9 ± 5.0 |

Beispiel 5

Bestimmung des Repressionsfaktors der verschiedenen Expressionskontrollsequenzen

A. Prinzip

Die Repressionsfaktoren, d.h. das Verhältnis der in vivo Promotorstärken unter induzierten und reprimierten Bedingungen, für die einzelnen Expressionskontrollsequenzen wurden wie folgt bestimmt:

a) Expressionskontrollsequenzen mit einem Repressionsfaktor von kleiner als 100

Die Expressionskontrollsequenzen tacOP29, N25*/0, N25OP29, OPUA1 und OPUA1CON initiieren in vivo unter reprimierten Bedingungen (Ueberschuss an Repressor, kein Induktor) genügende Mengen an RNA, so dass diese direkt bestimmt werden konnten. Mittels der in Beispiel 4 bestimmten Werte für die in vivo Promotorstärke (induziert) wurden dann die Repressionsfaktoren berechnet.

b) Expressionskontrollsequenzen mit einem Repressionsfaktor von grösser als 100

Für die Expressionskontrollsequenzen lacOP29, N25OPSN25OP29, A1OPSA1, A10PSA10P21, A10PSA10P29, A1OPSCONA1, A1pOPSA1 und OPA1OPSA1 wurde die in vivo Promotorstärke unter reprimierten Bedingungen indirekt bestimmt. Dazu wurde zunächst für die einzelnen Elemente die Menge an $\beta$-Galaktosidase, die in einem geeigneten System unter reprimierten Bedingungen produziert wurde, mittels eines enzymatischen Tests bestimmt. Die so erhaltenen Werte wurden dann mittels eines Korrekturfaktors in $P_{bla}$-Einheiten umgerechnet. Mit Hilfe der in Beispiel 4 bestimmten Werte wurden dann die Repressionsfaktoren berechnet.

B. Direkte Bestimmung der in vivo Promotorstärken ($P_{bla}$-Einheiten) der Expressionskontrollsequenzen unter reprimierten Bedingungen

Die in vivo Promotorstärken unter reprimierten Bedingungen wurde, wie in Beispiel 4 beschrieben, jedoch ohne Zugabe von IPTG, bestimmt. Die erhaltenen Werte sind in Tabelle 4 zusammengefasst:

Tabelle 4

| Expressionskontrollsequenz | Promotorstärken, reprimiert [$P_{bla}$-Einheiten] |
|---|---|
| tacOP29 | 0.36 ± 0.02 |
| N25OP29 | 1.5 ± 0.1 |
| N25*/0 | 1.5 ± 0.1 |
| OPUA1 | 2.7 ± 0.5 |
| OPUA1CON | 1.7 ± 0.6 |

C. Indirekte Bestimmung der in vivo Promotorstärken der Expressionskontrollsequenzen unter reprimierten Bedingungen

1. Bestimmung der β-Galaktosidase-Einheiten

Die pML3-Derivate mit den Expressionskontrollsequenzen lacOP29, tacOP29, N25OP29, N25OPSN25OP29, A1OPSA1, A10PSA10P21, A10PSA10P29, A1OPSCONA1, A1pOPSA1, OPA1OPSA1, OPUA1 und OPUA1CON wurden zunächst nach bekannten, in der Literatur beschriebenen Methoden (Maniatis et al., supra) in E. coli M15 Zellen transformiert. Die erhaltenen transformierten E. coli M15 Zellen mit den entsprechenden Plasmiden wurden dann bis zur logarithmsichen Phase wachsen gelassen und die Menge an β-Galaktosidase wurde nach der Vorschrift von J.H. Miller ("Experiments in Molecular Genetics", Cold Spring Harbor, New York, 1972) bestimmt. Eine solche Bestimmung ist nachstehend am Beispiel der Expressionskontrollsequenz N25OP29 beschrieben.

10 ml supplementiertes Minimalmedium (J.H. Miller, supra) wurden mit 0.05 ml einer Uebernachtkultur von transformierten E. coli M15 Zellen, enthaltend Plasmid pML3/N25OP29 angeimpft, und bei 37°C im Schüttelinkubator (200 Upm) inkubiert. Nach Erreichen einer $OD_{600}$ (optische Dichte bei einer Wellenlänge von 600 nm) von 0.55 wurde die Kultur für 20 Minuten auf Eis gestellt. Danach wurde die optische Dichte erneut bestimmt. Es wurde ein Wert von $OD_{600}$ = 0.607 erhalten. 0.1 ml der Kultur wurden dann mit Z-Puffer (J.H. Miller, supra) auf ein Endvolumen von 1 ml verdünnt. Diese Probe wurde zusammen mit einer Kontrolle (1ml Z-Puffer) wie folgt behandelt:

Zugabe von 60 µl Chloroform und 30 µl 0.1% SDS (Zellaufschluss);

Mischen der Probe für 10 Sekunden mittels Vortex;

Inkubation der Probe für 5 Minuten bei 28°C;

Zugabe von 200 µl ONPG (J.H. Miller, supra);

Mischen der Probe mittels Vortex;

Inkubation der Probe bei 28°C bis Gelbfärbung sichtbar wurde;

Zugabe von 250µl 2M $Na_2CO_3$ (die Zeit zwischen der Zugabe von ONPG und $Na_2CO_3$ betrug 1.5 Minuten);

Mischen der Probe mittels Vortex;

Abzentrifugation der Zelltrümmer (Eppendorf-Tischzentrifuge, 13000 Upm, 5 Minuten); und

Bestimmung der Extinktion bei 420 nm gegen die Kontrolle.

Es wurden ein Wert von $\Delta E_{420}$ = 0.737 erhalten. Nach der im vorstehend genannten Lehrbuch von J.H. Miller beschriebenen Formel:

$$\beta\text{-Galaktosidase-Einheiten} = 1000 \times \Delta E_{420}/OD_{600} x V x t$$

worin $\Delta E_{420}$ der $E_{420}$ Messwert des Reaktionsansatzes gegen die Kontrolle (0.737), $OD_{600}$ die Zelldichte der eingesetzten Kulturprobe (0.607), t die Reaktionszeit (1.5 Minuten) und V das eingesetzte Volumen der Kultur (0.1 ml) ist,
wurden 8094 β-Galaktosidase-Einheiten berechnet. Gemittelt über 8 Versuche wurden 8160 ± 450 β-Galaktosidase-Einheiten erhalten.

In Tabelle 5 sind für alle vermessenen Expressionskontrollsequenzen die erhaltenen Werte zusammengefasst, wobei für jede Expressionskontrollsequenz mindestens 4 Messungen durchgeführt wurden.

Tabelle 5

| Expressionskontrollsequenz | β-Galaktosidase-Einheiten |
|---|---|
| lacOP29 | 30 ± 5 |
| tacOP29 | 1510 ± 170 |
| N25OP29 | 8160 ± 450 |
| N25OPSN25OP29 | 99 ± 6 |
| A1OPSA1 | 110 ± 2 |
| A1OPSCONA1 | 220 ± 12 |
| A1pOPSA1 | 58 ± 3 |
| OPA1OPSA1 | 56 ± 1 |
| OPUA1 | 13700 ± 3000 |
| OPUA1CON | 8500 ± 2000 |
| A10PSA10P29 | 23 ± 3 |
| A10PSA10P21 | 15 ± 3 |

2. Bestimmung des Faktors zur Umrechnung von β-Galaktosidase-Einheiten in $P_{bla}$-Einheiten

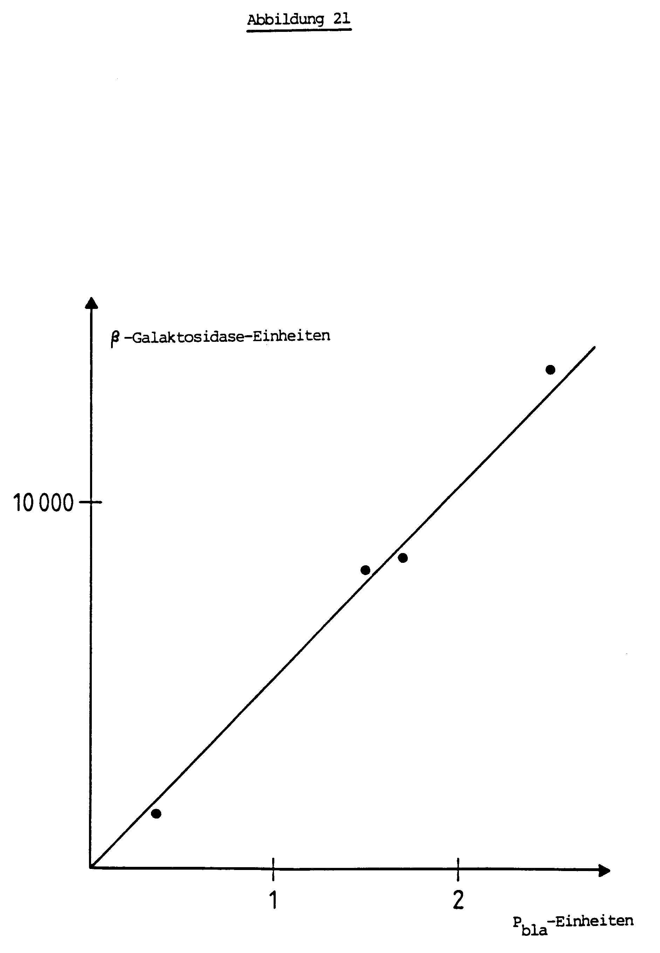

Für die Expressionskontrollsequenzen tacOP29, N25OP29, OPUA1 und OPUA1CON konnten die in vitro Promotorstärken unter reprimierten Bedingungen sowohl direkt ($P_{bla}$-Einheiten, Tabelle 4) als auch indirekt (β-Galaktosidase-Einheiten, Tabelle 5) bestimmt werden. In Abb. 21 sind die erhaltenen Werte gegeneinander aufgetragen. Die Steigung der Regressionsgeraden ist ein Mass für die Umrechnung der β-Galaktosidase-Einheiten in $P_{bla}$-Einheiten. Es wurde ein Wert von 5000 β-Galaktosidase-Einheiten / $P_{bla}$-Einheit ermittelt.

3. Umrechnung von β-Galaktosidase-Einheiten in $P_{bla}$-Einheiten

Mit Hilfe des Umrechnungsfaktors 5000 β-Galaktosidase-Einheiten / $P_{bla}$-Einheit wurden für die entsprechenden Expressionskontrollsequenzen aus den gemessenen β-Galaktosidase-Einheiten (Tabelle 5) die $P_{bla}$-Einheiten berechnet (Tabelle 6).

Tabelle 6

| Expressionskontrollsequenz | β-Galaktosidase-Einheiten | $P_{bla}$Einheiten |
|---|---|---|
| lacOP29 | 30 ± 5 | 0.006 ± 0.001 |
| N25OPSN25OP29 | 99 ± 6 | 0.02 ± 0.003 |
| A1OPSA1 | 110 ± 2 | 0.022 ± 0.001 |
| A1OPSCONA1 | 220 ± 12 | 0.044 ± 0.003 |
| A1pOPSA1 | 58 ± 3 | 0.012 ± 0.001 |
| OPA1OPSA1 | 56 ± 1 | 0.011 ± 0.001 |
| A10PSA10P29 | 23 ± 3 | 0.0046± 0.0006 |
| A10PSA10P21 | 15 ± 3 | 0.003 ± 0.0006 |

D. Berechnung der Repressionsfaktoren

Zur Berechnung der Repressionsfaktoren ($P_{bla}$-Einheiteninduziert/$P_{bla}$-Einheiten, reprimiert) für die einzelnen Expressionskontrollsequenzen wurden die in den Tabellen 3, 4 und 6 aufgeführten Werte herangezogen (Tabelle 7).

Tabelle 7

| in vivo Promotorstärken | | | |
|---|---|---|---|
| Expressionkontrollsequenz | $P_{bla}$-Einheiten, induziert | $P_{bla}$-Einheiten, reprimiert | Repressionsfaktor |
| lacOP29 | 5.5±1.0 | 0.006±0.001 | 920±230 |
| tacOP29 | 17.6±1.8 | 0.36 ±0.02[a] | 49± 6 |
| N25*/0 | 8.0±1.5 | 1.5 ±0.1[a] | 5.3±2 |
| N25OP29 | 7.7±1.3 | 1.5 ±0.1[a] | 5.1±2 |
| N25OPSN25OP29 | 9.3±1.2 | 0.02 ±0.003 | 465± 92 |
| A1OPSA1 | 38.1±3.4 | 0.022±0.001 | 1730±170 |
| A1OPSCONA1 | 16.8±2.0 | 0.044±0.003 | 380±50 |
| A1pOPSA1 | 25.1±1.3 | 0.012±0.001 | 2090±240 |
| OPA1OPSA1 | 38.1±3.4 | 0.011±0.001 | 3460±460 |
| A10PSA10P29 | 31.5±3.0 | 0.0046±0.0006 | 6850±610 |
| A10PSA10P21 | 32.9±5.0 | 0.003±0.0006 | 10970±2000 |

[a] direkte Bestimmung der in vivo Promotorstärke

E. Zusammenfassung der die einzelnen Expressionskontrolsequenzen charakterisierenden Eigenschaften

In der folgenden Tabelle 8 sind die für die einzelnen Expressionskontrollsequenzen erhaltenen Werte für die in vitro Komplexbildungsrate, die in vivo Promotorstärke und den Repressionsfaktor zusammengefasst;

Tabelle 8

| Expressions-kontrollsequenz | in vitro Komplexbildungs-rate ($K_{ass}$) [$10^8 M^{-1}$ x $sec^{-1}$] | in vivo Promotorstärken reprimiert [$P_{bla}$-Einheiten] | in vivo Promotorstärken induziert [$P_{bla}$-Einheiten] | Repressionsfaktor |
|---|---|---|---|---|
| lacOP29 | 0.02[a] | 0.006 ± 0.001 | 5.5 ± 1.0 | 920 ± 230 |
| tacOP29 | 0.85 | 0.36 ± 0.02 | 17.6 ± 1.8 | 49 ± 6 |
| N25 | 2.9 | - | 26.2 ± 2.0 | - |
| N25*/0 | 2.9 | 1.5 ± 0.1 | 8.0 ± 1.5 | 5.3 ± 2 |
| N25OP29 | 2.9 | 1.5 ± 0.1 | 7.7 ± 1.3 | 5.1 ± 2 |
| N25OPSN25OP29 | 2.9 | 0.02 ± 0.003 | 9.3 ± 1.2 | 465 ± 92 |
| A1 | 1.5 | - | 66.1 ± 2.5 | - |
| * A1OPSA1 | 0.6 | 0.022 ± 0.001 | 38.1 ± 3.4 | 1730 ± 170 |
| A1OPSCONA1 | 1.7 | 0.044 ± 0.003 | 16.8 ± 2.0 | 380 ± 50 |
| * A1pOPSA1 | 0.6 | 0.012 ± 0.001 | 25.1 ± 1.3 | 2090 ± 240 |
| * OPA1OPSA1 | 0.6 | 0.011 ± 0.001 | 38.1 ± 3.4 | 3460 ± 460 |
| * A1OPSA1OP29 | n.b. | 0.0046 ± 0.0006 | 31.5 ± 3.0 | 6850 ± 610 |
| * A1OPSA1OP21 | n.b. | 0.003 ± 0.0006 | 32.9 ± 5.0 | 10970 ± 2000 |

[a] Literaturwert aus McClure et al. ("Promoters, Structure and Function", Hrsg. Rodriguez und Chamberlin, Praeger, Seiten 111-120 [1982])
* erfindungsgemässe Expressionskontrollsequenzen , n.b. = nicht bestimmt.

## Patentansprüche

1. Eine Expressionskontrollsequenz, gekennzeichnet durch die Kombination von Promotersequenzen mit niedriger Signalstärke von etwa 1 • $10^6$ - 1.5 • $10^8$ $M^{-1}$ • $sec^{-1}$ und hoher in vivo Promoterstärke von 10-100 $P_{bla}$-Einheiten mit Operator/Repressorsystemen mit hoher Komplexbildungsrate von etwa 1 •

$10^8$ - $10^{11} M^{-1} \cdot sec^{-1}$ und die einen Repressionsfaktor von >1000 bewirkt.

2. Eine Expressionskontrollsequenz gemäss Anspruch 1, dadurch gekennzeichnet, dass die Promotersequenz mit niedriger Signalstärke und hoher in vivo Promoterstärke eine Signalstärke von etwa 6 • $10^7 M^{-1} \cdot sec^{-1}$ und eine in vivo Promoterstärke von mehr als 20 $P_{bla}$-Einheiten aufweist.

3. Eine Expressionskontrollsequenz gemäss Anspruch 1 oder 2, dadurch gekennzeichnet, dass das Operator/Repressorsystem mit hoher Komplexbildungsrate eine Komplexbildungsrate von 2 • $10^9 M^{-1} \cdot sec^{-1}$ aufweist.

4. Eine Expressionskontrollsequenz gemäss einem der Ansprüche 1 - 3, die durch die Kombination von Promotersequenzen mit Operator/Repressorsystemen aus gram-negativen Organismen erhalten wird.

5. Eine Expressionskontrollsequenz gemäss Anspruch 4, die durch Kombination von Promotersequenzen aus T-Coliphagen mit dem lac-Operator/Repressorsystem erhalten wird.

6. Eine Expressionskontrollsequenz gemäss Anspruch 5, die durch Kombination des T7AI Promoters mit dem lac-Operator/Repressorsystem erhalten wird.

7. Eine Expressionskontrollsequenz gemäss Anspruch 6 mit den funktionellen Teilen der Nukleotidsequenz

CTCGAGAAAA TTTATCAAAA AGAGTGTTGA CTTGTGAGCG GATAACAATG
ATACTTAGAT TCATCGAGAG GGACACGGCG AATTC (AIOPSA1).

8. Eine Expressionskontrollsequenz gemäss Anspruch 6 mit den funktionellen Teilen der Nukleotidsequenz

CTCGAGAAAA TTTATCAAAA AGAGTGTTGA CTTGTGAGCG CTCACAATTG
ATACTTAGAT TCATCGAGAG GGACACGGCG AATTC (A1POPSA1).

9. Eine Expressionskontrollsequenz gemäss Anspruch 6 mit den funktionellen Teilen der Nukleotidsequenz

GTCGACGTTG ATCCCCTAGA AATTGTGAGC GCTCACAATT TCTAGGGAAT
TAACGGTACC GAGCTTGTGG CAGTTTAAGG CGGGCGTCCT GCCCGCCACC
CTCCGGGCCG TTGCTTCGCA ACGTTCAAAT CCGCTCCCGG CGGATTTGTC
CTACTCAGGA GAGCGTTCAC CGACAAACAA CAGATAAAAC GAAAGGCCCA
GTCTTTCGAC TGAGCCTTTC GTTTTATTTG ATGCCTCAAG CTCGGTACCT
CGAGAAAATT TATCAAAAAG AGTGTTGACT TGTGAGCGGA TAACAATGAT
ACTTAGATTC ATCGAGAGGG ACACGGCGAA TTC (OPA1OPSA1).

10. Eine Expressionskontrollsequenz gemäss Anspruch 6 mit den funktionellen Teilen der Nukleotidsequenz

CTCGAGAAAA TTTATCAAAA AGAGTGTTGA CTTGTGAGCG GATAACAATG
ATACTTAGAT TCAATTGTGA GCGGATAACA ATTTCACACA GAATTC
(A1OPSA1OP21).

11. Eine Expressionskontrollsequenz gemäss Anspruch 6 mit den funktionellen Teilen der Nukleotidsequenz

CTCGAGAAAA TTTATCAAAA AGAGTGTTGA CTTGTGAGCG GATAACAATG
ATACTTAGAT TCAAATTGTG AGCGGATAAC AATTTGAATT C
(A1OPSA1OP29).

12. Ein Expressionsvektor, der eine Expressionskontrollsequenz gemäss einem der Ansprüche 1 - 11 enthält.

13. Ein Expressionsvektor gemäss Anspruch 12, der in gram-negativen und/oder gram-positiven Bakterien replizieren kann.

14. Ein Expressionsvektor gemäss Anspruch 13, der in E.coli replizieren kann.

15. Ein Expressionsvektor gemäss Anspruch 13, der in B.subtilis replizieren kann.

16. Ein Expressionsvektor gemäss Anspruch 13, der in Salmonella typhimurium replizieren kann.

17. Ein Bakterium, das einen Expressionsvektor gemäss den Ansprüchen 12-16 enthält.

18. Ein Bakterium gemäss Anspruch 17, das eine E.coli Zelle ist.

19. Ein Bakterium gemäss Anspruch 18, das eine E.coli M15 Zelle ist.

20. Ein Bakterium gemäss Anspruch 17, das eine B.subtilis Zelle ist.

21. Ein Bakterium gemäss Anspruch 17, das eine Salmonella typhimurium Zelle ist.

22. Ein Bakterium, das eine Expressionskontrollsequenz gemäss einem der Ansprüche 1 - 11 im Chromosom enthält.

23. Ein Bakterium gemäss Anspruch 22, das eine E.coli Zelle ist.

24. Ein Bakterium gemäss Anspruch 22, das eine B.subtilis Zelle ist.

25. Ein Bakterium gemäss Anspruch 22, das eine Salmonella typhimurium Zelle ist.

26. Verfahren zur Herstellung eines pro- oder eukaryoten Proteins, dadurch gekennzeichnet, dass man ein Bakterium mit einem Expressionsvektor, in welchem die für genanntes Protein kodierende DNA-Sequenz mit einer Expressionskontrollsequenz gemäss einem der Ansprüche 1-11 operabel verknüpft ist, transformiert, dann unter geeigneten Bedingungen kultiviert und das genannte Protein isoliert und, falls gewünscht, reinigt.

**27.** Verfahren gemäss Anspruch 26, worin das Bakterium eine E.coli, eine Salmonella typhimurium oder B. subtilis Zelle ist.

**28.** Verfahren zur Herstellung eines pro- oder eukaryoten Proteins, dadurch gekennzeichnet, dass man die mit einer Expressionskontrollsequenz gemäss einem der Ansprüche 1-11 operabel verknüpfte kodierende DNA-Sequenz für genanntes Protein in das Chromosom eines Bakteriums einbaut, dieses Bakterium dann unter geeigneten Bedingungen kultiviert und das genannte Protein isoliert und, falls gewünscht, reinigt.

**29.** Verfahren gemäss Anspruch 28, worin das Bakterium eine E.coli, eine Salmonella typhimurium oder B.subtilis Zelle ist.

**30.** Verwendung einer Expressionskontrollserquenz gemäss einem der Ansprüche 1-11 zur Expressions von pro- und eukaryoten Proteinen.

**Claims**

**1.** An expression control sequence, characterized by the combination of promoter sequences having a low signal strength of about $1 \bullet 10^6$-$1.5 \bullet 10^8 M^{-1} \bullet sec^{-1}$ and a high in vivo promoter stength of 10-100 $P_{bla}$ units with operator/repressor systems having a high complex formation rate of about $1 \bullet 10^8$-$10^{11} M^{-1} \bullet sec^{-1}$ and which produce a repression factor of >1000.

**2.** An expression control sequence in accordance with claim 1, characterized in that the promoter sequence having a low signal strength and a high in vivo promoter strength has a signal strength of about $6 \bullet 10^7$ $M^{-1} \bullet sec^{-1}$ and an in vivo promoter strength of more than 20 $P_{bla}$ units.

**3.** An expression control sequence in accordance with claim 1 or 2, characterized in that the operator/repressor system having a high complex formation rate has a complex formation rate of $2 \bullet 10^9 M^{-1} \bullet sec^{-1}$.

**4.** An expression control sequence in accordance with any one of claims 1-3, which is obtained by the combination of promoter sequences with operator/repressor systems from gram-negative organisms.

**5.** An expression control sequence in accordance with claim 4, which is obtained by the combination of promoter sequences from T-coliphages with the lac-operator/repressor system.

**6.** An expression control sequence in accordance with claim 5, which is obtained by the combination of the T7A1 promoter with the lac-operator/repressor system.

**7.** An expression control sequence in accordance with claim 6 having the functional parts of the nucleotide sequence

CTCGAGAAAA TTTATCAAAA AGAGTGTTGA CTTGTGAGCG
GATAACAATG ATACTTAGAT TCATCGAGAG GGACACGGCG AATTC
(A1OPSA1).

**8.** An expression control sequence in accordance with claim 6, having the functional parts of the nucleotide sequence

CTCGAGAAAA TTTATCAAAA AGAGTGTTGA CTTGTGAGCG
CTCACAATTG ATACTTAGAT TCATCGAGAG GGACACGGCG AATTC
(A1POPSA1).

9. An expression control sequence in accordance with claim 6 having the functional parts of the nucleotide sequence

GTCGACGTTG ATCCCCTAGA AATTGTGAGC GCTCACAATT
TCTAGGGAAT TAACGGTACC GAGCTTGTGG CAGTTTAAGG
CGGGCGTCCT GCCCGCCACC CTCCGGGCCG TTGCTTCGCA
ACGTTCAAAT CCGCTCCCGG CGGATTTGTC CTACTCAGGA
GAGCGTTCAC CGACAAACAA CAGATAAAAC GAAAGGCCCA
GTCTTTCGAC TGAGCCTTTC GTTTTATTTG ATGCCTCAAG
CTCGGTACCT CGAGAAAATT TATCAAAAAG AGTGTTGACT
TGTGAGCGGA TAACAATGAT ACTTAGATTC ATCGAGAGGG
ACACGGCGAA TTC (OPA1OPSA1).

10. An expression control sequence in accordance with claim 6, having the functional parts of the nucleotide sequence

CTCGAGAAAA TTTATCAAAA AGAGTGTTGA CTTGTGAGCG
GATAACAATG ATACTTAGAT TCAATTGTGA GCGGATAACA
ATTTCACACA GAATTC (A1OPSA1OP21).

11. An expression control sequence in accordance with claim 6 having the functional parts of the nucleotide sequence

CTCGAGAAAA TTTATCAAAA AGAGTGTTGA CTTGTGAGCG
GATAACAATG ATACTTAGAT TCAAATTGTG AGCGGATAAC
AATTTGAATT C (A1OPSA1OP29).

12. An expression vector, which contains an expression control sequence in accordance with any one of claims 1-11.

13. An expression vector in accordance with claim 12, which can replicate in gram-negative and/or gram-positive bacteria.

14. An expression vector in accordance with claim 13, which can replicate in E. coli.

15. An expression vector in accordance with claim 13, which can replicate in B. subtilis.

**16.** An expression vector in accordance with claim 13, which can replicate in Salmonella typhimurium.

**17.** A bacterium, which contains an expression vector in accordance with claims 12-16.

**18.** A bacterium in accordance with claim 17, which is an E. coli cell.

**19.** A bacterium in accordance with claim 18, which is an E. coli M15 cell.

**20.** A bacterium in accordance with claim 17, which is a B. subtilis cell.

**21.** A bacterium in accordance with claim 17, which is a Salmonella typhimurium cell.

**22.** A bacterium, which contains an expression control sequence in accordance with any one of claims 1-11 in the chromosome.

**23.** A bacterium in accordance with claim 22, which is an E. coli cell.

**24.** A bacterium in accordance with claim 22, which is a B. subtilis cell.

**25.** A bacterium in accordance with claim 22, which is a Salmonella typhimurium cell.

**26.** A method for the production of a pro- or eukaryotic protein, characterized in that a bacterium is transformed with an expression vector in which the DNA sequence coding for said protein is operably linked with an expression control sequence in accordance with any one of claims 1-11, then cultivated under suitable conditions and the said protein is isolated and, where desired, purified.

**27.** A method in accordance with claim 26, wherein the bacterium is an E. coli, a Salmonella typhimurium or B. subtilis cell.

**28.** A method for the production of a pro- or eukaryotic protein, characterized in that the DNA sequence coding for said protein and operably linked with an expression control sequence in accordance with any one of claims 1-11 is introduced into the chromosome of a bacterium, this bacterium is then cultivated under suitable conditions and the said protein is isolated and, where desired, purified.

**29.** A method in accordance with claim 28, wherein the bacterium is an E. coli, a Salmonella typhimurium or B. subtilis cell.

**30.** The use of an expression control sequence in accordance with any one of claims 1-11 for the expression of pro- and eukaryotic proteins.

**Revendications**

**1.** Séquence régulatrice d'expression, caractérisée par l'association de séquences promotrices ayant une faible intensité de signal d'environ $1.10^6$ à $1{,}5.10^8$ $M^{-1}.s^{-1}$ et une force élevée de promoteur in vivo de 10-100 unités $P_{bla}$ avec des systèmes opérateur/répresseur à grande vitesse de formation de complexes d'environ $1.10^8$ à $10^{11}$ $M^{-1}.s^{-1}$, et qui provoque un facteur de répression >1000.

**2.** Séquence régulatrice d'expression selon la revendication 1, caractérisée en ce que la séquence promotrice à faible intensité de signal et force élevée de promoteur in vivo présente une intensité de signal d'environ $6.10^7$ $M^{-1}.s^{-1}$ et une force de promoteur in vivo de plus de 20 unités $P_{bla}$.

**3.** Séquence régulatrice d'expression selon la revendication 1 ou 2, caractérisée en ce que le système opérateur/répresseur à grande vitesse de formation de complexes présente une vitesse de formation de complexes de $2.10^9$ $M^{-1}.s^{-1}$.

**4.** Séquence régulatrice d'expression selon l'une des revendications 1 à 3, qui est obtenue par l'association de séquences promotrices avec des systèmes opérateur/répresseur provenant d'organismes Gram négatifs.

**5.** Séquence régulatrice d'expression selon la revendication 4, qui est obtenue par association de séquences promotrices provenant de coliphages T avec le système opérateur/répresseur lac.

**6.** Séquence régulatrice d'expression selon la revendication 5, qui est obtenue par association du promoteur AI de T7 avec le système opérateur/répresseur lac.

**7.** Séquence régulatrice d'expression selon la revendication 6, comportant les parties fonctionnelles de la séquences nucléotidique

CTCGAGAAAA TTTATCAAAA AGAGTGTTGA CTTGTGAGCG GATAACAATG

ATACTTAGAT TCATCGAGAG GGACACGGCG AATTC (AIOPSA1).

**8.** Séquence régulatrice d'expression selon la revendication 6, comportant les parties fonctionnelles de la séquence nucléotidique

CTCGAGAAAA TTTATCAAAA AGAGTGTTGA CTTGTGAGCG CTCACAATTG

ATACTTAGAT TCATCGAGAG GGACACGGCG AATTC (A1POPSA1).

**9.** Séquence régulatrice d'expression selon la revendication 6, comportant les parties fonctionnelles de la séquence nucléotidique

GTCGACGTTG ATCCCCTAGA AATTGTGAGC GCTCACAATT TCTAGGGAAT

TAACGGTACC GAGCTTGTGG CAGTTTAAGG CGGGCGTCCT GCCCGCCACC

CTCCGGGCCG TTGCTTCGCA ACGTTCAAAT CCGCTCCCGG CGGATTTGTC

CTACTCAGGA GAGCGTTCAC CGACAAACAA CAGATAAAAC GAAAGGCCCA

GTCTTTCGAC TGAGCCTTTC GTTTTATTTG ATGCCTCAAG CTCGGTACCT

CGAGAAAATT TATCAAAAAG AGTGTTGACT TGTGAGCGGA TAACAATGAT

ACTTAGATTC ATCGAGAGGG ACACGGCGAA TTC (OPA1OPSA1).

**10.** Séquence régulatrice d'expression selon la revendication 6, comportant les parties fonctionnelles de la séquence nucléotidique

CTCGAGAAAA TTTATCAAAA AGAGTGTTGA CTTGTGAGCG GATAACAATG

ATACTTAGAT TCAATTGTGA GCGGATAACA ATTTCACACA GAATTC

(A1OPSA1OP21).

**11.** Séquence régulatrice d'expression selon la revendication 6, comportant les parties fonctionnelles de la séquence nucléotidique

CTCGAGAAAA TTTATCAAAA AGAGTGTTGA CTTGTGAGCG GATAACAATG

ATACTTAGAT TCAAATTGTG AGCGGATAAC AATTTGAATT C

(A1OPSA1OP29).

**12.** Vecteur d'expression, qui contient une séquence régulatrice d'expression selon l'une des revendications 1 à 11.

**13.** Vecteur d'expression selon la revendication 12, capable de se répliquer dans des bactéries Gram négatives et/ou des bactéries Gram positives.

**14.** Vecteur d'expression selon la revendication 13, capable de se répliquer dans E. coli.

**15.** Vecteur d'expression selon la revendication 13, capable de se répliquer dans B. subtilis.

**16.** Vecteur d'expression selon la revendication 13, capable de se répliquer dans Salmonella typhimurium.

**17.** Bactérie contenant un vecteur d'expression selon les revendications 12 à 16.

**18.** Bactérie selon la revendication 17, qui est une cellule de E. coli.

**19.** Bactérie selon la revendication 18, qui est une cellule de E. coli M15.

**20.** Bactérie selon la revendication 17, qui est une cellule de B. subtilis.

**21.** Bactérie selon la revendication 17, qui est une cellule de Salmonella typhimurium.

**22.** Bactérie contenant dans le chromosome une séquence régulatrice d'expression selon l'une des revendications 1 à 11.

**23.** Bactérie selon la revendication 22, qui est une cellule de E. coli.

**24.** Bactérie selon la revendication 22, qui est une cellule de B. subtilis.

**25.** Bactérie selon la revendication 22, qui est une cellule de Salmonella typhimurium.

**26.** Procédé pour la production d'une protéine de procaryote ou d'eucaryote, caractérisé en ce que l'on transforme une bactérie par un vecteur d'expression dans lequel la séquence d'ADN codant pour ladite protéine est fonctionnellement liée à une séquence régulatrice d'expression selon l'une des revendications 1 à 11, puis on la cultive dans des conditions appropriées et on isole ladite protéine et, si on le désire, on la purifie.

**27.** Procédé selon la revendication 26, dans lequel la bactérie est une cellule de E. coli, une cellule de Salmonella typhimurium ou une cellule de B. subtilis.

**28.** Procédé pour la production d'une protéine de procaryote ou d'eucaryote, caractérisé en ce que l'on introduit dans le chromosome d'une bactérie la séquence d'ADN codant pour ladite protéine, fonctionnellement liée à une séquence régulatrice d'expression selon l'une des revendications 1 à 11, on cultive cette bactérie dans des conditions appropriées et on isole ladite protéine, et, si on le désire, on la purifie.

**29.** Procédé selon la revendication 28, dans lequel la bactérie est une cellule de E. coli, une cellule de Salmonella typhimurium ou une cellule de B. subtilis.

**30.** Utilisation d'une séquence régulatrice d'expression selon l'une des revendications 1 à 11, pour l'expression de protéines de procaryotes et de protéines d'eucaryotes.

Abbildung la

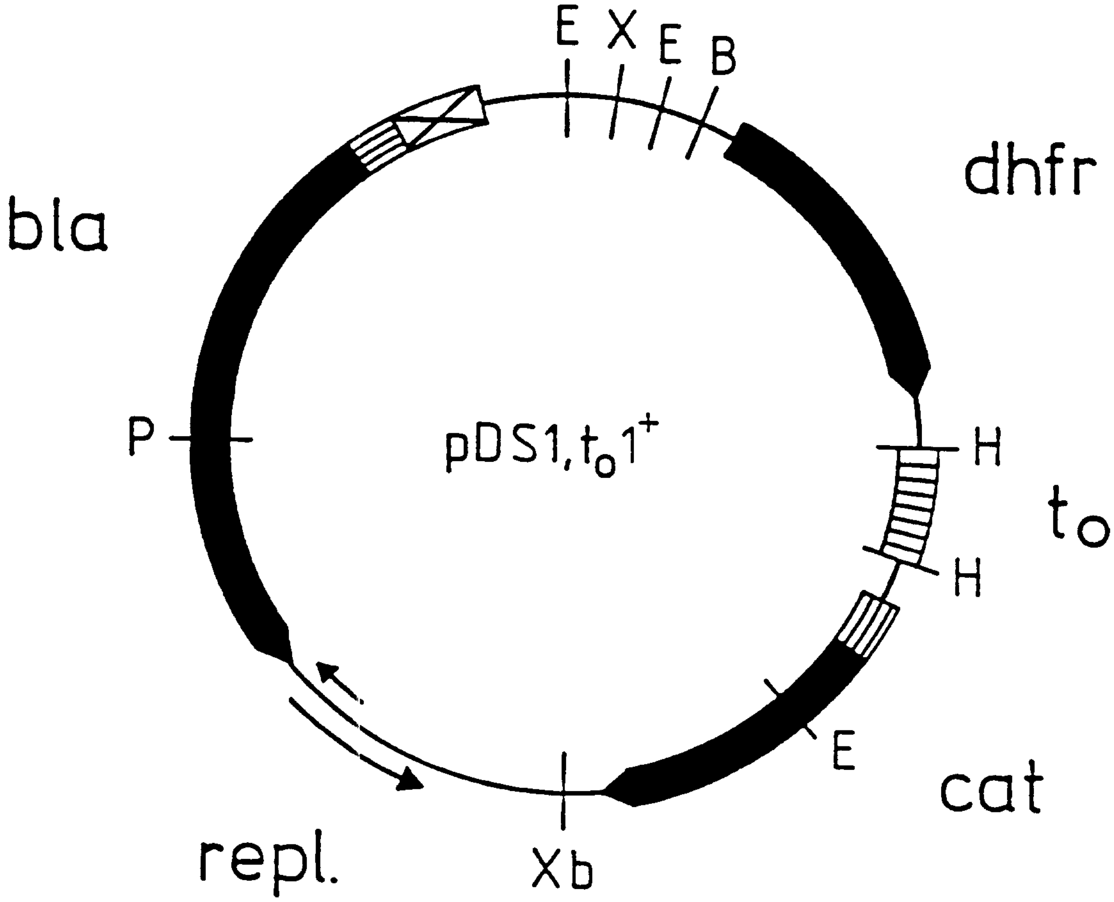

Abbildung 1b

```
              10         20         30         40         50
               |          |          |          |          |
   1 GAATTCCTCG AGGAATTCCG GATCCGGCAT CATGGTTCGA CCATTGAACT
  51 GCATCGTCGC CGTGTCCCAA AATATGGGGA TTGGCAAGAA CGGAGACCTA
 101 CCCTGGCCTC CGCTCAGGAA CGAGTTCAAG TACTTCCAAA GAATGACCAC
 151 AACCTCTTCA GTGGAAGGTA AACAGAATCT GGTGATTATG GGTAGGAAAA
 201 CCTGGTTCTC CATTCCTGAG AAGAATCGAC CTTTAAAGGA CAGAATTAAT
 251 ATAGTTCTCA GTAGAGAACT CAAAGAACCA CCACGAGGAG CTCATTTTCT
 301 TGCCAAAAGT TTGGATGATG CCTTAAGACT TATTGAACAA CCGGAATTGG
 351 CAAGTAAAGT AGACATGGTT TGGATAGTCG GAGGCAGTTC TGTTTACCAG
 401 GAAGCCATGA ATCAACCAGG CCACCTTAGA CTCTTTGTGA CAAGGATCAT
 451 GCAGGAATTT GAAAGTGACA CGTTTTTCCC AGAAATTGAT TTGGGGAAAT
 501 ATAAACTTCT CCCAGAATAC CCAGGCGTCC TCTCTGAGGT CCAGGAGGAA
 551 AAAGGCATCA AGTATAAGTT TGAAGTCTAC GAGAAGAAAG ACTAACAGGA
 601 AGATGCTTTC AAGTTCTCTG CTCCCCTCCT AAAGCTATGC ATTTTTATAA
 651 GACCATGGGA CTTTTGCTGG CTTTAGATCC GGCCAAGCTT GGACTCCTGT
 701 TGATAGATCC AGTAATGACC TCAGAACTCC ATCTGGATTT GTTCAGAACG
 751 CTCGGTTGCC GCCGGGCGTT TTTTATTGGT GAGAATCCAA GCTTGGCGAG
 801 ATTTTCAGGA GCTAAGGAAG CTAAAATGGA GAAAAAAATC ACTGGATATA
 851 CCACCGTTGA TATATCCCAA TGGCATCGTA AAGAACATTT TGAGGCATTT
 901 CAGTCAGTTG CTCAATGTAC CTATAACCAG ACCGTTCAGC TGGATATTAC
 951 GGCCTTTTTA AAGACCGTAA AGAAAAATAA GCACAAGTTT TATCCGGCCT
1001 TTATTCACAT TCTTGCCCGC CTGATGAATG CTCATCCGGA ATTCCGTATG
1051 GCAATGAAAG ACGGTGAGCT GGTGATATGG GATAGTGTTC ACCCTTGTTA
1101 CACCGTTTTC CATGAGCAAA CTGAAACGTT TTCATCGCTC TGGAGTGAAT
1151 ACCACGACGA TTTCCGGCAG TTTCTACACA TATATTCGCA AGATGTGGCG
```

Abbildung 1b (Fortsetzung)

1201 TGTTACGGTG AAAACCTGGC CTATTTCCCT AAAGGGTTTA TTGAGAATAT

1251 GTTTTTCGTC TCAGCCAATC CCTGGGTGAG TTTCACCAGT TTTGATTTAA

1301 ACGTGGCCAA TATGGACAAC TTCTTCGCCC CCGTTTTCAC CATGGGCAAA

1351 TATTATACGC AAGGCGACAA GGTGCTGATG CCGCTGGCGA TTCAGGTTCA

1401 TCATGCCGTC TGTGATGGCT TCCATGTCGG CAGAATGCTT AATGAATTAC

1451 AACAGTACTG CGATGAGTGG CAGGGCGGGG CGTAATTTTT TTAAGGCAGT

1501 TATTGGTGCC CTTAAACGCC TGGGGTAATG ACTCTCTAGA GCTGCCTCGC

1551 GCGTTTCGGT GATGACGGTG AAAACCTCTG ACACATGCAG CTCCCGGAGA

1601 CGGTCACAGC TTGTCTGTAA GCGGATGCCG GGAGCAGACA AGCCCGTCAG

1651 GGCGCGTCAG CGGGTGTTGG CGGGTGTCGG GGCGCAGCCA TGACCCAGTC

1701 ACGTAGCGAT AGCGGAGTGT ATACTGGCTT AACTATGCGG CATCAGAGCA

1751 GATTGTACTG AGAGTGCACC ATATGCGGTG TGAAATACCG CACAGATGCG

1801 TAAGGAGAAA ATACCGCATC AGGCGCTCTT CCGCTTCCTC GCTCACTGAC

1851 TCGCTGCGCT CGGTCTGTCG GCTGCGGCGA GCGGTATCAG CTCACTCAAA

1901 GGCGGTAATA CGGTTATCCA CAGAATCAGG GGATAACGCA GGAAAGAACA

1951 TGTGAGCAAA AGGCCAGCAA AAGGCCAGGA ACCGTAAAAA GGCCGCGTTG

2001 CTGGCGTTTT TCCATAGGCT CCGCCCCCCT GACGAGCATC ACAAAAATCG

2051 ACGCTCAAGT CAGAGGTGGC GAAACCCGAC AGGACTATAA AGATACCAGG

2101 CGTTTCCCCC TGGAAGCTCC CTCGTGCGCT CTCCTGTTCC GACCCTGCCG

2151 CTTACCGGAT ACCTGTCCGC CTTTCTCCCT TCGGGAAGCG TGGCGCTTTC

2201 TCAATGCTCA CGCTGTAGGT ATCTCAGTTC GGTGTAGGTC GTTCGCTCCA

2251 AGCTGGGCTG TGTGCACGAA CCCCCCGTTC AGCCCGACCG CTGCGCCTTA

2301 TCCGGTAACT ATCGTCTTGA GTCCAACCCG GTAAGACACG ACTTATCGCC

2351 ACTGGCAGCA GCCACTGGTA ACAGGATTAG CAGAGCGAGG TATGTAGGCG

2401 GTGCTACAGA GTTCTTGAAG TGGTGGCCTA ACTACGGCTA CACTAGAAGG

2451 ACAGTATTTG GTATCTGCGC TCTGCTGAAG CCAGTTACCT TCGGAAAAAG

2501 AGTTGGTAGC TCTTGATCCG GCAAACAAAC CACCGCTGGT AGCGGTGGTT

Abbildung 1b (Fortsetzung)

2551 TTTTTGTTTG CAAGCAGCAG ATTACGCGCA GAAAAAAAGG ATCTCAAGAA
2601 GATCCTTTGA TCTTTTCTAC GGGGTCTGAC GCTCAGTGGA ACGAAAACTC
2651 ACGTTAAGGG ATTTTGGTCA TGAGATTATC AAAAAGGATC TTCACCTAGA
2701 TCCTTTTAAA TTAAAAATGA AGTTTTAAAT CAATCTAAAG TATATATGAG
2751 TAAACTTGGT CTGACAGTTA CCAATGCTTA ATCAGTGAGG CACCTATCTC
2801 AGCGATCTGT CTATTTCGTT CATCCATAGC TGCCTGACTC CCCGTCGTGT
2851 AGATAACTAC GATACGGGAG GGCTTACCAT CTGGCCCCAG TGCTGCAATG
2901 ATACCGCGAG ACCCACGCTC ACCGGCTCCA GATTTATCAG CAATAAACCA
2951 GCCAGCCGGA AGGGCCGAGC GCAGAAGTGG TCCTGCAACT TTATCCGCCT
3001 CCATCCAGTC TATTAATTGT TGCCGGGAAG CTAGAGTAAG TAGTTCGCCA
3051 GTTAATAGTT TGCGCAACGT TGTTGCCATT GCTGCAGGCA TCGTGGTGTC
3101 ACGCTCGTCG TTTGGTATGG CTTCATTCAG CTCCGGTTCC CAACGATCAA
3151 GGCGAGTTAC ATGATCCCCC ATGTTGTGCA AAAAAGCGGT TAGCTCCTTC
3201 GGTCCTCCGA TCGTTGTCAG AAGTAAGTTG GCCGCAGTGT TATCACTCAT
3251 GGTTATGGCA GCACTGCATA ATTCTCTTAC TGTCATGCCA TCCGTAAGAT
3301 GCTTTTCTGT GACTGGTGAG TACTCAACCA AGTCATTCTG AGAATAGTGT
3351 ATGCGGCGAC CGAGTTGCTC TTGCCCGGCG TCAACACGGG ATAATACCGC
3401 GCCACATAGC AGAACTTTAA AAGTGCTCAT CATTGGAAAA CGTTCTTCGG
3451 GGCGAAAACT CTCAAGGATC TTACCGCTGT TGAGATCCAG TTCGATGTAA
3501 CCCACTCGTG CACCCAACTG ATCTTCAGCA TCTTTTACTT TCACCAGCGT
3551 TTCTGGGTGA GCAAAAACAG GAAGGCAAAA TGCCGCAAAA AAGGGAATAA
3601 GGGCGACACG GAAATGTTGA ATACTCATAC TCTTCCTTTT TCAATATTAT
3651 TGAAGCATTT ATCAGGGTTA TTGTCTCATG AGCGGATACA TATTTGAATG
3701 TATTTAGAAA AATAAACAAA TAGGGGTTCC GCGCACATTT CCCCGAAAAG
3751 TGCCACCTGA CGTCTAAGAA ACCATTATTA TCATGACATT AACCTATAAA
3801 AATAGGCGTA TCACGAGGCC CTTTCGTCTT CAA

Abbildung 2a

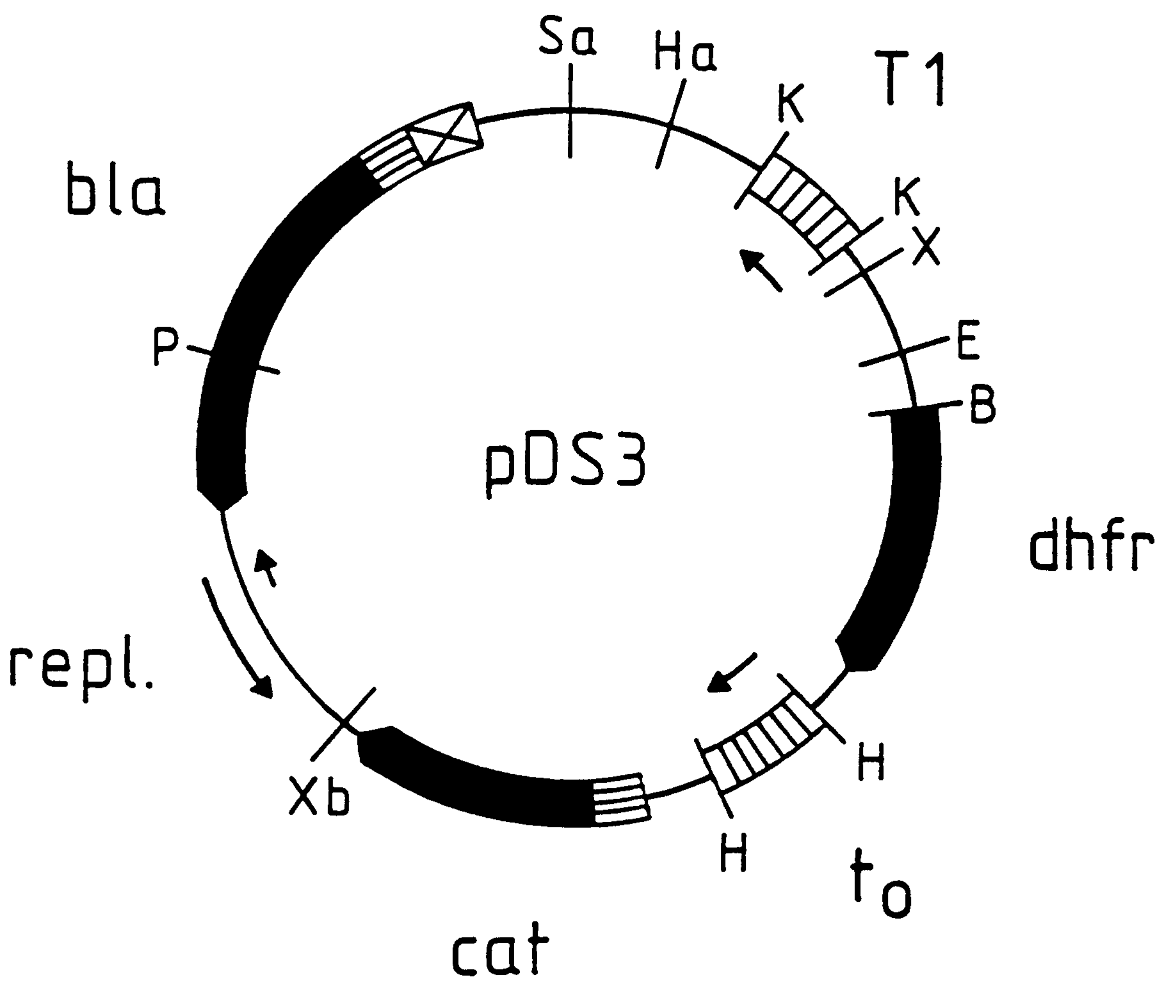

Abbildung 2b

| | 10 | 20 | 30 | 40 | 50 |
|---|---|---|---|---|---|
| 1 | CCTCGACGTC | GACGTTAACG | GTACCGAGCT | TGTGGCAGTT | TAAGGCGGGC |
| 51 | GTCCTGCCCG | CCACCCTCCG | GGCCGTTGCT | TCGCAACGTT | CAAATCCGCT |
| 101 | CCCGGCGGAT | TTGTCCTACT | CAGGAGAGCG | TTCACCGACA | AACAACAGAT |
| 151 | AAAACGAAAG | GCCCAGTCTT | TCGACTGAGC | CTTTCGTTTT | ATTTGATGCC |
| 201 | TCAAGCTCGG | TACCTCGAGG | GAATTCCGGA | TCCGGCATCA | TGGTTCGACC |
| 251 | ATTGAACTGC | ATCGTCGCCG | TGTCCCAAAA | TATGGGGATT | GGCAAGAACG |
| 301 | GAGACCTACC | CTGGCCTCCG | CTCAGGAACG | AGTTCAAGTA | CTTCCAAAGA |
| 351 | ATGACCACAA | CCTCTTCAGT | GGAAGGTAAA | CAGAATCTGG | TGATTATGGG |
| 401 | TAGGAAAACC | TGGTTCTCCA | TTCCTGAGAA | GAATCGACCT | TTAAAGGACA |
| 451 | GAATTAATAT | AGTTCTCAGT | AGAGAACTCA | AAGAACCACC | ACGAGGAGCT |
| 501 | CATTTTCTTG | CCAAAAGTTT | GGATGATGCC | TTAAGACTTA | TTGAACAACC |
| 551 | GGAATTGGCA | AGTAAAGTAG | ACATGGTTTG | GATAGTCGGA | GGCAGTTCTG |
| 601 | TTTACCAGGA | AGCCATGAAT | CAACCAGGCC | ACCTTAGACT | CTTTGTGACA |
| 651 | AGGATCATGC | AGGAATTTGA | AAGTGACACG | TTTTTCCCAG | AAATTGATTT |
| 701 | GGGGAAATAT | AAACTTCTCC | CAGAATACCC | AGGCGTCCTC | TCTGAGGTCC |
| 751 | AGGAGGAAAA | AGGCATCAAG | TATAAGTTTG | AAGTCTACGA | GAAGAAAGAC |
| 801 | TAACAGGAAG | ATGCTTTCAA | GTTCTCTGCT | CCCCTCCTAA | AGCTATGCAT |
| 851 | TTTTATAAGA | CCATGGGACT | TTTGCTGGCT | TTAGATCCGG | CCAAGCTTGG |
| 901 | ACTCCTGTTG | ATAGATCCAG | TAATGACCTC | AGAACTCCAT | CTGGATTTGT |
| 951 | TCAGAACGCT | CGGTTGCCGC | CGGGCGTTTT | TTATTGGTGA | GAATCCAAAG |
| 1001 | CTTGGCGAGA | TTTTCAGGAG | CTAAGGAAGC | TAAAATGGAG | AAAAAAATCA |
| 1051 | CTGGATATAC | CACCGTTGAT | ATATCCCAAT | GGCATCGTAA | AGAACATTTT |
| 1101 | GAGGCATTTC | AGTCAGTTGC | TCAATGTACC | TATAACCAGA | CCGTTCAGCT |
| 1151 | GGATATTACG | GCCTTTTTAA | AGACCGTAAA | GAAAAATAAG | CACAAGTTTT |
| 1201 | ATCCGGCCTT | TATTCACATT | CTTGCCCGCC | TGATGAATGC | TCATCCGGAA |
| 1251 | TTTCGTATGG | CAATGAAAGA | CGGTGAGCTG | GTGATATGGG | ATAGTGTTCA |

Abbildung 2b (Fortsetzung)

1301 CCCTTGTTAC ACCGTTTTCC ATGAGCAAAC TGAAACGTTT TCATCGCTCT
1351 GGAGTGAATA CCACGACGAT TTCCGGCAGT TTCTACACAT ATATTCGCAA
1401 GATGTGGCGT GTTACGGTGA AAACCTGGCC TATTTCCCTA AAGGGTTTAT
1451 TGAGAATATG TTTTTCGTCT CAGCCAATCC CTGGGTGAGT TTCACCAGTT
1501 TTGATTTAAA CGTGGCCAAT ATGGACAACT TCTTCGCCCC CGTTTTCACC
1551 ATGGGCAAAT ATTATACGCA AGGCGACAAG GTGCTGATGC CGCTGGCGAT
1601 TCAGGTTCAT CATGCCGTCT GTGATGGCTT CCATGTCGGC AGAATGCTTA
1651 ATGAATTACA ACAGTACTGC GATGAGTGGC AGGGCGGGGC GTAATTTTTT
1701 TAAGGCAGTT ATTGGTGCCC TTAAACGCCT GGGGTAATGA CTCTCTAGAG
1751 CTGCCTCGCG CGTTTCGGTG ATGACGGTGA AAACCTCTGA CACATGCAGC
1801 TCCCGGAGAC GGTCACAGCT TGTCTGTAAG CGGATGCCGG GAGCAGACAA
1851 GCCCGTCAGG GCGCGTCAGC GGGTGTTGGC GGGTGTCGGG GCGCAGCCAT
1901 GACCCAGTCA CGTAGCGATA GCGGAGTGTA TACTGGCTTA ACTATGCGGC
1951 ATCAGAGCAG ATTGTACTGA GAGTGCACCA TATGCGGTGT GAAATACCGC
2001 ACAGATGCGT AAGGAGAAAA TACCGCATCA GGCGCTCTTC CGCTTCCTCG
2051 CTCACTGACT CGCTGCGCTC GGTCGTTCGG CTGCGGCGAG CGGTATCAGC
2101 TCACTCAAAG GCGGTAATAC GGTTATCCAC AGAATCAGGG GATAACGCAG
2151 GAAAGAACAT GTGAGCAAAA GGCCAGCAAA AGGCCAGGAA CCGTAAAAAG
2201 GCCGCGTTGC TGGCGTTTTT CCATAGGCTC CGCCCCCCTG ACGAGCATCA
2251 CAAAAATCGA CGCTCAAGTC AGAGGTGGCG AAACCCGACA GGACTATAAA
2301 GATACCAGGC GTTTCCCCCT GGAAGCTCCC TCGTGCGCTC TCCTGTTCCG
2351 ACCCTGCCGC TTACCGGATA CCTGTCCGCC TTTCTCCCTT CGGGAAGCGT
2401 GGCGCTTTCT CAATGCTCAC GCTGTAGGTA TCTCAGTTCG GTGTAGGTCG
2451 TTCGCTCCAA GCTGGGCTGT GTGCACGAAC CCCCCGTTCA GCCCGACCGC
2501 TGCGCCTTAT CCGGTAACTA TCGTCTTGAG TCCAACCCGG TAAGACACGA
2551 CTTATCGCCA CTGGCAGCAG CCACTGGTAA CAGGATTAGC AGAGCGAGGT
2601 ATGTAGGCGG TGCTACAGAG TTCTTGAAGT GGTGGCCTAA CTACGGCTAC

Abbildung 2b (Fortsetzung)

2651 ACTAGAAGGA CAGTATTTGG TATCTGCGCT CTGCTGAAGC CAGTTACCTT
2701 CGGAAAAAGA GTTGGTAGCT CTTGATCCGG CAAACAAACC ACCGCTGGTA
2751 GCGGTGGTTT TTTTGTTTGC AAGCAGCAGA TTACGCGCAG AAAAAAAGGA
2801 TCTCAAGAAG ATCCTTTGAT CTTTTCTACG GGGTCTGACG CTCAGTGGAA
2851 CGAAAACTCA CGTTAAGGGA TTTTGGTCAT GAGATTATCA AAAAGGATCT
2901 TCACCTAGAT CCTTTTAAAT TAAAAATGAA GTTTTAAATC AATCTAAAGT
2951 ATATATGAGT AAACTTGGTC TGACAGTTAC CAATGCTTAA TCAGTGAGGC
3001 ACCTATCTCA GCGATCTGTC TATTTCGTTC ATCCATAGTT GCCTGACTCC
3051 CCGTCGTGTA GATAACTACG ATACGGGAGG GCTTACCATC TGGCCCCAGT
3101 GCTGCAATGA TACCGCGAGA CCCACGCTCA CCGGCTCCAG ATTTATCAGC
3151 AATAAACCAG CCAGCCGGAA GGGCCGAGCG CAGAAGTGGT CCTGCAACTT
3201 TATCCGCCTC CATCCAGTCT ATTAATTGTT GCCGGGAAGC TAGAGTAAGT
3251 AGTTCGCCAG TTAATAGTTT GCGCAACGTT GTTGCCATTG CTGCAGGCAT
3301 CGTGGTGTCA CGCTCGTCGT TTGGTATGGC TTCATTCAGC TCCGGTTCCC
3351 AACGATCAAG GCGAGTTACA TGATCCCCCA TGTTGTGCAA AAAAGCGGTT
3401 AGCTCCTTCG GTCCTCCGAT CGTTGTCAGA AGTAAGTTGG CCGCAGTGTT
3451 ATCACTCATG GTTATGGCAG CACTGCATAA TTCTCTTACT GTCATGCCAT
3501 CCGTAAGATG CTTTTCTGTG ACTGGTGAGT ACTCAACCAA GTCATTCTGA
3551 GAATAGTGTA TGCGGCGACC GAGTTGCTCT TGCCCGGCGT CAACACGGGA
3601 TAATACCGCG CCACATAGCA GAACTTTAAA AGTGCTCATC ATTGGAAAAC
3651 GTTCTTCGGG GCGAAAACTC TCAAGGATCT TACCGCTGTT GAGATCCAGT
3701 TCGATGTAAC CCACTCGTGC ACCCAACTGA TCTTCAGCAT CTTTTACTTT
3751 CACCAGCGTT TCTGGGTGAG CAAAAACAGG AAGGCAAAAT GCCGCAAAAA
3801 AGGGAATAAG GGCGACACGG AAATGTTGAA TACTCATACT CTTCCTTTTT
3851 CAATATTATT GAAGCATTTA TCAGGGTTAT TGTCTCATGA GCGGATACAT
3901 ATTTGAATGT ATTTAGAAAA ATAAACAAAT AGGGGTTCCG CGCACATTTC
3951 CCCGAAAAGT GCCACCTGAC GTCTAAGAAA CCATTATTAT CATGACATTA
4001 ACCTATAAAA ATAGGCGTAT CACGAGGCCC TTTCGTCTTC A

Abbildung 3a

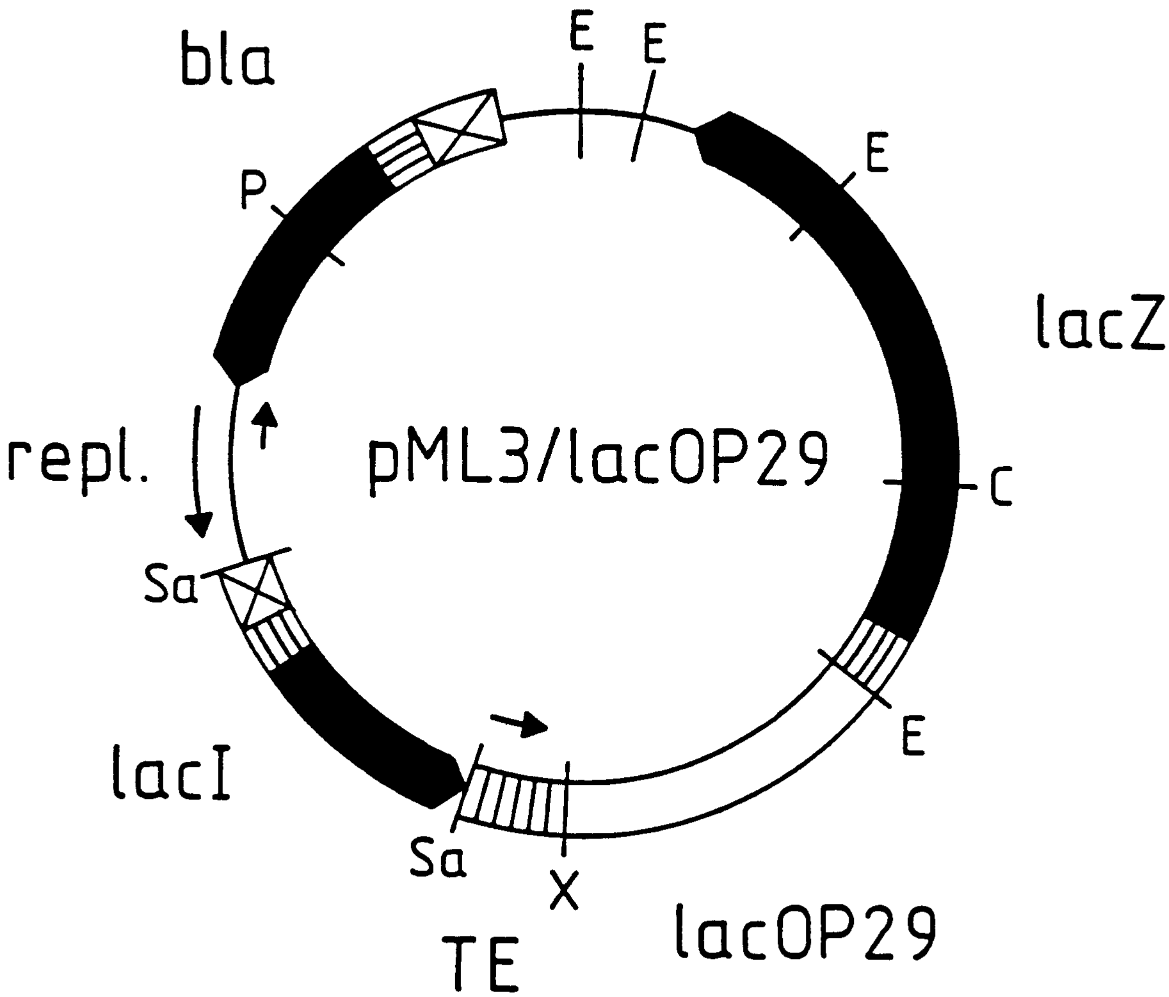

Abbildung 3b

```
             10          20          30          40          50

   1 GAATTC.... .......... .......... .......... ..........
  51 .......... .......... .......... .......... ..........
 101 .......... .......... .......... .......... ..........
 151 GAATTC.... .......... .......... .......AAA TAGTACATAA
 201 TGGATTTCCT TACGCGAAAT ACGGGCAGAC ATGGCCTGCC CGGTTATTAT
 251 TATTTTTGAC ACCAGACCAA CTGGTAATGG TAGCGACCGG CGCTCAGCTG
 301 GAATTCCGCC GATACTGACG GGCTCCAGGA GTCGTCGCCA CCAATCCCCA
 351 TATGGAAACC GTCGATATTC AGCCATGTGC CTTCTTCCGC GTGCAGCAGA
 401 TGGCGATGGC TGGTTTCCAT CAGTTGCTGT TGACTGTAGC GGCTGATGTT
 451 GAACTGGAAG TCGCCGCGCC ACTGGTGTGG GCCATAATTC AATTCGCGCG
 501 TCCCGCAGCG CAGACCGTTT TCGCTCGGGA AGACGTACGG GGTATACATG
 551 TCTGACAATG GCAGATCCCA GCGGTCAAAA CAGGCGGCAG TAAGGCGGTC
 601 GGGATAGTTT TCTTGCGGCC CTAATCCGAG CCAGTTTACC CGCTCTGCTA
 651 CCTGCGCCAG CTGGCAGTTC AGGCCAATCC GCGCCGGATG CGGTGTATCG
 701 CTCGCCACTT CAACATCAAC GGTAATCGCC ATTTGACCAC TACCATCAAT
 751 CCGGTAGGTT TTCCGGCTGA TAAATAAGGT TTTCCCCTGA TGCTGCCACG
 801 CGTGAGCGGT CGTAATCAGC ACCGCATCAG CAAGTGTATC TGCCGTGCAC
 851 TGCAACAACG CTGCTTCGGC CTGGTAATGG CCCGCCGCCT TCCAGCGTTC
 901 GACCCAGGCG TTAGGGTCAA TGCGGGTCGC TTCACTTACG CCAATGTCGT
 951 TATCCAGCGG TGCACGGGTG AACTGATCGC GCAGCGGCGT CAGCAGTTGT
1001 TTTTTATCGC CAATCCACAT CTGTGAAAGA AAGCCTGACT GGCGGTTAAA
1051 TTGCCAACGC TTATTACCCA GCTCGATGCA AAAATCCATT TCGCTGGTGG
1101 TCAGATGCGG GATGGCGTGG GACGCGGCGG GGAGCGTCAC ACTGAGGTTT
1151 TCCGCCAGAC GCCACTGCTG CCAGGCGCTG ATGTGCCCGG CTTCTGACCA
1201 TGCGGTCGCG TTCGGTTGCA CTACGCGTAC TGTGAGCCAG AGTTGCCCGG
1251 CGCTCTCCGG CTGCGGTAGT TCAGGCAGTT CAATCAACTG TTTACCTTGT
```

Abbildung 3b (Fortsetzung)

1301 GGAGCGACAT CCAGAGGCAC TTCACCGCTT GCCAGCGGCT TACCATCCAG

1351 CGCCACCATC CAGTGCAGGA GCTCGTTATC GCTATGACGG AACAGGTATT

1401 CGCTGGTCAC TTCGATGGTT TGCCCGGATA AACGGAACTG GAAAAACTGC

1451 TGCTGGTGTT TTGCTTCCGT CAGCGCTGGA TGCGGCGTGC GGTCGGCAAA

1501 GACCAGACCG TTCATACAGA ACTGGCGATC GTTCGGCGTA TCGCCAAAAT

1551 CACCGCCGTA AGCCGACCAC GGGTTGCCGT TTTCATCATA TTTAATCAGC

1601 GACTGATCCA CCCAGTCCCA GACGAAGCCG CCCTGTAAAC GGGGATACTG

1651 ACGAAACGCC TGCCAGTATT TAGCGAAACC GCCAAGACTG TTACCCATCG

1701 CGTGGGCGTA TTCGCAAAGG ATCAGCGGGC GCGTCTCTCC AGGTAGCGAA

1751 AGCCATTTTT TGATGGACCA TTTCGGCACA GCCGGGAAGG GCTGGTCTTC

1801 ATCCACGCGC GCGTACATCG GGCAAATAAT ATCGGTGGCC GTGGTGTCGG

1851 CTCCGCCGCC TTCATACTGC ACCGGGCGGG AAGGATCGAC AGATTTGATC

1901 CAGCGATACA GCGCGTCGTG ATTAGCGCCG TGGCCTGATT CATTCCCCAG

1951 CGACCAGATG ATCACACTCG GGTGATTACG ATCGCGCTGC ACCATTCGCG

2001 TTACGCGTTC GCTCATCGCC GGTAGCCAGC GCGGATCATC GGTCAGACGA

2051 TTCATTGGCA CCATGCCGTG GGTTTCAATA TTGGCTTCAT CCACCACATA

2101 CAGGCCGTAG CGGTCGCACA GCGTGTACCA CAGCGGATGG TTCGGATAAT

2151 GCGAACAGCG CACGGCGTTA AAGTTGTTCT GCTTCATCAG CAGGATATCC

2201 TGCACCATCG TCTGCTCATC CATGACCTGA CCATGCAGAG GATGATGCTC

2251 GTGACGGTTA ACGCCTCGAA TCAGCAACGG CTTGCCGTTC AGCAGCAGCA

2301 GACCATTTTC AATCCGCACC TCGCGGAAAC CGACATCGCA GGCTTCTGCT

2351 TCAATCAGCG TGCCGTCGGC GGTGTGCAGT TCAACCACCG CACGATAGAG

2401 ATTCGGGATT TCGGCGCTCC ACAGTTTCGG GTTTTCGACG TTCAGACGTA

2451 GTGTGACGCG ATCGGCATAA CCACCACGCT CATCGATAAT TTCACCGCCG

2501 AAAGGCGCGG TGCCGCTGGC GACCTGCGTT TCACCCTGCC ATAAAGAAAC

2551 TGTTACCCGT AGGTAGTCAC GCAACTCGCC GCACATCTGA ACTTCAGCCT

2601 CCAGTACAGC GCGGCTGAAA TCATCATTAA AGCGAGTGGC AACATGGAAA

Abbildung 3b (Fortsetzung)

2651 TCGCTGATTT GTGTAGTCGG TTTATGCAGC AACGAGACGT CACGGAAAAT
2701 GCCGCTCATC CGCCACATAT CCTGATCTTC CAGATAACTG CCGTCACTCC
2751 AACGCAGCAC CATCACCGCG AGGCGGTTTT CTCCGGCGCG TAAAAATGCG
2801 CTCAGGTCAA ATTCAGACGG CAAACGACTG TCCTGGCCGT AACCGACCCA
2851 GCGCCCGTTG CACCACAGAT GAAACGCCGA GTTAACGCCA TCAAAAATAA
2901 TTCGCGTCTG GCCTTCCTGT AGCCAGCTTT CATCAACATT AAATGTGAGC
2951 GAGTAACAAC CCGTCGGATT CTCCGTGGGA ACAAACGGCG GATTGACCGT
3001 AATGGGATAG GTTACGTTGG TGTAGATGGG CGCATCGTAA CCGTGCATCT
3051 GCCAGTTTGA GGGGACGACG ACAGTATCGG CCTCAGGAAG ATCGCACTCC
3101 AGCCAGCTTT CCGGCACCGC TTCTGGTGCC GGAAACCAGG CAAAGCGCCA
3151 TTCGCCATTC AGGCTGCGCA ACTGTTGGGA AGGGCGATCG GTGCGGGCCT
3201 CTTCGCTATT ACGCCAGCTG GCGAAAGGGG GATGTGCTGC AAGGCGATTA
3251 AGTTGGGTAA CGCCAGGGTT TTCCCAGTCA CGACGTTGTA AAACGACGGC
3301 CAGTGAATCC GTAATCATGG TCATAGCTGT TTCCTGTGTG AATTCTGTGT
3351 GAAATTGTTA TCCGCTCACA ATTCCACACA ACATACGAGC CGGAAGCATA
3401 AAGTGTAAAG CCTGGGGTGC CTAATGAGTG AGCTAACTCA CATTAATTGC
3451 GTTGCCTCGA GGTACCTTAT AAACGCAGAA AGGCCCACCC GAAGGTGAGC
3501 CAGTTAACGT CGACAATTCG CGCTAACTTA CATTAATTGC GTTGCGCTCA
3551 CTGCCCGCTT TCCAGTCGGG AAACCTGTCG TGCCAGCTGC ATTAATGAAT
3601 CGGCCAACGC GCGGGGAGAG GCGGTTTGCG TATTGGGCGC CAGGGTGGTT
3651 TTTCTTTTCA CCAGTGAGAC GGGCAACAGC TGATTGCCCT TCACCGCCTG
3701 GCCCTGAGAG AGTTGCAGCA AGCGGTCCAC GCTGGTTTGC CCCAGCAGGC
3751 GAAAATCCTG TTTGATGGTG GTTAACGGCG GGATATAACA TGAGCTGTCT
3801 TCGGTATCGT CGTATCCCAC TACCGAGATA TCCGCACCAA CGCGCAGCCC
3851 GGACTCGGTA ATGGCGCGCA TTGCGCCCAG CGCCATCTGA TCGTTGGCAA
3901 CCAGCATCGC AGTGGGAACG ATGCCCTCAT TCAGCATTTG CATGGTTTGT
3951 TGAAAACCGG ACATGGCACT CCAGTCGCCT TCCCGTTCCG CTATCGGCTG

Abbildung 3b (Fortsetzung)

4001 AATTTGATTG CGAGTGAGAT ATTTATGCCA GCCAGCCAGA CGCAGACGCG

4051 CCGAGACAGA ACTTAATGGG CCCGCTAACA GCGCGATTTG CTGGTGACCC

4101 AATGCGACCA GATGCTCCAC GCCCAGTCGC GTACCGTCTT CATGGGAGAA

4151 AATAATACTG TTGATGGGTG TCTGGTCAGA GACATCAAGA AATAACGCCG

4201 GAACATTAGT GCAGGCAGCT TCCACAGCAA TGGCATCCTG GTCATCCAGC

4251 GGATAGTTAA TGATCAGCCC ACTGACGCGT TGCGCGAGAA GATTGTGCAC

4301 CGCCGCTTTA CAGGCTTCGA CGCCGCTTCG TTCTACCATC GACACCACCA

4351 CGCTGGCACC CAGTTGATCG GCGCGAGATT TAATCGCCGC GACAATTTGC

4401 GACGGCGCGT GCAGGGCCAG ACTGGAGGTG GCAACGCCAA TCAGCAACGA

4451 CTGTTTGCCC GCCAGTTGTT GTGCCACGCG GTTGGGAATG TAATTCAGCT

4501 CCGCCATCGC CGCTTCCACT TTTTCCCGCG TTTTCGCAGA AACGTGGCTG

4551 GCCTGGTTCA CCACGCGGGA AACGGTCTGA TAAGAGACAC CGGCATACTC

4601 TGCGACATCG TATAACGTTA CTGGTTTCAC ATTCACCACC CTGAATTGAC

4651 TCTCTTCCGG GCGCTATCAT GCCATACCGC GAAAGGTTTT GCACCATTCG

4701 ATGGTGTCAA CGTAAATGCA TGCCGCTTCG CCTTCGCGCG CGAATTGTCG

4751 ACTAGAGCTG CCTCGCGCGT TTCGGTGATG ACGGTGAAAA CCTCTGACAC

4801 ATGCAGCTCC CGGAGACGGT CACAGCTTGT CTGTAAGCGG ATGCCGGGAG

4851 CAGACAAGCC CGTCAGGGCG CGTCAGCGGG TGTTGGCGGG TGTCGGGGCG

4901 CAGCCATGAC CCAGTCACGT AGCGATAGCG GAGTGTATAC TGGCTTAACT

4951 ATGCGGCATC AGAGCAGATT GTACTGAGAG TGCACCATAT GCGGTGTGAA

5001 ATACCGCACA GATGCGTAAG GAGAAAATAC CGCATCAGGC GCTCTTCCGC

5051 TTCCTCGCTC ACTGACTCGC TGCGCTCGGT CGTTCGGCTG CGGCGAGCGG

5101 TATCAGCTCA CTCAAAGGCG GTAATACGGT TATCCACAGA ATCAGGGGAT

5151 AACGCAGGAA AGAACATGTG AGCAAAAGGC CAGCAAAAGG CCAGGAACCG

5201 TAAAAAGGCC GCGTTGCTGG CGTTTTTCCA TAGGCTCCGC CCCCCTGACG

5251 AGCATCACAA AAATCGACGC TCAAGTCAGA GGTGGCGAAA CCCGACAGGA

5301 CTATAAAGAT ACCAGGCGTT TCCCCCTGGA AGCTCCCTCG TGCGCTCTCC

Abbildung 3b (Fortsetzung)

5351 TGTTCCGACC CTGCCGCTTA CCGGATACCT GTCCGCCTTT CTCCCTTCGG
5401 GAAGCGTGGC GCTTTCTCAA TGCTCACGCT GTAGGTATCT CAGTTCGGTG
5451 TAGGTCGTTC GCTCCAAGCT GGGCTGTGTG CACGAACCCC CCGTTCAGCC
5501 CGACCGCTGC GCCTTATCCG GTAACTATCG TCTTGAGTCC AACCCGGTAA
5551 GACACGACTT ATCGCCACTG GCAGCAGCCA CTGGTAACAG GATTAGCAGA
5601 GCGAGGTATG TAGGCGGTGC TACAGAGTTC TTGAAGTGGT GGCCTAACTA
5651 CGGCTACACT AGAAGGACAG TATTTGGTAT CTGCGCTCTG CTGAAGCCAG
5701 TTACCTTCGG AAAAAGAGTT GGTAGCTCTT GATCCGGCAA ACAAACCACC
5751 GCTGGTAGCG GTGGTTTTTT TGTTTGCAAG CAGCAGATTA CGCGCAGAAA
5801 AAAAGGATCT CAAGAAGATC CTTTGATCTT TTCTACGGGG TCTGACGCTC
5851 AGTGGAACGA AAACTCACGT TAAGGGATTT TGGTCATGAG ATTATCAAAA
5901 AGGATCTTCA CCTAGATCCT TTTAAATTAA AAATGAAGTT TTAAATCAAT
5951 CTAAAGTATA TATGAGTAAA CTTGGTCTGA CAGTTACCAA TGCTTAATCA
6001 GTGAGGCACC TATCTCAGCG ATCTGTCTAT TTCGTTCATC CATAGTTGCC
6051 TGACTCCCCG TCGTGTAGAT AACTACGATA CGGGAGGGCT TACCATCTGG
6101 CCCCAGTGCT GCAATGATAC CGCGAGACCC ACGCTCACCG GCTCCAGATT
6151 TATCAGCAAT AAACCAGCCA GCCGGAAGGG CCGAGCGCAG AAGTGGTCCT
6201 GCAACTTTAT CCGCCTCCAT CCAGTCTATT AATTGTTGCC GGGAAGCTAG
6251 AGTAAGTAGT TCGCCAGTTA ATAGTTTGCG CAACGTTGTT GCCATTGCTG
6301 CAGGCATCGT GGTGTCACGC TCGTCGTTTG GTATGGCTTC ATTCAGCTCC
6351 GGTTCCCAAC GATCAAGGCG AGTTACATGA TCCCCCATGT TGTGCAAAAA
6401 AGCGGTTAGC TCCTTCGGTC CTCCGATCGT TGTCAGAAGT AAGTTGGCCG
6451 CAGTGTTATC ACTCATGGTT ATGGCAGCAC TGCATAATTC TCTTACTGTC
6501 ATGCCATCCG TAAGATGCTT TTCTGTGACT GGTGAGTACT CAACCAAGTC
6551 ATTCTGAGAA TAGTGTATGC GGCGACCGAG TTGCTCTTGC CCGGCGTCAA
6601 CACGGGATAA TACCGCGCCA CATAGCAGAA CTTTAAAAGT GCTCATCATT
6651 GGAAAACGTT CTTCGGGGCG AAAACTCTCA AGGATCTTAC CGCTGTTGAG

Abbildung 3b (Fortsetzung)

6701 ATCCAGTTCG ATGTAACCCA CTCGTGCACC CAACTGATCT TCAGCATCTT
6751 TTACTTTCAC CAGCGTTTCT GGGTGAGCAA AAACAGGAAG GCAAAATGCC
6801 GCAAAAAAGG GAATAAGGGC GACACGGAAA TGTTGAATAC TCATACTCTT
6851 CCTTTTTCAA TATTATTGAA GCATTTATCA GGGTTATTGT CTCATGAGCG
6901 GATACATATT TGAATGTATT TAGAAAAATA AACAAATAGG GGTTCCGCGC
6951 ACATTTCCCC GAAAAGTGCC ACCTGACGTC TAAGAAACCA TTATTATCAT
7001 GACATTAACC TATAAAAATA GGCGTATCAC GAGGCCCTTT CGTCTTCAA

Abbildung 4a

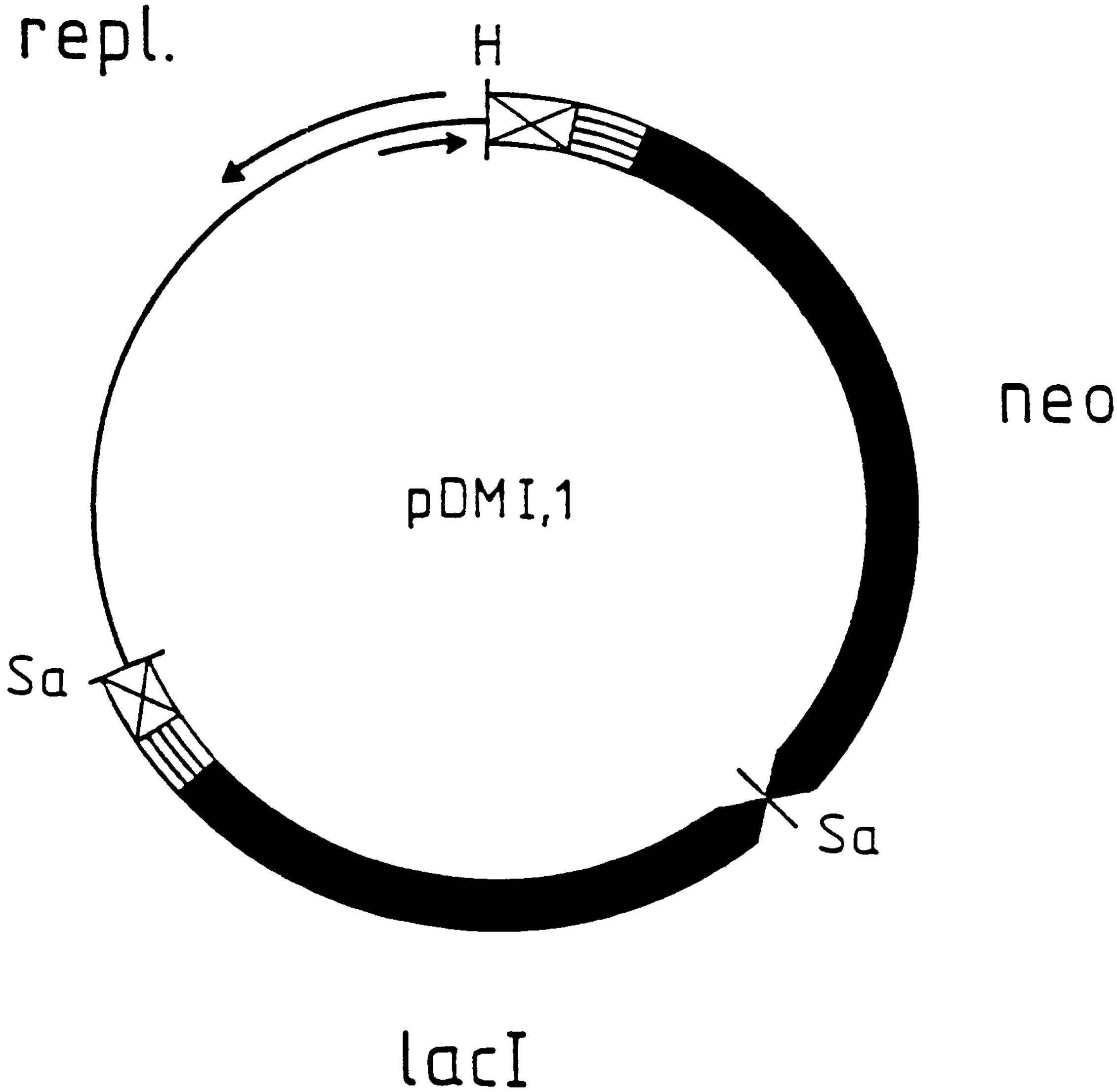

Abbildung 4b

| | 10 | 20 | 30 | 40 | 50 |
|---|---|---|---|---|---|
| 1 | AAGCTTCACG | CTGCCGCAAG | CACTCAGGGC | GCAAGGGCTG | CTAAAGGAAG |
| 51 | CGGAACACGT | AGAAAGCCAG | TCCGCAGAAA | CGGTGCTGAC | CCCGGATGAA |
| 101 | TGTCAGCTAC | TGGGCTATCT | GGACAAGGGA | AAACGCAAGC | GCAAAGAGAA |
| 151 | AGCAGGTAGC | TTGCAGTGGG | CTTACATGGC | GATAGCTAGA | CTGGGCGGTT |
| 201 | TTATGGACAG | CAAGCGAACC | GGAATTGCCA | GCTGGGGCGC | CCTCTGGTAA |
| 251 | GGTTGGGAAG | CCCTGCAAAG | TAAACTGGAT | GGCTTTCTTG | CCGCCAAGGA |
| 301 | TCTGATGGCG | CAGGGGATCA | AGATCTGATC | AAGAGACAGG | ATGAGGATCG |
| 351 | TTTCGCATGA | TTGAACAAGA | TGGATTGCAC | GCAGGTTCTC | CGGCCGCTTG |
| 401 | GGTGGAGAGG | CTATTCGGCT | ATGACTGGGC | ACAACAGACA | ATCGGCTGCT |
| 451 | CTGATGCCGC | CGTGTTCCGG | CTGTCAGCGC | AGGGGCGCCC | GGTTCTTTTT |
| 501 | GTCAAGACCG | ACCTGTCCGG | TGCCCTGAAT | GAACTGCAGG | ACGAGGCAGC |
| 551 | GCGGCTATCG | TGGCTGGCCA | CGACGGGCGT | TCCTTGCGCA | GCTGTGCTCG |
| 601 | ACGTTGTCAC | TGAAGCGGGA | AGGGACTGGC | TGCTATTGGG | CGAAGTGCCG |
| 651 | GGGCAGGATC | TCCTGTCATC | TCACCTTGCT | CCTGCCGAGA | AAGTATCCAT |
| 701 | CATGGCTGAT | GCAATGCGGC | GGCTGCATAC | GCTTGATCCG | GCTACCTGCC |
| 751 | CATTCGACCA | CCAAGCGAAA | CATCGCATCG | AGCGAGCACG | TACTCGGATG |
| 801 | GAAGCCGGTC | TTGTCGATCA | GGATGATCTG | GACGAAGAGC | ATCAGGGGCT |
| 851 | CGCGCCAGCC | GAACTGTTCG | CCAGGCTCAA | GGCGCGCATG | CCCGACGGCG |
| 901 | AGGATCTCGT | CGTGACCCAT | GGCGATGCCT | GCTTGCCGAA | TATCATGGTG |
| 951 | GAAAATGGCC | GCTTTTCTGG | ATTCATCGAC | TGTGGCCGGC | TGGGTGTGGC |
| 1001 | GGACCGCTAT | CAGGACATAG | CGTTGGCTAC | CCGTGATATT | GCTGAAGAGC |
| 1051 | TTGGCGGCGA | ATGGGCTGAC | CGCTTCCTCG | TGCTTTACGG | TATCGCCGCT |
| 1101 | CCCGATTCGC | AGCGCATCGC | CTTCTATCGC | CTTCTTGACG | AGTTCTTCTG |
| 1151 | AGCGGGACTC | TGGGGTTCGA | AATGACCGAC | CAAGCGACGC | CCAACCTGCC |

Abbildung 4b (Fortsetzung)

```
1201 ATCACGAGAT TTCGATTCCA CCGCCGCCTT CTATGAAAGG TTGGGCTTCG
1251 GAATCGTTTT CCGGGACGCC GGCTGGATGA TCCTCCAGCG CGGGGATCTC
1301 ATGCTGGAGT TCTTCGCCCA CCCCGGGCTC GATCCCCTCG CGAGTTGGTT
1351 CAGCTGCTGC CTGAGGCTGG ACGACCTCGC GGAGTTCTAC CGGCAGTGCA
1401 AATCCGTCGG CATCCAGGAA ACCAGCAGCG GCTATCCGCG CATCCATGCC
1451 CCCGAACTGC AGGAGTGGGG AGGCACGATG GCCGCTTTGG TCGACAATTC
1501 GCGCTAACTT ACATTAATTG CGTTGCGCTC ACTGCCCGCT TTCCAGTCGG
1551 GAAACCTGTC GTGCCAGCTG CATTAATGAA TCGGCCAACG CGCGGGGAGA
1601 GGCGGTTTGC GTATTGGGCG CCAGGGTGGT TTTTCTTTTC ACCAGTGAGA
1651 CGGGCAACAG CTGATTGCCC TTCACCGCCT GGCCCTGAGA GAGTTGCAGC
1701 AAGCGGTCCA CGCTGGTTTG CCCCAGCAGG CGAAAATCCT GTTTGATGGT
1751 GGTTAACGGC GGGATATAAC ATGAGCTGTC TTCGGTATCG TCGTATCCCA
1801 CTACCGAGAT ATCCGCACCA ACGCGCAGCC CGGACTCGGT AATGGCGCGC
1851 ATTGCGCCCA GCGCCATCTG ATCGTTGGCA ACCAGCATCG CAGTGGGAAC
1901 GATGCCCTCA TTCAGCATTT GCATGGTTTG TTGAAAACCG GACATGGCAC
1951 TCCAGTCGCC TTCCCGTTCC GCTATCGGCT GAATTTGATT GCGAGTGAGA
2001 TATTTATGCC AGCCAGCCAG ACGCAGACGC GCCGAGACAG AACTTAATGG
2051 GCCCGCTAAC AGCGCGATTT GCTGGTGACC CAATGCGACC AGATGCTCCA
2101 CGCCCAGTCG CGTACCGTCT TCATGGGAGA AAATAATACT GTTGATGGGT
2151 GTCTGGTCAG AGACATCAAG AAATAACGCC GGAACATTAG TGCAGGCAGC
2201 TTCCACAGCA ATGGCATCCT GGTCATCCAG CGGATAGTTA ATGATCAGCC
2251 CACTGACGCG TTGCGCGAGA AGATTGTGCA CCGCCGCTTT ACAGGCTTCG
2301 ACGCCGCTTC GTTCTACCAT CGACACCACC ACGCTGGCAC CCAGTTGATC
2351 GGCGCGAGAT TTAATCGCCG CGACAATTTG CGACGGCGCG TGCAGGGCCA
2401 GACTGGAGGT GGCAACGCCA ATCAGCAACG ACTGTTTGCC CGCCAGTTGT
2451 TGTGCCACGC GGTTGGGAAT GTAATTCAGC TCCGCCATCG CCGCTTCCAC
2501 TTTTTCCCGC GTTTTCGCAG AAACGTGGCT GGCCTGGTTC ACCACGCGGG
```

Abbildung 4b (Fortsetzung)

```
2551 AAACGGTCTG ATAAGAGACA CCGGCATACT CTGCGACATC GTATAACGTT
2601 ACTGGTTTCA CATTCACCAC CCTGAATTGA CTCTCTTCCG GGCGCTATCA
2651 TGCCATACCG CGAAAGGTTT TGCACCATTC GATGGTGTCA ACGTAAATGC
2701 ATGCCGCTTC GCCTTCGCGC GCGAATTGTC GACCCTGTCC CTCCTGTTCA
2751 GCTACTGACG GGGTGGTGCG TAACGGCAAA AGCACCGCCG GACATCAGCG
2801 CTAGCGGAGT GTATACTGGC TTACTATGTT GGCACTGATG AGGGTGTCAG
2851 TGAAGTGCTT CATGTGGCAG GAGAAAAAAG GCTGCACCGG TGCGTCAGCA
2901 GAATATGTGA TACAGGATAT ATTCCGCTTC CTCGCTCACT GACTCGCTAC
2951 GCTCGGTCGT TCGACTGCGG CGAGCGGAAA TGGCTTACGA ACGGGGCGGA
3001 GATTTCCTGG AAGATGCCAG GAAGATACTT AACAGGGAAG TGAGAGGGCC
3051 GCGGCAAAGC CGTTTTTCCA TAGGCTCCGC CCCCCTGACA AGCATCACGA
3101 AATCTGACGC TCAAATCAGT GGTGGCGAAA CCCGACAGGA CTATAAAGAT
3151 ACCAGGCGTT TCCCCTGGCG GCTCCCTCGT GCGCTCTCCT GTTCCTGCCT
3201 TTCGGTTTAC CGGTGTCATT CCGCTGTTAT GGCCGCGTTT GTCTCATTCC
3251 ACGCCTGACA CTCAGTTCCG GGTAGGCAGT TCGCTCCAAG CTGGACTGTA
3301 TGCACGAACC CCCCGTTCAG TCCGACCGCT GCGCCTTATC CGGTAACTAT
3351 CGTCTTGAGT CCAACCCGGA AAGACATGCA AAAGCACCAC TGGCAGCAGC
3401 CACTGGTAAT TGATTTAGAG GAGTTAGTCT TGAAGTCATG CGCCGGTTAA
3451 GGCTAAACTG AAAGGACAAG TTTTGGTGAC TGCGCTCCTC CAAGCCAGTT
3501 ACCTCGGTTC AAAGAGTTGG TAGCTCAGAG AACCTTCGAA AAACCGCCCT
3551 GCAAGGCGGT TTTTTCGTTT TCAGAGCAAG AGATTACGCG CAGACCAAAA
3601 CGATCTCAAG AAGATCATCT TATTAATCAG ATAAAATATT TCTAGATTTC
3651 AGTGCAATTT ATCTCTTCAA ATGTAGCACC TGAAGTCAGC CCCATACGAT
3701 ATAAGTTGTT AATTCTCATG TTTGACAGCT TATCATCGAT
```

Abbildung 5

a

```
           10        20        30        40        50
            |         |         |         |         |
  1 CTCGAGGCAACGCAATTAATGTGAGTTAGCTCACTCATTAGGCACCCCAG
 51 GCTTTACACTTTATGCTTCCGGCTCGTATGTTGTGTGGAATTGTGAGCGG
101 ATAACAATTTCACACAGAATTC
```

b

```
           10        20        30        40        50
            |         |         |         |         |
  1 CTCGAGGATTCTGAAATGAGCTGTTGACAATTAATCATCGGCTCGTATAA
 51 TGTGTGGAATTGTGAGCGGATAACAATTTCACACAGAATTC
```

c

```
           10        20        30        40        50
            |         |         |         |         |
  1 CTCGAGAAATCATAAAAAATTTATTTGCTTTGTGAGCGGATAACAATTAT
 51 AATAGATTCAATTGTGAGCGGATAACAATTTCACACAGAATTC
```

d

```
           10        20        30        40        50
            |         |         |         |         |
  1 CTCGAGAAAATTTATCAAAAAGAGTGTTGACTTGTGAGCGGATAACAATG
 51 ATACTTAGATTCATCGAGAGGGACACGGCGAATTC
```

e

```
           10        20        30        40        50
            |         |         |         |         |
  1 CTCGAGAAAATTTATCAAAAAGAGTGTTGACTTGTGAGCGGATAACAATT
 51 ATAATTACAGCCATCGAGAGGGACACGGCGAATTC
```

f

```
           10        20        30        40        50
            |         |         |         |         |
  1 CTCGAGAAAATTTATCAAAAAGAGTGTTGACTTGTGAGCGCTCACAATTG
 51 ATACTTAGATTCATCGAGAGGGACACGGCGAATTC
```

g

```
           10        20        30        40        50
            |         |         |         |         |
  1 CTCGAGAATTGTGAGCGGATAACAATTTAGTTGACTTAAAGTCTAACCTA
 51 TAGGATACTTAGATTCATCGAGAGGGACACGGCGAATTC
```

h

```
           10        20        30        40        50
            |         |         |         |         |
  1 CTCGAGAATTGTGAGCGGATAACAATTTAGTTGACTTAAAGTCTAACCTA
 51 TAGTATAATTAGATTCATCGAGAGGGACACGGCGAATTC
```

Abbildung 5 (Fortsetzung)

```
i          10        20        30        40        50
           |         |         |         |         |
      1 CTCGAGAAAATTTATCAAAAAGAGTGTTGACTTGTGAGCGGATAACAATG
     51 ATACTTAGATTCAAATTGTGAGCGGATAACAATTTGAATTC

j          10        20        30        40        50
           |         |         |         |         |
      1 CTCGAGAAAATTTATCAAAAAGAGTGTTGACTTGTGAGCGGATAACAATG
     51 ATACTTAGATTCAATTGTGAGCGGATAACAATTTCACACAGAATTC
```

Abbildung 6

1 GAATTCCTCG AGGCTGGCAT CCCTAACATA TCCGAATGGT TACTTAAACA

51 ACGGAGGACT AGCGTATCCC TTCGCATAGG GTTTGAGTTA GATAAAGTAT

101 ATGCTGAACT TTCTTCTTTG CTCAAAGAAT CATAAAAAAT TTATTTGCTT

151 TCAGGAAAAT TTTTCTGTAT AATAGATTCA TAAATTTGAG AGAGGAGTTT

201 AAATATGGCT GGTTCTCGCA GAAAGAAACA TATCCATGAA ATCCCGCCTC

251 GAGGAATTC

Abbildung 7

N25*/O

1 CTCGAGGCTG GCATCCCTAA CATATCCGAA TGGTTACTTA AACAACGGAG

51 GACTAGCGTA TCCCTTCGCA TAGGGTTTGA GTTAGATAAA GTATATGCTG

101 AACTTTCTTC TTTGCTCAAA GAATCATAAA AAATTTATTT GCTTTCAGGA

151 AAATTTTTCT GTATAATAGA TTCAAATTGT GAGCGGATAA CAATTTGAAT

201 TC

N25OP29

1 CTCGAGGCTG GCATCCCTAA CATATCCGAA TGGTTACTTA AACAACGGAG

51 GACTAGCGTA TCCCTTCGCA TAGGGTTTGA GTTAGATAAA GTATATGCTG

101 AACTTTCTTC TTTGCTCAAA GAATCATAAA AAATTTATTT GCTTTCAGGA

151 AAATTTTTCT GTATAATAGA TTCAATTGTG AGCGGATAAC AATTTCACAC

201 AGAATTC

Abbildung 8

1 CTCGAGGAAC GCCTATCTTA AAGTTTAAAC ATAAAGACCA GACCTAAAGA

51 CCAGACCTAA AGACACTACA TAAAGACCAG ACCTAAAGAC GCCTTGTTGT

101 TAGCCATAAA GTGATAACCT TTAATCATTG TCTTTATTAA TACAACTCAC

151 TATAAGGAGA GACAACTTAA AGAGACTTAA AAGATTAATT TAAAATTTAT

201 CAAAAAGAGT ATTGACTTAA AGTCTAACCT ATAGGATACT TACAGCCATC

251 GAGAGGGACA CGGCGAATAG CCATCCCAAT CGACACCGGG GTCCCTCGAG

301 GCGAATTCCG GATCC

Abbildung 9

```
          10        20        30        40        50
  1 GTCGACGTTGATCCCCTAGAAATTGTGAGCGCTCACAATTTCTAGGGAAT
 51 TAACGGTACCGAGCTTGTGGCAGTTTAAGGCGGGCGTCCTGCCCGCCACC
101 CTCCGGGCCGTTGCTTCGCAACGTTCAAATCCGCTCCCGGCGGATTTGTC
151 CTACTCAGGAGAGCGTTCACCGACAAACAACAGATAAAACGAAAGGCCCA
201 GTCTTTCGACTGAGCCTTTCGTTTTATTTGATGCCTCAAGCTCGGTACCT
251 CGAGAAAATTTATCAAAAAGAGTGTTGACTTGTGAGCGGATAACAATGAT
301 ACTTAGATTCATCGAGAGGGACACGGCGAATTC
```

Abbildung 10

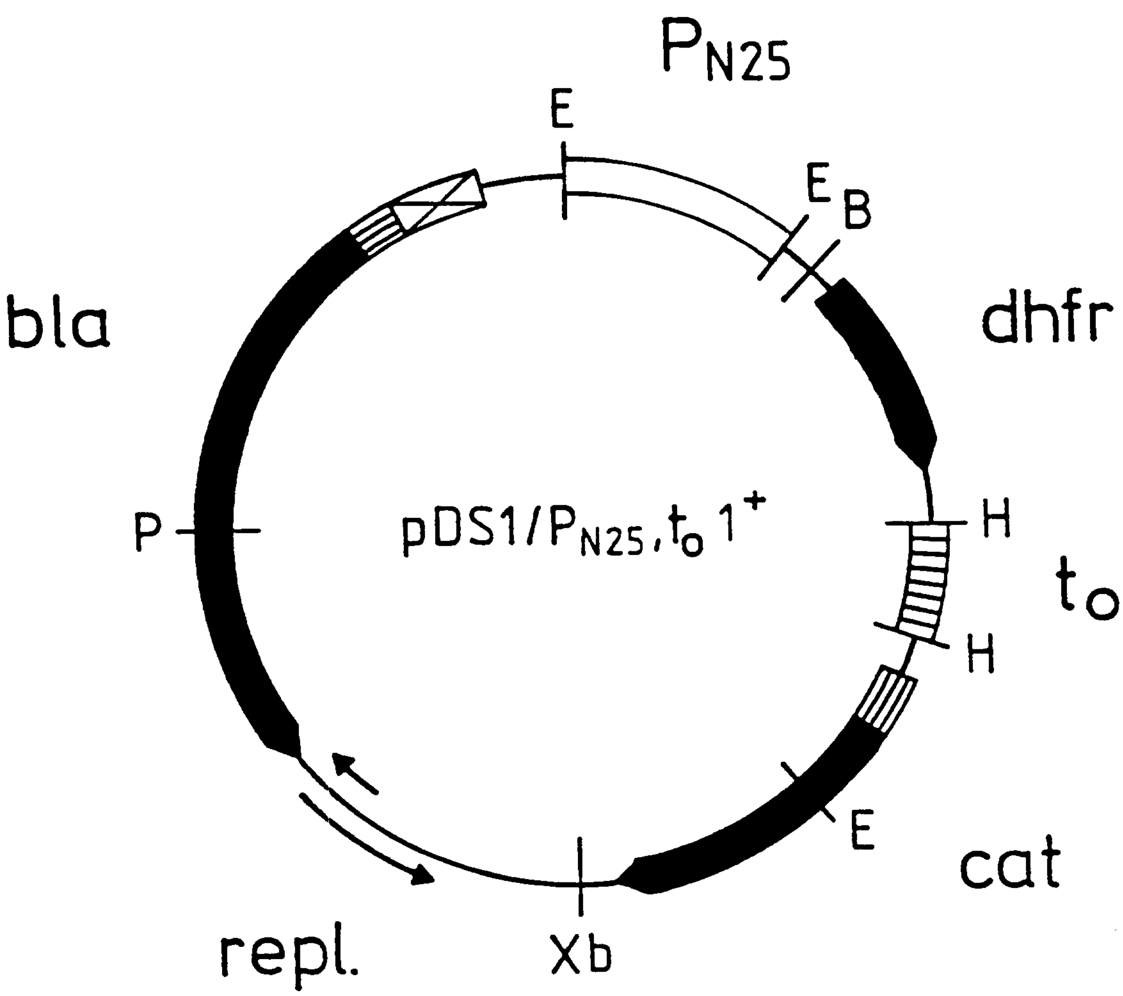

Abbildung 11

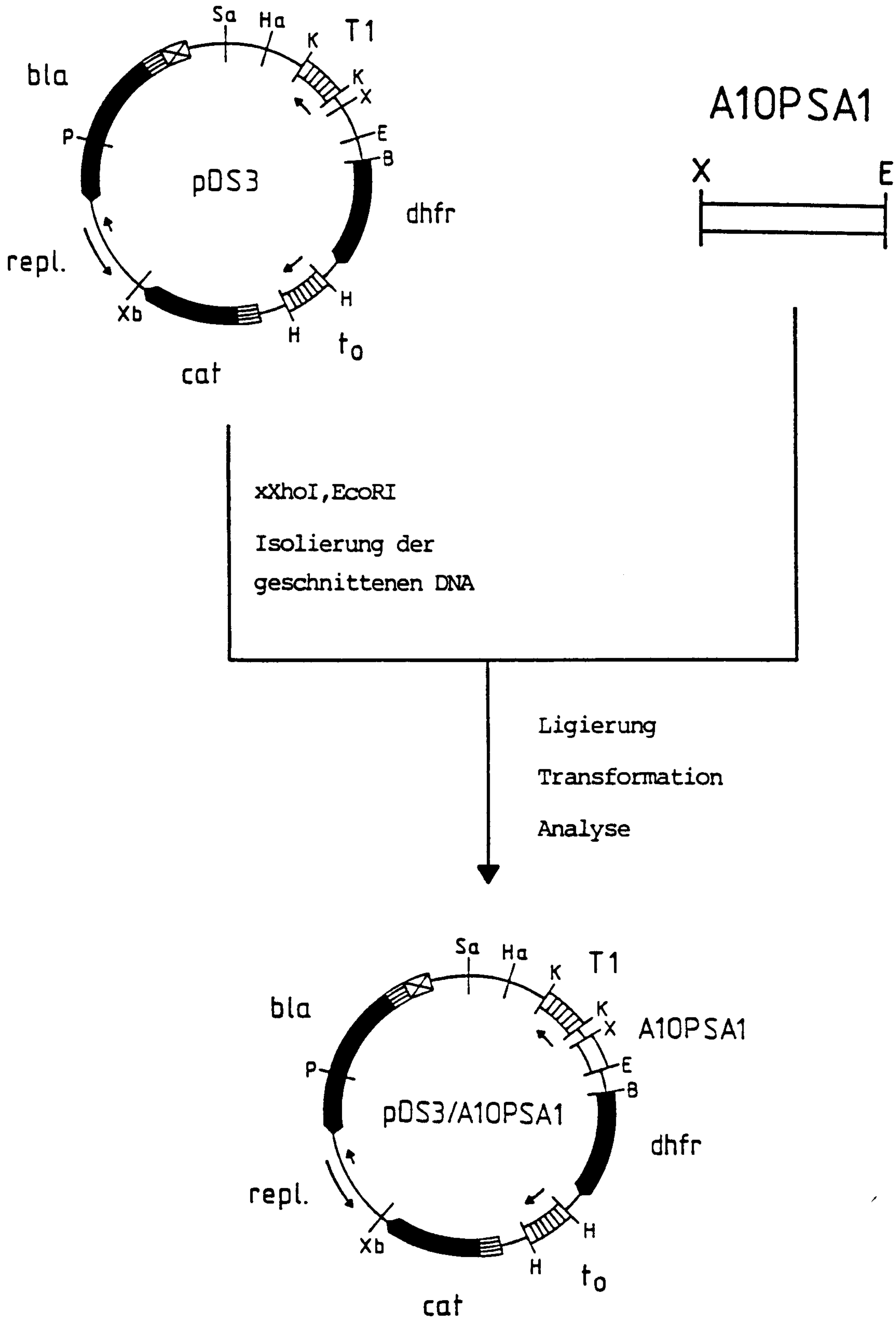

Abbildung 12

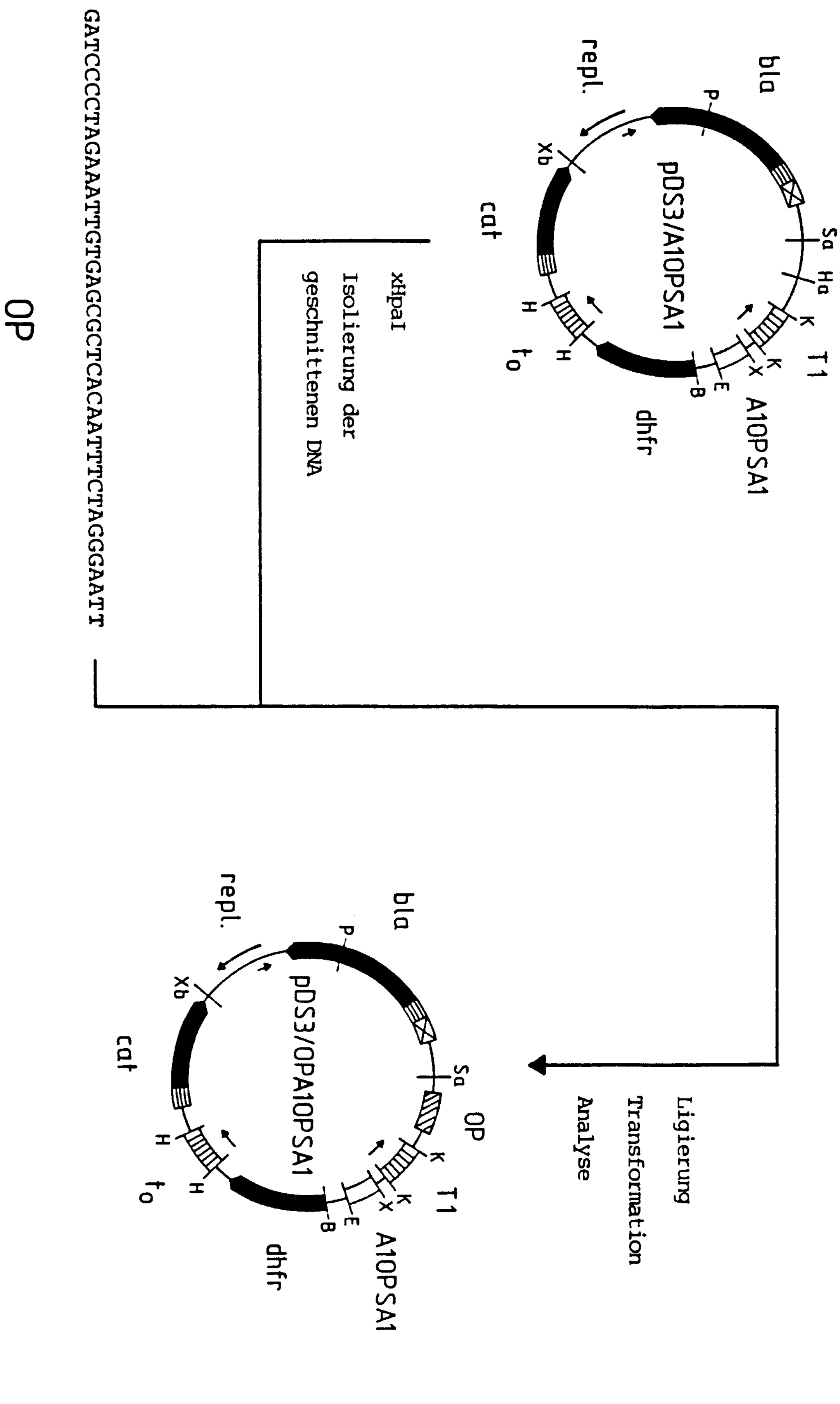

Abbildung 13

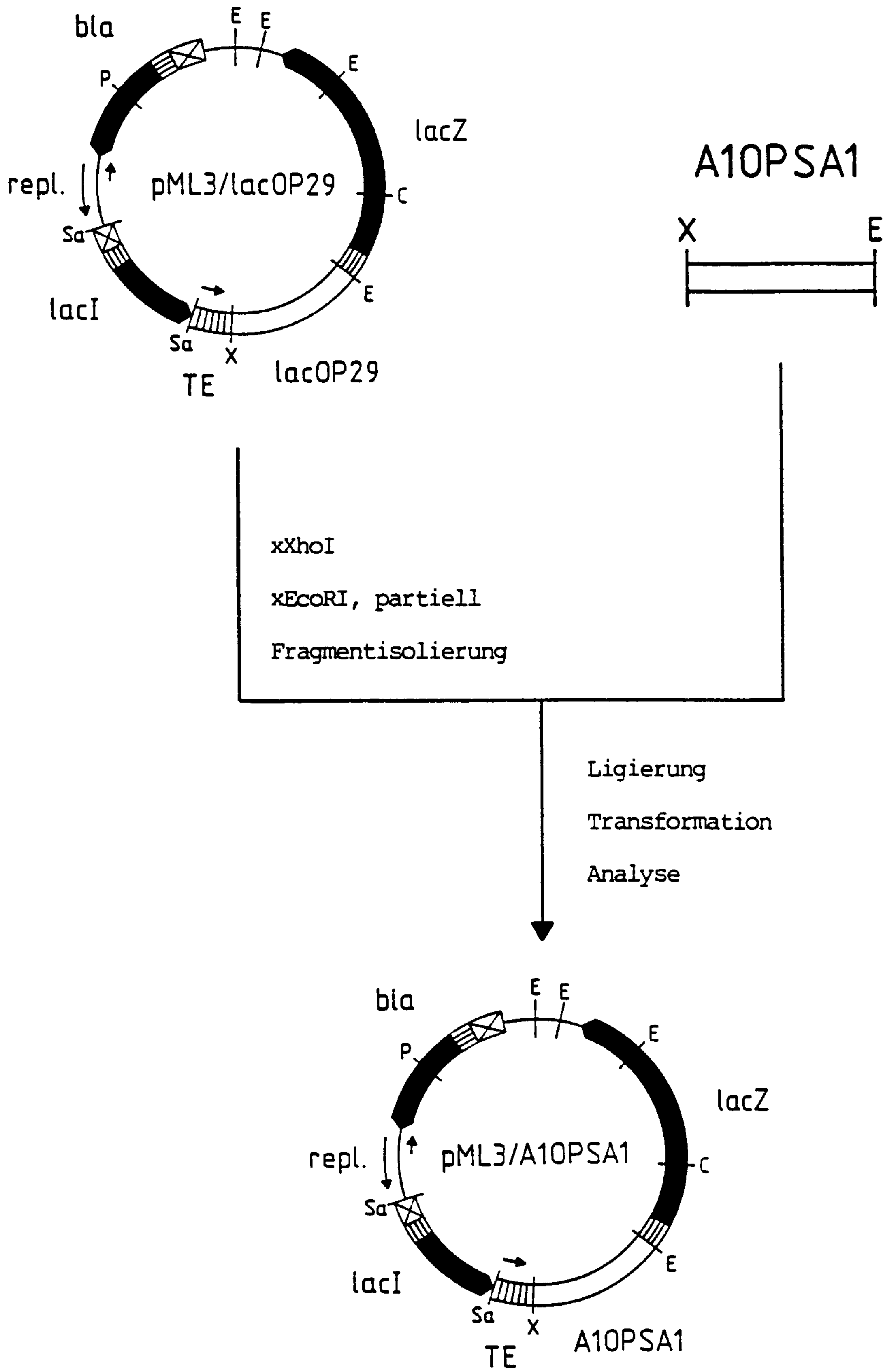

Abbildung 14

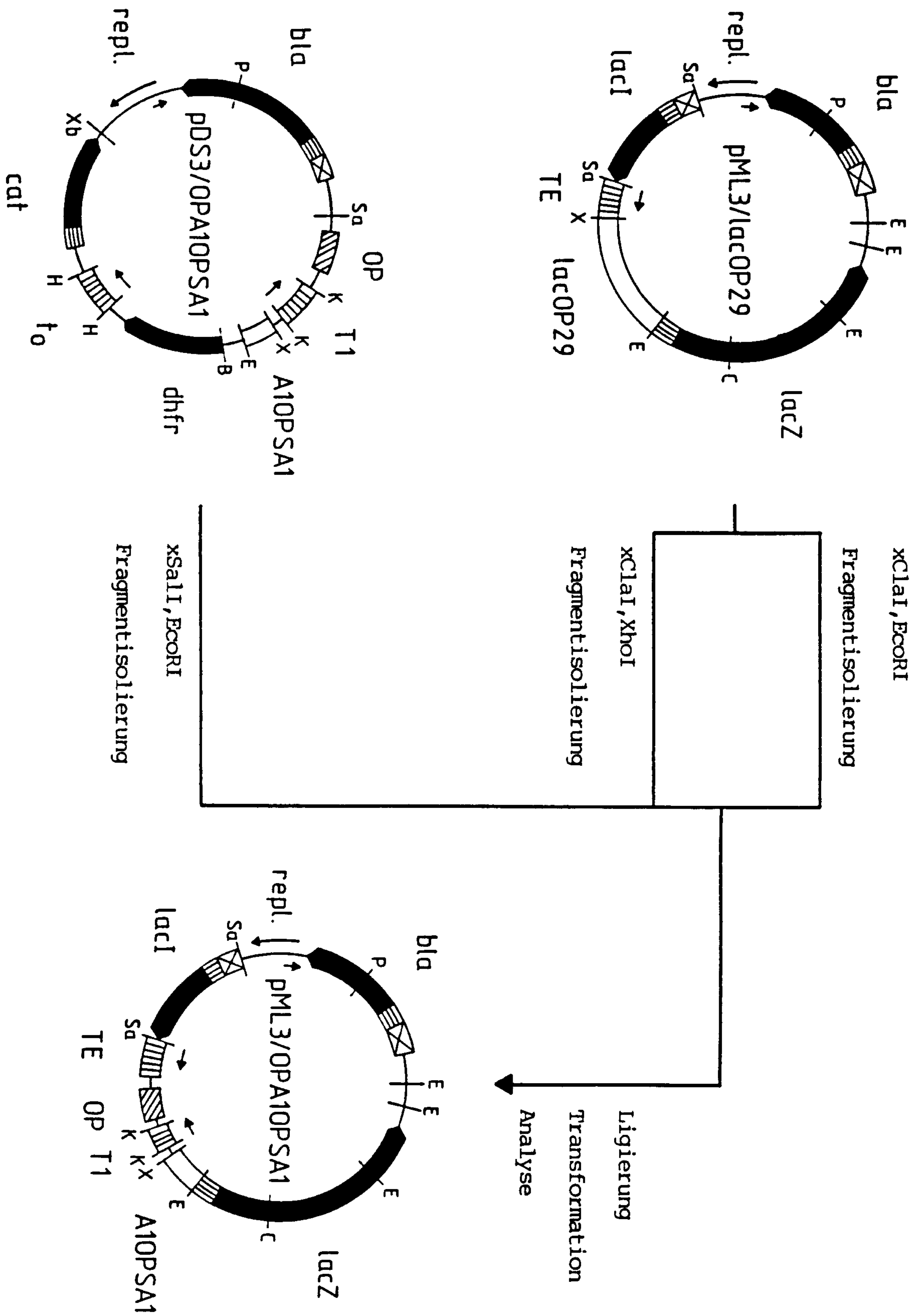

Abbildung 15

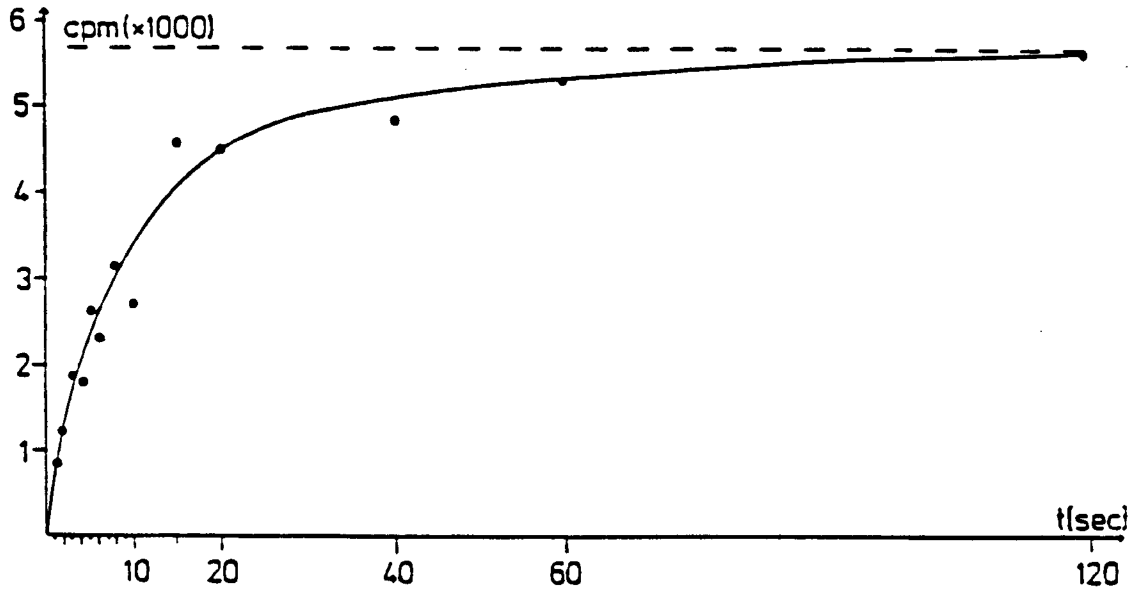

Abbildung 16

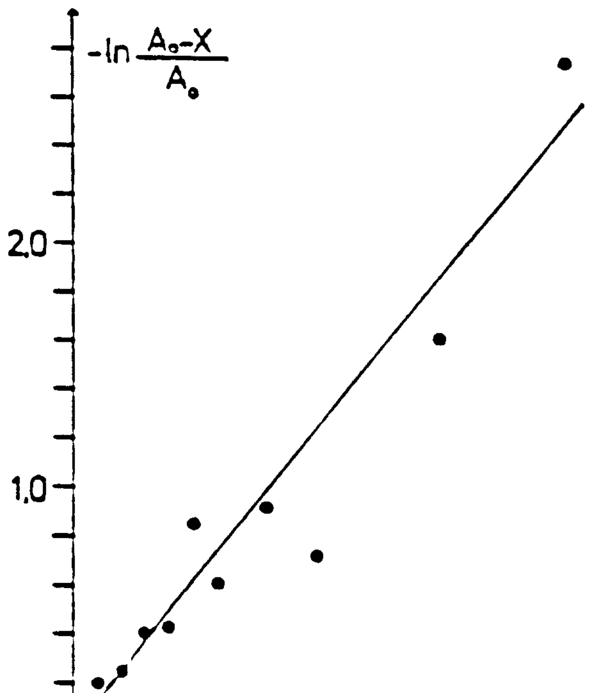

Abbildung 17

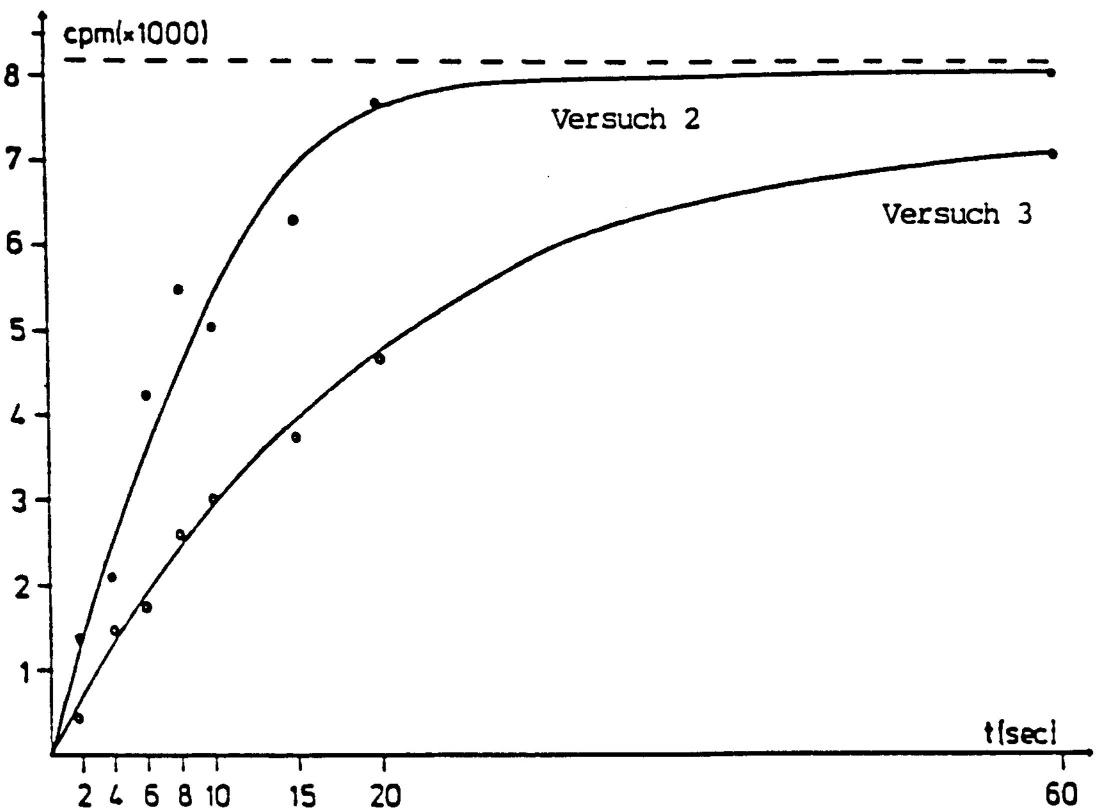

Abbildung 18

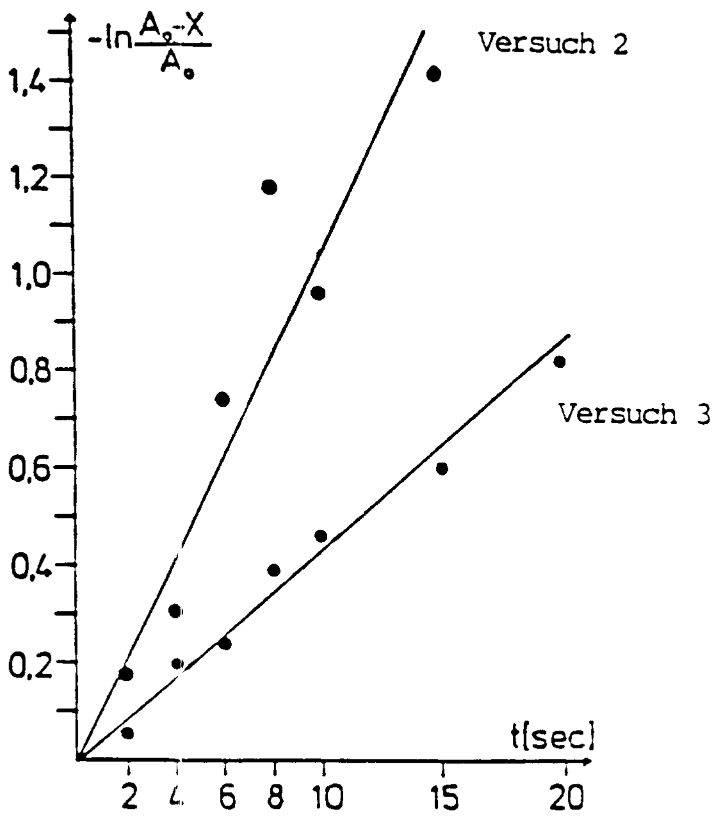

Abbildung 19

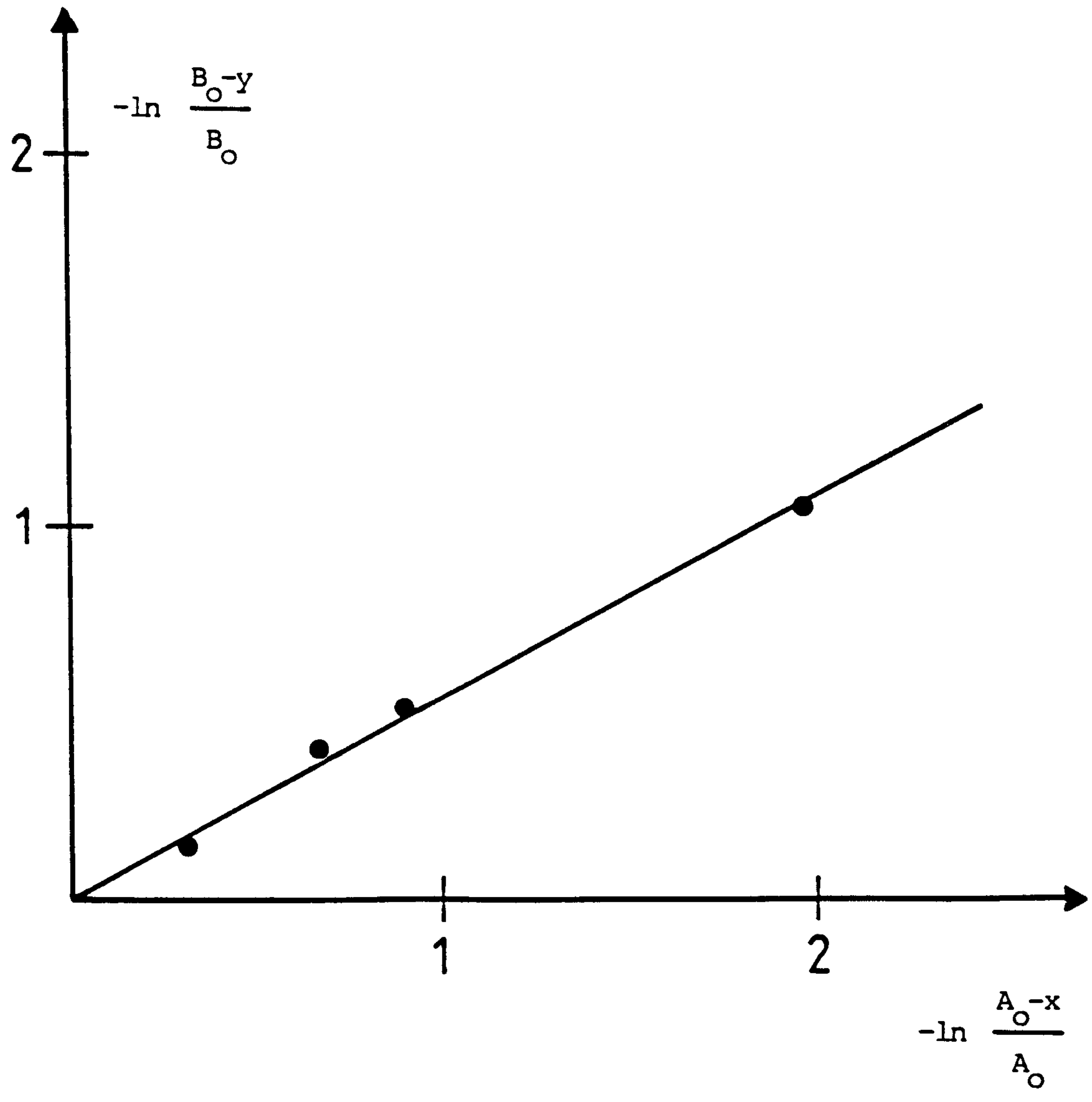

Abbildung 20

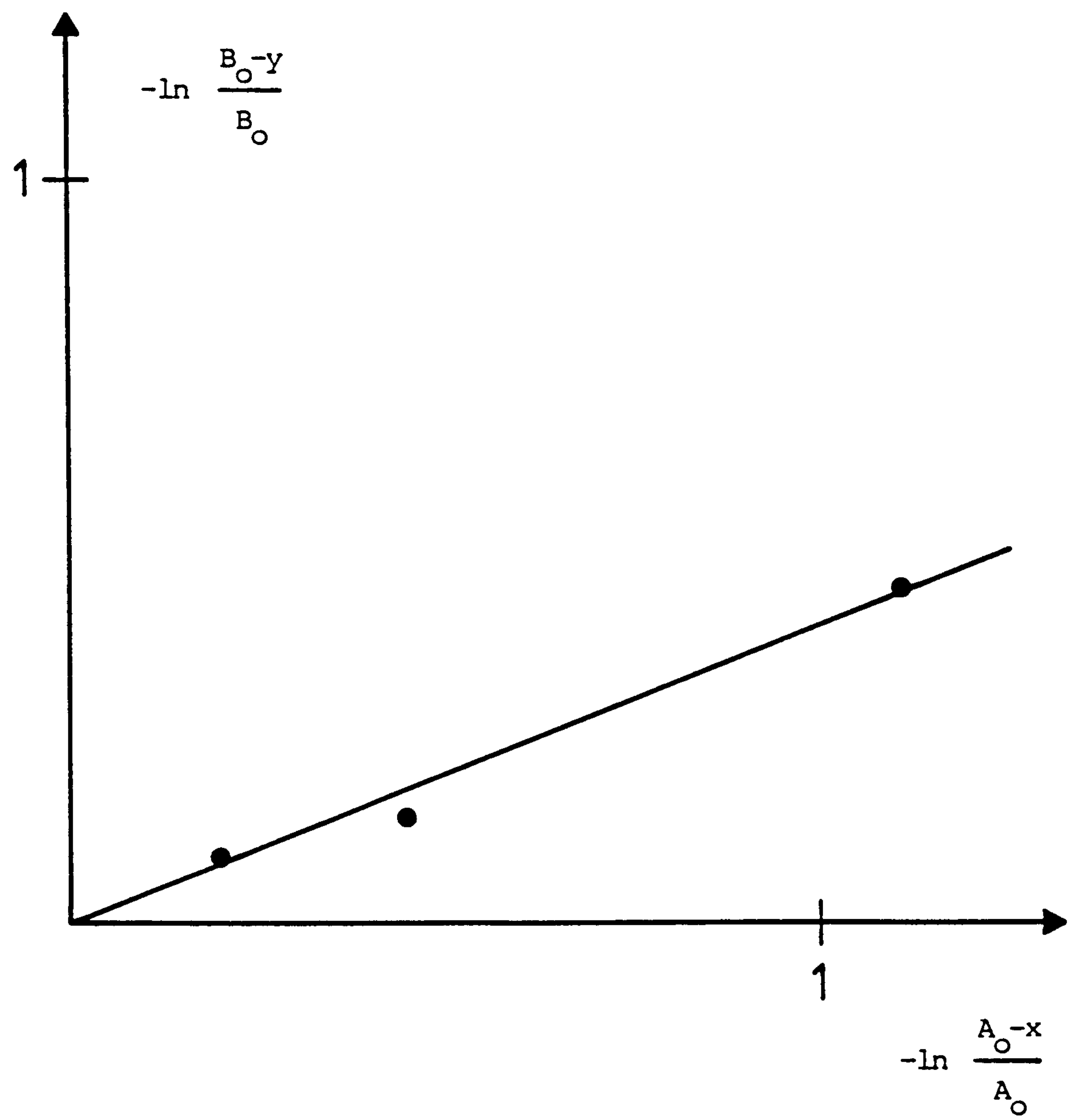

Abbildung 21